# EUROPEAN PATENT APPLICATION

(11) **EP 4 552 613 A2**
(43) Date of publication of application: **14.05.2025**
(21) Application number: 25165816.7
(22) Date of filing: 06.10.2021
(51) Int. Cl.: A61F 2/24

(54) **PROTECTIVE COVERS FOR PROSTHETIC VALVES**

(30) Priority: 06.10.2020 US 202063088352 P; 29.10.2020 US 202063107377 P; 04.08.2021 US 202163229212 P
(62) Divisional of application: 21801745.7
(71) Applicant: Edwards Lifesciences Corporation, Irvine, CA 92614 (US)
(72) Inventor: Ben Zaken, Nadav, IRVINE, CA, 92614 (US); Bukin, Michael, IRVINE, CA, 92614 (US); Yousef, Abdorruhman M., IRVINE, CA, 92614 (US); Pawar, Sandip Vasant, IRVINE, CA, 92614 (US); Bash, Assaf, IRVINE, CA, 92614 (US)
(74) Representative: Eisenführ Speiser

(57) **Abstract**

The present invention relates to implantable prosthetic devices, such as prosthetic heart valves, provided with a plurality of protective covers over various components of the frame or commissural support members of the valves.

## Description

### FIELD OF THE INVENTION

The present invention relates to implantable prosthetic devices, such as prosthetic heart valves, provided with a plurality of protective covers over various components of the frame or commissural support members of the valves.

### BACKGROUND OF THE INVENTION

Native heart valves, such as the aortic, pulmonary and mitral valves, function to assure adequate directional flow from and to the heart, and between the heart's chambers, to supply blood to the whole cardiovascular system. Various valvular diseases can render the valves ineffective and require replacement with artificial valves. Surgical procedures can be performed to repair or replace a heart valve. Surgeries are prone to an abundance of clinical complications, hence alternative less invasive techniques of delivering a prosthetic heart valve over a catheter and implanting it over the native malfunctioning valve, have been developed over the years.

Different types of prosthetic heart valves are known to date, including balloon expandable valve, self-expandable valves and mechanically-expandable valves. Different methods of delivery and implantation are also known, and may vary according to the site of implantation and the type of prosthetic valve. One exemplary technique includes utilization of a delivery assembly for delivering a prosthetic valve in a crimped state, from an incision which can be located at the patient's femoral or iliac artery, toward the native malfunctioning valve. Once the prosthetic valve is properly positioned at the desired site of implantation, it can be expanded against the surrounding anatomy, such as an annulus of a native valve, and the delivery assembly can be retrieved thereafter.

A prosthetic valve conventionally includes a circumferential frame that can be a metallic frame configured to transition between compressed and expanded configurations, and soft components sutured thereto, such as a leaflet assembly composed of a plurality of leaflets attached to the frame via a plurality of commissure assemblies, and configured to regulate blood flow through the prosthetic valve, as well as a skirt that can prevent perivalvular leakage as further serve as an intermediate means of attachment of the leaflet assembly, along a lower scalloped edge thereof, to the frame. The average human heart beats about 100,000 times per day. Thus, such soft components may be subject to wear due to ongoing movement thereof relative to the frame, as the leaflets move between open and closed states during systolic and diastolic phases of the blood flow through the valve.

Leaflets of conventional prosthetic valves are typically designed to open to only a limited extent at the outflow end area, in order to prevent the valve leaflets from coming into contact with the frame of the prosthetic valve. Contact with the frame as the leaflets open can damage, weaken, and/or cause wear to the leaflets. As a result, the opening at the outflow end of the leaflets can be more narrow than the inner diameter of the valve frame, leaving a gap between the fully-open leaflets and the inner surface of the valve frame. By limiting the extent to which the leaflets may open, the blood flow through the valve is thereby limited.

Similarly, obtaining adequate effective orifice area (EOA) in a valve replacement is important to minimize pressure gradients across the prosthetic aortic valve, for example. By limiting the extent to which the leaflets can open, the EOA is also thereby limited.

### SUMMARY OF THE INVENTION

The present disclosure is directed toward prosthetic heart valves that include an expandable frame and a leaflet assembly mounted therein, wherein the leaflet assembly is coupled to the frame via a plurality of commissure assemblies, formed from tabs of adjacent asymmetrical leaflets secured to each other, and potentially attached along an opposite scalloped edge thereof to an inner skirt, which is sutured in turn to the frame, wherein various components of the valve to which commissure assemblies and/or the inner skirt are attached, are covered by heat-shrink wraps tightly shrunk thereover.

According to an aspect of the invention, there is provided a prosthetic valve comprising a frame, a plurality of commissural support members attached to the frame, and a leaflet assembly mounted within the frame. The frame is movable between a radially compressed and a radially expanded configuration. Each commissural support member is covered by a heat-shrink wrap, tightly shrunk thereover. The leaflet assembly comprises a plurality of leaflets configured to regulate flow through the prosthetic valve, wherein each leaflet comprises a pair of oppositely-directed tabs, wherein the tabs of adjacent leaflets are paired to form commissure assemblies. Each commissure assembly is attached to a corresponding commissural support member over the heat-shrink wrap.

According to some examples, the prosthetic valve further comprises a plurality of expansion and locking assemblies. Each expansion and locking assembly comprises an outer member coupled to the frame at a first location, and an inner member coupled to the frame at a second location spaced apart from the first location, wherein a portion of each outer member, extending from the first location in the first direction, is the commissural support member. Movement of the inner member in a first direction, relative to the outer member, causes the frame to foreshorten axially and expand radially.

According to some examples, the first direction is a proximally oriented direction.

According to some examples, the frame comprises a plurality of inflow apices, a plurality of outflow apices, and a plurality of non-apical junctions, and wherein the first location is a proximal-most non-apical junction.

According to some examples, the commissural support member has an exposed proximal end.

According to some examples, the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

According to some examples, the heat-shrink wrap is a heat-shrink sleeve.

According to some examples, the length of the heat-shrink sleeve is in the range of 90% to 110% of the length of the commissural support member.

According to some examples, the commissural support member has a rectangular cross section, defining a support member radial depth and a support member lateral width.

According to some examples, the heat-shrink sleeve has an elliptical cross section in a pre-shrunk state thereof, defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

According to some examples, the heat-shrink sleeve has a circular cross section in a pre-shrunk state thereof that can be squeezed to an elliptical shape defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

According to some examples, the sleeve first diameter is at least as great as 120% of the support member radial depth.

According to some examples, the sleeve first diameter is at least as great as 150% of the support member radial depth.

According to some examples, the sleeve first diameter is at least as great as 200% of the support member radial depth.

According to some examples, the sleeve second diameter is at least as great as 120% of the support member lateral width.

According to some examples, the sleeve second diameter is at least as great as 150% of the support member lateral width.

According to some examples, the sleeve second diameter is at least as great as 200% of the support member lateral width.

According to some examples, the commissural support member defines a maximal support member diameter, and wherein the heat-shrink sleeve, in a pre-shrunk state thereof, defines a minimal sleeve diameter which is greater than the maximal support member diameter.

According to some examples, the minimal sleeve diameter is at least as great as 120% of the maximal support member diameter.

According to some examples, the minimal sleeve diameter is at least as great as 150% of the maximal support member diameter.

According to some examples, the minimal sleeve diameter is at least as great as 200% of the maximal support member diameter.

According to another aspect of the invention, there is provided a prosthetic valve comprising a frame, an inner skirt disposed along an inner surface of the frame, a leaflet assembly mounted within the frame, and a plurality of heat-shrink wraps. The frame is movable between a radially compressed and a radially expanded configuration, and comprises a plurality of interconnected strut segments, each strut segment having a strut width, a strut thickness, and a strut segment length. The inner skirt comprises a skirt proximal end attached to strut segments extending along the circumference of the frame. The leaflet assembly comprises a plurality of leaflets configured to regulate flow through the prosthetic valve, wherein the leaflet assembly is sutured to the inner skirt along a scallop line that tracks the lower edge of the leaflet assembly. The heat-shrink wraps are tightly shrunk over the strut segments to which the skirt proximal end is attached.

According to some examples, the skirt proximal end is sutured to the strut segment with at least one skirt attachment suture.

According to some examples, the frame comprises a plurality of struts arranged in a lattice pattern and pivotably coupled to each other, wherein each strut comprises a plurality of the strut segments.

According to some examples, the strut segments are angled strut segments.

According to some examples, the skirt proximal end is attached to the strut segments following a zig-zag pattern.

According to some examples, each strut segment has a rectangular cross-section, wherein the strut width is a linear strut width, and wherein the strut thickness is a linear strut thickness.

According to some examples, each strut segment has an elliptic cross-section, wherein the strut width is a first diameter of the cross-section, and wherein the strut thickness is a second diameter of the cross-section.

According to some examples, each strut segment has circular cross-section, and wherein the strut width and the strut thickness are equal to each other and to a circular diameter of the cross-section.

According to some examples, the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

According to some examples, the heat-shrink wrap is a heat-shrink film, having a pre-shrunk rectangular shape with a film length and a film width.

According to some examples, the film length is not greater than the strut segment length.

According to some examples, the film width in a pre-shrunk state thereof, is greater than the strut width plus twice the strut thickness.

According to some examples, the film width in a pre-shrunk state thereof, is greater than the twice the strut width plus twice the strut thickness.

According to some examples, the skirt attachment suture is looped through the skirt proximal end and the heat-shrink film, in a whip stitch pattern around the strut segment.

According to some examples, the stitch pattern comprises two sections of the skirt attachment suture extending substantially parallel to each other within each loop formed by the stitching pattern, between the strut segment and opposite sections of the heat-shrink film.

According to some examples, the stitch pattern comprises two sections of the skirt attachment suture extending substantially parallel to each other within each loop formed by the stitching pattern, wherein one section extends between the strut segment and an inner side of a section of the heat-shrink film, and another section extends parallel thereto over an external side of a section of the heat-shrink film.

According to some examples, the skirt attachment suture is sutured over the external surface of the heat-shrink film, without extending through the heat-shrink film.

According to some examples, the heat-shrink wrap is a C-shaped heat-shrink tube, having a tube length, a tube first diameter and a tube second diameter, wherein the tube second diameter is oriented in the radial direction, wherein the tube first diameter is substantially orthogonal to the radial direction, wherein the tube first diameter is greater than the strut width, and wherein the tube second diameter is greater than the strut thickness.

According to some examples, the C-shaped heat-shrink tube has a circular cross-section such that the tube first diameter is equal to the tube second diameter.

According to some examples, the tube length is not greater than the strut segment length.

According to some examples, the C-shaped heat-shrink tube comprises, in a pre-shrunk state thereof, a gap having a gap width that is smaller than the strut width and/or the strut thickness, in a free state of the C-shaped heat-shrink tube.

According to some examples, the tube first diameter is at least as great as 120% of the strut width.

According to some examples, the tube first diameter is at least as great as 150% of the strut width.

According to some examples, the tube first diameter is at least as great as 200% of the strut width.

According to some examples, the tube second diameter is at least as great as 120% of the strut thickness.

According to some examples, the tube second diameter is at least as great as 150% of the strut thickness.

According to some examples, the tube second diameter is at least as great as 200% of the strut thickness.

According to some examples, at least one skirt attachment suture is looped through the skirt proximal end and the C-shaped heat-shrink tube, in a whip stitch pattern around the strut segment.

According to some examples, the stitch pattern comprises two sections of the skirt attachment suture extending substantially parallel to each other within each loop formed by the stitching pattern, between the strut segment and opposite sections of the heat-shrink tube.

According to some examples, the stitch pattern comprises two sections of the skirt attachment suture extending substantially parallel to each other within each loop formed by the stitching pattern, wherein one section extends between the strut segment and an inner side of a section of the heat-shrink tube, and another section extends parallel thereto over an external side of a section of the heat-shrink tube.

According to some examples, at least one skirt attachment suture is sutured through the inner skirt and over the external surface of the heat-shrink tube, without extending through the heat-shrink tube.

According to yet another aspect of the invention, there is provided a prosthetic valve comprising a frame movable between a radially compressed and a radially expanded configuration, and a leaflet assembly mounted within the frame. The frame comprises a plurality of commissural support members, each commissural support member having a support member radial depth and a support member lateral width, wherein each commissural support member is covered by a heat-shrink wrap, tightly shrunk thereover. The leaflet assembly comprises a plurality of leaflets configured to regulate flow through the prosthetic valve, wherein each leaflet comprises a pair of oppositely-directed tabs, wherein the tabs of adjacent leaflets are paired to form commissure assemblies. Each commissure assembly is attached to a corresponding commissural support member over the heat-shrink wrap.

According to some examples, the frame comprises a plurality of axially extending strut segments, wherein at least some of the axially extending strut segments are the commissural support members.

According to some examples, the commissural support members are integrally formed with the frame.

According to some examples, the plurality of commissural support members includes three commissural support members.

According to some examples, the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

According to some examples, each commissural support member has a rectangular cross section, wherein the support member radial depth is a linear radial depth, and wherein the support member lateral width is a linear lateral width.

According to some examples, each commissural support member has an elliptic cross section, wherein the support member radial depth is a support member first diameter, and wherein the support member lateral width is a support member second diameter.

According to some examples, each commissural support member has a circular cross section, and wherein the support member radial depth and the support member lateral width are equal to each other and to a circular diameter of the commissural support member.

According to some examples, each commissural support member has an exposed proximal end.

According to some examples, the heat-shrink wrap is a heat-shrink sleeve.

According to some examples, the length of the heat-shrink sleeve is in the range of 90% to 110% of the length of the commissural support member.

According to some examples, the heat-shrink sleeve has a circular cross section in a pre-shrunk state thereof, defining a sleeve circular diameter that is greater than the support member radial depth and/or than the support member lateral width.

According to some examples, the sleeve circular diameter is at least as great as 120% of the support member radial depth and/or of the support member lateral width.

According to some examples, the sleeve circular diameter is at least as great as 150% of the support member radial depth and/or of the support member lateral width.

According to some examples, the sleeve circular diameter is at least as great as 200% of the support member radial depth and/or of the support member lateral width.

According to some examples, each commissural support member is bound between angled strut segments of the frame.

According to some examples, the heat-shrink wrap is a heat-shrink film, having a pre-shrunk rectangular shape with a film length and a film width.

According to some examples, the film length is not greater than the support member length.

According to some examples, the film width in a pre-shrunk state thereof, is greater than the support member lateral width plus twice the support member radial depth.

According to some examples, the film width in a pre-shrunk state thereof, is greater than twice the support member lateral width plus twice the support member radial depth.

According to some examples, the heat-shrink wrap is a C-shaped heat-shrink tube, having a tube length, a tube first diameter and a tube second diameter, wherein the tube second diameter is oriented in the radial direction, wherein the tube first diameter is substantially orthogonal to the radial direction, wherein the tube first diameter is greater than the strut width, and wherein the tube second diameter is greater than the strut thickness.

According to some examples, the tube length is not greater than the support member length.

According to some examples, the C-shaped heat-shrink tube comprises, in a pre-shrunk state thereof, a gap having a gap width that is smaller than the support member radial depth and/or the support member lateral width, in a free state of the C-shaped heat-shrink tube.

According to some examples, the tube first diameter is at least as great as 120% of the support member lateral width.

According to some examples, the tube first diameter is at least as great as 150% of the support member lateral width.

According to some examples, the tube first diameter is at least as great as 200% of the support member lateral width.

According to some examples, the tube second diameter is at least as great as 120% of the support member radial depth.

According to some examples, the tube second diameter is at least as great as 150% of the support member radial depth.

According to some examples, the tube second diameter is at least as great as 200% of the support member radial depth.

According to yet another aspect of the invention, there is provided a method of assembling a prosthetic valve. The method comprises providing a prosthetic valve comprising frame movable between a radially compressed and a radially expanded configuration, and a plurality of commissural support members attached to the frame. The method further comprises disposing a heat-shrink sleeve over each commissural support member. The method further comprises applying heat to the heat-shrink sleeve, thereby causing it to shrink radially inward and to become tightly attached around the corresponding commissural support member. The method further comprises attaching a commissure assembly of a leaflet assembly to each commissural support member covered by the heat-shrink sleeve.

According to some examples, the commissural support member has an exposed proximal end, and wherein disposing the heat-shrink sleeve over each commissural support member comprises sliding the heat-shrink sleeve from the exposed upper end over the corresponding commissural support member.

According to some examples, the leaflet assembly comprises a plurality of leaflets configured to regulate flow through the prosthetic valve, wherein each leaflet comprises a pair of oppositely-directed tabs, and wherein the tabs of adjacent leaflets are paired to form the commissure assemblies.

According to some examples, the prosthetic valve comprises a plurality of expansion and locking assemblies, each comprising an outer member coupled to the frame at a first location and an inner member coupled to the frame at a second location spaced apart from the first location, wherein movement of the inner member in a first direction, relative to the outer member, causes the frame to foreshorten axially and expand radially, and wherein a portion of each outer member, extending from the first location in the first direction, is the commissural support member.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, applying heat to the heat-shrink sleeve comprises applying heat at a temperature of above 90°C.

According to some examples, applying heat to the heat-shrink sleeve comprises applying heat at a temperature of above 135°C.

According to some examples, the length of the heat-shrink sleeve is in the range of 90% to 110% of the length of the commissural support member.

According to some examples, the length of the heat-shrink sleeve is greater than 110% of the length of the commissural support member, and wherein the method further comprises a step of cutting the heat-shrink sleeve to a length that is not higher than 110% of the length of the commissural support member prior to the step of applying heat.

According to some examples, the commissural support member has a rectangular cross section, defining a support member radial depth and a support member lateral width, wherein the heat-shrink sleeve has an elliptical cross section in a pre-shrunk state thereof, defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

According to some examples, the commissural support member has a rectangular cross section, defining a support member radial depth and a support member lateral width, wherein the heat-shrink sleeve has a circular cross section in a pre-shrunk state thereof that can be squeezed to an elliptical shape defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

According to some examples, the sleeve first diameter is at least as great as 120% of the support member radial depth.

According to some examples, the sleeve first diameter is at least as great as 150% of the support member radial depth.

According to some examples, the sleeve first diameter is at least as great as 200% of the support member radial depth.

According to some examples, the sleeve second diameter is at least as great as 120% of the support member lateral width.

According to some examples, the sleeve second diameter is at least as great as 150% of the support member lateral width.

According to some examples, the sleeve second diameter is at least as great as 200% of the support member lateral width.

According to some examples, the commissural support member defines a maximal support member diameter, and wherein the heat-shrink sleeve, in a pre-shrunk state thereof, defines a minimal sleeve which is greater than the maximal support member diameter.

According to some examples, the minimal sleeve diameter is at least as great as 120% of the maximal support member diameter.

According to some examples, the minimal sleeve diameter is at least as great as 150% of the maximal support member diameter.

According to some examples, the minimal sleeve diameter is at least as great as 200% of the maximal support member diameter.

According to yet another aspect of the invention, there is provided a method of assembling a prosthetic valve. The method comprises providing a prosthetic valve comprising frame movable between a radially compressed and a radially expanded configuration, an inner skirt having a skirt proximal end, and a leaflet assembly sutured to the inner skirt along a scallop line that tracks the lower edge of the leaflet assembly.

The method further comprises folding a plurality of heat-shrink films over a matching plurality of strut segments of the frame, wherein each heat-shrink film has a film length and a film width prior to folding thereof, and wherein each strut segment has a strut width, a strut thickness, and a strut segment length. The method further comprises suturing the inner skirt along the skirt proximal end to the folded heat-shrink films with at least one skirt attachment suture. The method further comprises applying heat to the heat-shrink films, thereby causing them to shrink radially inward and to become tightly attached around the corresponding strut segments.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, the film length is not greater than the strut segment length.

According to some examples, the film width in a pre-shrunk state thereof, is greater than the strut width plus twice the strut thickness.

According to some examples, the film width in a pre-shrunk state thereof, is greater than twice the strut width plus twice the strut thickness.

According to some examples, suturing comprises looping the skirt attachment suture in a whip stitch pattern.

According to some examples, the heat-shrink film is folded over a corresponding strut segment in a manner that defines a heat-shrink outer section disposed radially outward to the strut segment, heat shrink proximal and distal sections folded over proximal and distal edges of the strut segment, and first and second opposing heat-shrink inner sections folded therefrom and disposed radially inward to the strut segment.

According to some examples, the method further comprises: (a) passing the skirt attachment suture through the inner skirt and the first heat-shrink inner section, (b) extending the skirt attachment suture between the strut segment and the heat-shrink distal section toward and through the heat-shrink outer section, (c) folding the skirt attachment suture over the outer side of the heat-shrink outer section and passing it back through the heat-shrink outer section such that it further extends between the strut segment and the heat-shrink proximal section, forming a substantially U-shaped configuration around the strut segment, and (d) passing the skirt attachment suture back through the second heat-shrink inner section and the inner skirt, thereby forming a looped portion of the skirt attachment suture around the strut segment.

According to some examples, the method further comprises: (a) passing the skirt attachment suture through the inner skirt and the first heat-shrink inner section, (b) extending the skirt attachment suture between the strut segment and the heat-shrink distal section toward and through the heat-shrink outer section, (c) folding the skirt attachment suture over the outer side of the heat-shrink outer section and folding it back to extend over the external side of the heat-shrink proximal section, forming a substantially U-shaped configuration around the strut segment, and (d) passing the skirt attachment suture back through the inner skirt, thereby forming a looped portion of the skirt attachment suture around the strut segment.

According to some examples, suturing the skirt proximal end is performed following a zig-zag pattern of the strut segments

According to some examples, applying heat to the heat-shrink films comprises applying heat at a temperature of above 90°C.

According to some examples, applying heat to the heat-shrink sleeve comprises applying heat at a temperature of above 135°C.

According to yet another aspect of the invention, there is provided a method of assembling a prosthetic valve. The method comprises providing a prosthetic valve comprising frame movable between a radially compressed and a radially expanded configuration, an inner skirt having a skirt proximal end, and a leaflet assembly sutured to the inner skirt along a scallop line that tracks the lower edge of the leaflet assembly.

The method further comprises folding a plurality of heat-shrink films over a matching plurality of strut segments of the frame, wherein each heat-shrink film has a film length and a film width prior to folding thereof, and wherein each strut segment has a strut width, a strut thickness, and a strut segment length. The method further comprises forming at least one suture loop around each folded heat-shrink film. The method further comprises applying heat to the heat-shrink films, thereby causing them to shrink radially inward and to become tightly attached around the corresponding strut segments. The method further comprises suturing the inner skirt to the strut segments by passing at least one skirt attachment suture through the skirt proximal end and around the shrunken heat-shrink films.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, the film length is not greater than the strut segment length.

According to some examples, the film width in a pre-shrunk state thereof, is greater than the strut width plus twice the strut thickness.

According to some examples, the film width in a pre-shrunk state thereof, is greater than twice the strut width plus twice the strut thickness.

According to some examples, the heat-shrink film is folded over a corresponding strut segment in a manner that defines a heat-shrink outer section disposed radially outward to the strut segment, heat shrink proximal and distal sections folded over proximal and distal edges of the strut segment, and first and second opposing heat-shrink inner sections folded therefrom and disposed radially inward to the strut segment.

According to some examples, forming at least one suture loop comprises forming at least two suture loops around each folded heat-shrink film.

According to some examples, the two suture loops are positioned at opposite end portions of the folded heat-shrink film.

According to some examples, the perimeter of each suture loop is at least as great as the film width.

According to some examples, forming the suture loop comprises surrounding a suture around the folded heat-shrink film, and tying a knot between free ends thereof.

According to some examples, applying heat to the heat-shrink films comprises applying heat at a temperature of above 90°C.

According to some examples, applying heat to the heat-shrink sleeve comprises applying heat at a temperature of above 135°C.

According to some examples, the method further comprises cutting and removing the suture loops after applying heat to shrink the heat-shrink films.

According to some examples, the method further comprises cutting and removing the suture loops after suturing the inner skirt.

According to yet another aspect of the invention, there is provided a method of assembling a prosthetic valve. The method comprises providing a prosthetic valve comprising frame movable between a radially compressed and a radially expanded configuration, and an inner skirt having a skirt proximal end. The method further comprises disposing a plurality of C-shaped heat-shrink tubes over a matching plurality of strut segments of the frame, wherein each C-shaped heat-shrink tube has a tube length, a tube first diameter and a tube second diameter, and wherein each strut segment has a strut width, a strut thickness, and a strut segment length. The method further comprises applying heat to the C-shaped heat-shrink tubes, thereby causing them to shrink radially inward and to become tightly attached around the corresponding strut segments.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, disposing the C-shaped heat-shrink tubes over the strut segments comprises pushing each C-shaped heat-shrink tube at the side including a gap thereof, against the corresponding strut segment, until the strut segment is accommodated within the C-shaped heat-shrink tube.

According to some examples, the gap has a gap width that is smaller than the strut width and/or the strut thickness, in a free state of the C-shaped heat-shrink tube.

According to some examples, the tube length is not greater than the strut segment length.

According to some examples, the tube first diameter is at least as great as 120% of the strut width.

According to some examples, the tube first diameter is at least as great as 150% of the strut width.

According to some examples, the tube first diameter is at least as great as 200% of the strut width.

According to some examples, the tube second diameter is at least as great as 120% of the strut thickness.

According to some examples, the tube second diameter is at least as great as 150% of the strut thickness.

According to some examples, the tube second diameter is at least as great as 200% of the strut thickness.

According to some examples, tube second diameter is at least as great as 200% of the strut thickness.

According to some examples, applying heat to the heat-shrink sleeve comprises applying heat at a temperature of above 135°C.

According to some examples, the method further comprises suturing an inner skirt along a skirt proximal end thereof, to the C-shaped heat-shrink tubes, with at least one skirt attachment suture, performed prior to the step of applying heat.

According to some examples, each C-shaped heat-shrink tube is disposed over a corresponding strut segment such that it defines a heat-shrink outer section disposed radially outward to the strut segment, heat shrink proximal and distal sections folded over proximal and distal edges of the strut segment, and first and second opposing side arms disposed radially inward to the strut segment.

According to some examples, the method further comprises: (a) passing the skirt attachment suture through the inner skirt and the first side arm, (b) extending the skirt attachment suture between the strut segment and the heat-shrink distal section toward and through the heat-shrink outer section, (c) folding the skirt attachment suture over the outer side of the heat-shrink outer section and passing it back through the heat-shrink outer section such that it further extends between the strut segment and the heat-shrink proximal section, forming a substantially U-shaped configuration around the strut segment, and (d) passing the skirt attachment suture back through the second side arm and the inner skirt, thereby forming a looped portion of the skirt attachment suture around the strut segment.

According to some examples, the method further comprises: (a) passing the skirt attachment suture through the inner skirt and the first side arm, (b) extending the skirt attachment suture between the strut segment and the heat-shrink distal section toward and through the heat-shrink outer section, (c) folding the skirt attachment suture over the outer side of the heat-shrink outer section and folding it back to extend over the external side of the heat-shrink proximal section, forming a substantially U-shaped configuration around the strut segment, and (d) passing the skirt attachment suture back through the inner skirt, thereby forming a looped portion of the skirt attachment suture around the strut segment.

According to some examples, the method further comprises suturing an inner skirt to the strut segments, by passing at least one skirt attachment suture through a skirt proximal end of the inner skirt, and around the shrunken heat-shrink films.

According to yet another aspect of the invention, there is provided a method of assembling a prosthetic valve. The method comprises providing a prosthetic valve comprising frame movable between a radially compressed and a radially expanded configuration, the frame comprising a plurality of commissural support members, each commissural support member having a support member radial depth and a support member lateral width. The method further comprises disposing a heat-shrink sleeve over each commissural support member, wherein each heat-shrink sleeve has a sleeve length and a sleeve circular diameter, and wherein each commissural support member has support member length, a support member radial depth, and a support member lateral width.

The method further comprises applying heat to the heat-shrink sleeve, thereby causing it to shrink radially inward and to become tightly attached around the corresponding commissural support member. The method further comprises attaching a commissure assembly of a leaflet assembly to each commissural support member covered by the heat-shrink sleeve.

According to some examples, each commissural support member is an axial strut segment integrally formed with the frame.

According to some examples, the commissural support member has an exposed proximal end, and wherein disposing the heat-shrink sleeve over each commissural support member comprises sliding the heat-shrink sleeve from the exposed upper end over the corresponding commissural support member.

According to some examples, the leaflet assembly comprises a plurality of leaflets configured to regulate flow through the prosthetic valve, wherein each leaflet comprises a pair of oppositely-directed tabs, and wherein the tabs of adjacent leaflets are paired to form the commissure assemblies.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, applying heat to the heat-shrink sleeve comprises applying heat at a temperature of above 90°C.

According to some examples, applying heat to the heat-shrink sleeve comprises applying heat at a temperature of above 135°C.

According to some examples, the sleeve length is in the range of 90% to 110% of the support member length.

According to some examples, the sleeve length is greater than 110% of the support member length, and wherein the method further comprises a step of cutting the heat-shrink sleeve to a length that is not higher than 110% of the support member length prior to the step of applying heat.

According to some examples, the sleeve circular diameter is at least as great as 120% of the support member radial depth and/or of the support member lateral width.

According to some examples, the sleeve circular diameter is at least as great as 150% of the support member radial depth and/or of the support member lateral width.

According to some examples, the sleeve circular diameter is at least as great as 200% of the support member radial depth and/or of the support member lateral width.

According to yet another aspect of the invention, there is provided a method of assembling a prosthetic valve. The method comprises providing a prosthetic valve comprising frame movable between a radially compressed and a radially expanded configuration, the frame comprising a plurality of commissural support members, each commissural support member having a support member radial depth and a support member lateral width. The method further comprises folding a plurality of heat-shrink films over the matching plurality of commissural support members, wherein each heat-shrink film has a film length and a film width prior to folding thereof, and wherein each commissural support member has support member length, a support member radial depth, and a support member lateral width.

The method further comprises forming at least one suture loop around each folded heat-shrink film. The method further comprises applying heat to the heat-shrink films, thereby causing them to shrink radially inward and to become tightly attached around the corresponding commissural support member. The method further comprises attaching a commissure assembly of a leaflet assembly to each commissural support member covered by the heat-shrink film.

According to some examples, each commissural support member is an axial strut segment integrally formed with the frame.

According to some examples, each commissural support member is bound between angled strut segments of the frame.

According to some examples, the the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, the film length is not greater than the support member length.

According to some examples, the film width in a pre-shrunk state thereof, is greater than the support member lateral width plus twice the support member radial depth.

According to some examples, the film width in a pre-shrunk state thereof, is greater than twice the support member lateral width plus twice the support member radial depth.

According to some examples, forming at least one suture loop comprises forming at least two suture loops around each folded heat-shrink film.

According to some examples, the two suture loops are positioned at opposite end portions of the folded heat-shrink film.

According to some examples, the perimeter of each suture loop is at least as great as the film width.

According to some examples, forming the suture loop comprises surrounding a suture around the folded heat-shrink film, and tying a knot between free ends thereof.

According to some examples, applying heat to the heat-shrink sleeve comprises applying heat at a temperature of above 90°C.

According to some examples, applying heat to the heat-shrink sleeve comprises applying heat at a temperature of above 135°C.

According to some examples, the method further comprises cutting and removing the suture loops after applying heat to shrink the heat-shrink films.

According to some examples, the method further comprises cutting and removing the suture loops after attaching the commissure assembly.

According to yet another aspect of the invention, there is provided a method of assembling a prosthetic valve. The method comprises providing a prosthetic valve comprising frame movable between a radially compressed and a radially expanded configuration, the frame comprising a plurality of commissural support members, each commissural support member having a support member radial depth and a support member lateral width. The method further comprises disposing a plurality of C-shaped heat-shrink tubes over the matching plurality of commissural support members, wherein each C-shaped heat-shrink tube has a tube length, a tube first diameter and a tube second diameter, and wherein each commissural support member has support member length, a support member radial depth, and a support member lateral width.

The method further applying heat to the C-shaped heat-shrink tubes, thereby causing them to shrink radially inward and to become tightly attached around the corresponding commissural support member. The method further comprises attaching a commissure assembly of a leaflet assembly to each commissural support member covered by the heat-shrink film.

According to some examples, each commissural support member is an axial strut segment integrally formed with the frame.

According to some examples, each commissural support member is bound between angled strut segments of the frame.

According to some examples, the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

According to some examples, disposing the C-shaped heat-shrink tubes over the commissural support members comprises pushing each C-shaped heat-shrink tube at the side including a gap thereof, against the corresponding commissural support member, until the commissural support member is accommodated within the C-shaped heat-shrink tube.

According to some examples, the gap has a gap width that is smaller than the strut width and/or the strut thickness, in a free state of the C-shaped heat-shrink tube.

According to some examples, the tube length is not greater than the support member length.

According to some examples, the tube first diameter is at least as great as 120% of the of the support member lateral width.

According to some examples, the tube first diameter is at least as great as 150% of the of the support member lateral width.

According to some examples, the tube first diameter is at least as great as 200% of the of the support member lateral width.

According to some examples, the tube second diameter is at least as great as 120% of the of the support member radial depth.

According to some examples, the tube second diameter is at least as great as 150% of the of the support member radial depth.

According to some examples, the tube second diameter is at least as great as 200% of the of the support member radial depth.

According to some examples, applying heat to the heat-shrink films comprises applying heat at a temperature of above 90°C.

According to some examples, applying heat to the heat-shrink sleeve comprises applying heat at a temperature of above 135°C.

According to yet another aspect of the invention, there is provided a prosthetic valve. The prosthetic valve comprises a frame movable between a radially compressed and a radially expanded configuration. The frame comprises a plurality of strut segments interconnected at junctions. The prosthetic valve further comprises a leaflet assembly mounted within the frame and comprising a plurality of leaflets configured to regulate flow through the prosthetic valve, wherein the leaflet assembly is coupled to the frame along a scallop line that tracks the lower edge of the leaflet assembly. The prosthetic valve further comprises a plurality of pads covering at least some of the strut segments, wherein each pad has an outer surface having a surface roughness Ra value of 0.2 µm or less.

According to some examples, the strut segments comprise a plurality of angled strut segments covered by the pads.

According to some examples, the strut segments comprise a plurality of axially extending strut segments covered by the pads.

According to some examples, each pad comprises an attachment line facing radially outward from the frame.

According to some examples, each pad comprises a base layer and a coating layer, wherein the coating layer defines the outer surface.

According to some examples, each strut segment has an elliptic cross-section, wherein the strut width is a first diameter of the cross-section, wherein the base layer defines an inner surface facing the corresponding strut segment, and wherein the strut thickness is a second diameter of the cross-section.

According to some examples, all of the strut segments covered by the pads are proximal to the scallop line.

According to some examples, the plurality of pads further comprise a plurality of junction-pads covering at least portions of the junctions.

According to some examples, the outer surface of each pad has a durometer hardness ranging from about 40 Shore A to about 98 Shore A.

According to some examples, the outer surface of each pad has a durometer hardness ranging from about 0 Shore D to about 55 Shore D.

According to some examples, the outer surface of each pad has an abrasion resistance value of less than about 0.05%.

According to some examples, the outer surface of each pad has a kinetic coefficient of friction in the range of about 0.3-1.7.

According to some examples, the outer surface of each pad has a water absorption capacity of less than 1.5%.

According to some examples, each pad has a Vicat softening temperature selected from the range of about 150-180 °C.

According to some examples, each pad has an MFI in the range of 5-6 g/10 min.

According to some examples, each pad has a flexural strength in the range of about 5,000-10,000 psi.

According to some examples, each pad has a tensile strength in the range of about 6,000-9,000 psi.

According to some examples, each pad has a tensile strain at the failure point of about 200-500 %.

According to some examples, each pad has a secant tensile modulus in the range of about 700-2,400 psi, for a tensile strain of about 100 % or 300 %.

According to some examples, each pad is biodurable.

According to some examples, each pad comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, each pad comprises TPU.

According to yet another aspect of the invention, there is provided a prosthetic valve. The prosthetic valve comprises a frame movable between a radially compressed and a radially expanded configuration. The frame comprises a plurality of strut segments interconnected at junctions. The prosthetic valve further comprises a leaflet assembly mounted within the frame and comprising a plurality of leaflets configured to regulate flow through the prosthetic valve, wherein the leaflet assembly is coupled to the frame along a scallop line that tracks the lower edge of the leaflet assembly. The prosthetic valve further comprises a plurality of pads covering at least some of the strut segments, wherein each pad has an outer surface having a surface roughness Ra value that is 80% or less of the Ra value of a surface of the frame.

According to some examples, each pad has a Ra value that is 50% or less of the Ra value of the surface of the frame.

According to some examples, the strut segments comprise a plurality of angled strut segments covered by the pads.

According to some examples, the strut segments comprise a plurality of axially extending strut segments covered by the pads.

According to some examples, each pad comprises an attachment line facing radially outward from the frame.

According to some examples, each pad comprises a base layer and a coating layer, wherein the coating layer defines the outer surface.

According to some examples, each strut segment has an elliptic cross-section, wherein the strut width is a first diameter of the cross-section, wherein the base layer defines an inner surface facing the corresponding strut segment, and wherein the strut thickness is a second diameter of the cross-section.

According to some examples, all of the strut segments covered by the pads are proximal to the scallop line.

According to some examples, the plurality of pads further comprise a plurality of junction-pads covering at least portions of the junctions.

According to some examples, the outer surface of each pad has a durometer hardness ranging from about 40 Shore A to about 98 Shore A.

According to some examples, the outer surface of each pad has a durometer hardness ranging from about 0 Shore D to about 55 Shore D.

According to some examples, the outer surface of each pad has an abrasion resistance value of less than about 0.05%.

According to some examples, the outer surface of each pad has a kinetic coefficient of friction in the range of about 0.3-1.7.

According to some examples, the outer surface of each pad has a water absorption capacity of less than 1.5%.

According to some examples, each pad has a Vicat softening temperature selected from the range of about 150-180 °C.

According to some examples, each pad has an MFI in the range of 5-6 g/10 min.

According to some examples, each pad has a flexural strength in the range of about 5,000-10,000 psi.

According to some examples, each pad has a tensile strength in the range of about 6,000-9,000 psi.

According to some examples, each pad has a tensile strain at the failure point of about 200-500 %.

According to some examples, each pad has a secant tensile modulus in the range of about 700-2,400 psi, for a tensile strain of about 100 % or 300 %.

According to some examples, each pad is biodurable.

According to some examples, each pad comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, each pad comprises TPU.

According to yet another aspect of the invention, there is provided a prosthetic valve. The prosthetic valve comprises a frame movable between a radially compressed and a radially expanded configuration. The frame comprises a plurality of strut segments interconnected at junctions. The prosthetic valve further comprises a leaflet assembly mounted within the frame and comprising a plurality of leaflets configured to regulate flow through the prosthetic valve by transitioning between a fully-open state and a closed state. The leaflet assembly is coupled to the frame along a scallop line that tracks the lower edge of the leaflet assembly. The prosthetic valve further comprises a plurality of pads covering at least some of the strut segments, wherein each pad comprises an outer surface, wherein the leaflets are configured to at least partially contact the pads in the fully-open state.

According to some examples, the strut segments comprise a plurality of angled strut segments covered by the pads.

According to some examples, the strut segments comprise a plurality of axially extending strut segments covered by the pads.

According to some examples, each pad comprises an attachment line facing radially outward from the frame.

According to some examples, each pad comprises a base layer and a coating layer, wherein the coating layer defines the outer surface.

According to some examples, each strut segment has an elliptic cross-section, wherein the strut width is a first diameter of the cross-section, wherein the base layer defines an inner surface facing the corresponding strut segment, and wherein the strut thickness is a second diameter of the cross-section.

According to some examples, all of the strut segments covered by the pads are proximal to the scallop line.

According to some examples, the plurality of pads further comprise a plurality of junction-pads covering at least portions of the junctions.

According to some examples, the outer surface of each pad has a surface roughness Ra value of 0.2 µm or less.

According to some examples, the outer surface of each pad has a surface roughness Ra value that is 80% or less of the Ra value of a surface of the frame. According to further examples, each pad has a Ra value that is 50% or less of the Ra value of the surface of the frame.

According to some examples, the outer surface of each pad has a durometer hardness ranging from about 40 Shore A to about 98 Shore A.

According to some examples, the outer surface of each pad has a durometer hardness ranging from about 0 Shore D to about 55 Shore D.

According to some examples, the outer surface of each pad has an abrasion resistance value of less than about 0.05%.

According to some examples, the outer surface of each pad has a kinetic coefficient of friction in the range of about 0.3-1.7.

According to some examples, the outer surface of each pad has a water absorption capacity of less than 1.5%.

According to some examples, each pad has a Vicat softening temperature selected from the range of about 150-180 °C.

According to some examples, each pad has an MFI in the range of 5-6 g/10 min.

According to some examples, each pad has a flexural strength in the range of about 5,000-10,000 psi.

According to some examples, each pad has a tensile strength in the range of about 6,000-9,000 psi.

According to some examples, each pad has a tensile strain at the failure point of about 200-500 %.

According to some examples, each pad has a secant tensile modulus in the range of about 700-2,400 psi, for a tensile strain of about 100 % or 300 %.

According to some examples, each pad comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, each pad comprises TPU.

According to yet another aspect of the invention, there is provided a prosthetic valve. The prosthetic valve comprises a frame movable between a radially compressed and a radially expanded configuration. The frame comprises a plurality of strut segments interconnected at junctions, and defines an inner frame surface and an outer frame surface. The prosthetic valve further comprises a leaflet assembly mounted within the frame and comprising a plurality of leaflets configured to regulate flow through the prosthetic valve, wherein the leaflet assembly is coupled to the frame along a scallop line that tracks the lower edge of the leaflet assembly. The prosthetic valve further comprises a coating covering the inner frame surface or at least a portion thereof, wherein the coating forms a polymeric covering layer characterized by having a surface roughness Ra value of 0.2 µm or less.

According to some examples, the coating covers the portion of the inner frame surface which is proximal to the scallop line.

According to some examples, the coating further covers the outer frame surface or a portion thereof.

According to some examples, the polymeric covering layer forming the coating is characterized by having a durometer hardness ranging from about 40 Shore A to about 98 Shore A.

According to some examples, the polymeric covering layer forming the coating is characterized by having a durometer hardness ranging from about 0 Shore D to about 55 Shore D.

According to some examples, the polymeric covering layer forming the coating is characterized by having an abrasion resistance value of less than about 0.05%.

According to some examples, the polymeric covering layer forming the coating is characterized by having a kinetic coefficient of friction in the range of about 0.3-1.7.

According to some examples, the polymeric covering layer forming the coating is characterized by having a water absorption capacity of less than 1.5%.

According to some examples, the polymeric covering layer forming the coating is characterized by having a Vicat softening temperature selected from the range of about 150-180 °C.

According to some examples, the polymeric covering layer forming the coating is characterized by having an MFI in the range of 5-6 g/10 min.

According to some examples, the polymeric covering layer forming the coating is characterized by having a tensile strength in the range of about 6,000-9,000 psi.

According to some examples, each pad has a tensile strain at the failure point of about 200-500 %.

According to some examples, wherein each pad has a secant tensile modulus in the range of about 700-2,400 psi, for a tensile strain of about 100 % or 300 %.

According to some examples, the coating is non-biodegradable.

According to some examples, the coating is non-porous.

According to some examples, the coating is biodurable.

According to some examples, the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, the coating comprises TPU.

According to some examples, the coating covering the frame is applied by a method comprising: (a) coating strut segments of the frame using a first polymeric layer, and (b) coating the first polymeric layer using a second polymeric layer configured to bond with the first polymeric layer without bonding with the junctions.

According to yet another aspect of the invention, there is provided a prosthetic valve. The prosthetic valve comprises a frame movable between a radially compressed and a radially expanded configuration. The frame comprises a plurality of strut segments interconnected at junctions, and defines an inner frame surface and an outer frame surface. The prosthetic valve further comprises a leaflet assembly mounted within the frame and comprising a plurality of leaflets configured to regulate flow through the prosthetic valve, wherein the leaflet assembly is coupled to the frame along a scallop line that tracks the lower edge of the leaflet assembly. The prosthetic valve further comprises a coating covering the inner frame surface or at least a portion thereof, wherein the coating forms a polymeric covering layer characterized by having a surface roughness Ra value that is 80% or less of the Ra value of a surface of the frame.

According to some examples, the polymeric covering layer forming the coating has a Ra value that is 50% or less of the Ra value of the surface of the frame.

According to some examples, the coating covers the portion of the inner frame surface which is proximal to the scallop line.

According to some examples, the coating further covers the outer frame surface or a portion thereof.

According to some examples, the polymeric covering layer forming the coating is characterized by having a durometer hardness ranging from about 40 Shore A to about 98 Shore A.

According to some examples, the polymeric covering layer forming the coating is characterized by having a durometer hardness ranging from about 0 Shore D to about 55 Shore D.

According to some examples, the polymeric covering layer forming the coating is characterized by having an abrasion resistance value of less than about 0.05%.

According to some examples, the polymeric covering layer forming the coating is characterized by having a kinetic coefficient of friction in the range of about 0.3-1.7.

According to some examples, the polymeric covering layer forming the coating is characterized by having a water absorption capacity of less than 1.5%.

According to some examples, the polymeric covering layer forming the coating is characterized by having a Vicat softening temperature selected from the range of about 150-180 °C.

According to some examples, the polymeric covering layer forming the coating is characterized by having an MFI in the range of 5-6 g/10 min.

According to some examples, the polymeric covering layer forming the coating is characterized by having a tensile strength in the range of about 6,000-9,000 psi.

According to some examples, each pad has a tensile strain at the failure point of about 200-500 %.

According to some examples, wherein each pad has a secant tensile modulus in the range of about 700-2,400 psi, for a tensile strain of about 100 % or 300 %.

According to some examples, the coating is non-biodegradable.

According to some examples, the coating is non-porous.

According to some examples, the coating is biodurable.

According to some examples, the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, the coating comprises TPU.

According to some examples, the coating covering the frame is applied by a method comprising: (a) coating strut segments of the frame using a first polymeric layer, and (b) coating the first polymeric layer using a second polymeric layer configured to bond with the first polymeric layer without bonding with the junctions.

According to yet another aspect of the invention, there is provided a method of assembling a prosthetic valve. The method comprises (a) providing a prosthetic valve comprising a frame movable between a radially compressed and a radially expanded configuration. The frame comprises a plurality of strut segments interconnected at junctions, and defines an inner frame surface and an outer frame surface.

The method further comprises (b) coating the frame via dip coating. The coating comprises immersing the frame in a solution comprising a coating material. The coating further comprises extracting the frame from within the coating material solution, wherein a thin coating layer forms on the surface of the frame during the extraction thereof. The coating further comprises solidifying the coating layer formed on the surface of the frame, thereby forming the coating thereon. The coating is characterized by having a surface roughness Ra value of 0.2 µm or less and/or a Ra value that is 80% or less of the Ra value of a surface of the frame.

The method further comprises (c) mounting within the frame a leaflet assembly. The leaflet assembly comprises a plurality of leaflets. The leaflet assembly is coupled to the frame along a scallop line that tracks the lower edge of the leaflet assembly.

According to some examples, the Ra value of the coating is 50% or less of the Ra value of the surface of the frame.

According to some examples, the coating covers both of the inner and outer frame surfaces.

According to some examples, step (a) further comprises masking a portion of the frame, so that the frame is provided having a first unmasked portion and a second masked portion, wherein the first unmasked portion comprises a portion of the frame which is proximal to the designated scallop line along which leaflet assembly is to be coupled to the frame in step (c).

According to some examples, step (b) comprises forming a coating layer on the first unmasked portion and the second masked portion comprising the masking layer, during the extraction of the frame from within the coating material solution. According to some examples, step (b) further comprises removing the masking layer from the second masked portion following the formation of the solidified coating on the first unmasked portion.

According to some examples, the masking of the second masked portion comprises utilizing a removable adhesive layer.

According to some examples, the coating is characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

According to some examples, the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, the coating comprises TPU.

According to yet another aspect of the invention, there is provided a method of assembling a prosthetic valve. The method comprises (a) providing a prosthetic valve comprising a frame movable between a radially compressed and a radially expanded configuration. The frame comprises a plurality of strut segments interconnected at junctions, and defines an inner frame surface and an outer frame surface.

The method further comprises (b) spray-coating the surface of the frame or a portion thereof. The spray-coating comprises positioning a spray nozzle internally within the inner frame surface, thereby forming a coating layer thereon. The coating is characterized by having a surface roughness Ra value of 0.2 µm or less and/or a Ra value that is 80% or less of the Ra value of a surface of the frame.

The method further comprises (c) mounting within the frame a leaflet assembly. The leaflet assembly comprises a plurality of leaflets. The leaflet assembly is coupled to the frame along a scallop line that tracks the lower edge of the leaflet assembly.

According to some examples, step (b) further comprises spray-coating the surface of the outer frame surface, or a portion thereof, by positioning the spray nozzle externally to the outer frame surface, thereby forming a coating layer thereon.

According to some examples, step (b) further comprises rotating at least one of the frame, the spray nozzle, or both during the coating process, in order to create a uniform coating around or along frame.

According to some examples, the Ra value of the coating is 50% or less of the Ra value of the surface of the frame.

According to some examples, step (a) further comprises masking a plurality of junctions of the frame utilizing removable adhesive layers wrapped therearound, wherein following the spray-coating of step (b) the masking layers are removed from the junctions, resulting in coating solely the strut segments of the frame.

According to some examples, step (a) further comprises masking a portion of the frame utilizing a removable adhesive layer thereby forming a masked portion and an unmasked portion thereof, wherein following the spray-coating of step (b) the masking layer is removed from the masked portion, resulting in coating solely of the unmasked portion, and wherein the unmasked portion of the frame is configured to be contacted at least partially by the leaflets of step (c).

According to some examples, step (b) comprises targeting the coating nozzle to coat solely a desired portion of the frame, wherein said desired portion is configured to be contacted at least partially by the leaflets of step (c).

According to some examples, the coating is characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

According to some examples, the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, the coating comprises TPU.

According to yet another aspect of the invention, there is provided a method of assembling a prosthetic valve. The method comprises (a) providing a prosthetic valve comprising a frame movable between a radially compressed and a radially expanded configuration. The frame comprises a plurality of strut segments interconnected at junctions, and defines an inner frame surface and an outer frame surface.

The method further comprises (b) situating an inner coating layer around a mandrel.

The method further comprises (c) situating the frame of step (a) around the inner coating layer of step (b), thereby contacting the inner frame surface therewith.

The method further comprises (d) laminating the inner coating layer to the inner surface of the frame.

The method further comprises (e) removing the laminated frame of step (d) from the mandrel.

The method further comprises (f) mounting within the frame a leaflet assembly comprising a plurality of leaflets. The leaflet assembly is coupled to the frame along a scallop line that tracks the lower edge of the leaflet assembly.

The inner coating layer is characterized by having a surface roughness Ra value of 0.2 µm or less and/or a Ra value that is 80% or less of the Ra value of a surface of the frame.

According to some examples, the inner coating layer is in the form of a pre-fabricated thin polymeric sheet that is applied by being wrapped around the mandrel.

According to some examples, the inner coating layer is applied to the mandrel by dip coating, spray-coating, electrospinning, or a combination thereof.

According to some examples, the inner coating layer has a Ra value that is 50% or less of the Ra value of the surface of the frame.

According to some examples, step (c) further comprises situating an outer coating layer around the frame, thereby contacting the outer frame surface therewith.

According to some examples, the outer coating layer is in the form of a pre-fabricated thin polymeric sheet that is applied by being wrapped around the frame.

According to some examples, the outer coating layer is applied to the frame by dip coating, spray-coating, electrospinning, or a combination thereof.

According to some examples, step (d) further comprises laminating the inner and outer coating layers to the frame or to each other, so that the frame becomes encapsulated between.

According to some examples, step (c) further comprises situating an outer skirt on the outer coating layer. According to some examples, step (d) further comprises laminating the inner and outer coating layers to the frame or to each other, and laminating the outer skirt to the outer coating layer, so that the outer skirt becomes bonded to the outer coating layer and the frame becomes encapsulated between the inner and outer coating layers.

According to some examples, step (c) further comprises situating an outer skirt around the frame, thereby contacting the outer frame surface therewith. According to some examples, step (d) further comprises laminating the outer skirt to the inner coating layer, so that the outer skirt becomes bonded directly to the inner coating layer, through openings defined between the strut segments.

According to some examples, the lamination of step (d) is performed by the application of at least one of heat, pressure, ultrasonic welding, adhesives, or a combination thereof.

According to some examples, the lamination of step (d) comprises pressure applied externally to the frame by utilizing at least one of a clamp, a crimper, a pincer, or a combination thereof.

According to some examples, the lamination of step (d) comprises pressure applied internally to the frame by utilizing an inflatable or an expandable mandrel.

According to some examples, at least one of the inner and outer coating layers comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

According to some examples, at least one of the inner and outer coating layers are characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the invention will become more apparent from the following detailed description, which proceeds with reference to the accompanying figures.

### BRIEF DESCRIPTION OF THE FIGURES

Some examples of the invention are described herein with reference to the accompanying figures. The description, together with the figures, makes apparent to a person having ordinary skill in the art how some examples may be practiced. The figures are for the purpose of illustrative description and no attempt is made to show structural details of an example in more detail than is necessary for a fundamental understanding of the invention. For the sake of clarity, some objects depicted in the figures are not to scale.

### In the Figures:

Figs. 1A and 1B are views in perspective of a mechanically expandable prosthetic valve with and without soft components of the valve, respectively, according to some examples.
Fig. 2 is a view in perspective of a commissure assembly mounted over a commissural support member, according to some examples.
Figs. 3A-3D show stages of a method for covering a commissural support member with a heat-shrink sleeve, followed by mounting a commissure assembly thereover, according to some examples.
Fig. 4 is an enlarged view of an inner skirt attached to a frame, according to some examples.
Figs. 5A-5C show stages of a method of covering a strut segment with a heat-shrink film, followed by suturing a skirt thereover, according to some examples.
Figs. 6A-6B show stages of another method of covering a strut segment with a heat-shrink film, followed by suturing a skirt thereover, according to some examples.
Figs. 7A-7C show stages of another method of covering a strut segment with a heat-shrink film, followed by suturing a skirt thereover, according to some examples.
Figs. 8A-8D show stages of a method of covering a strut segment with a C-shaped heat-shrink tube, followed by suturing a skirt thereover, according to some examples.
Fig. 9 is a view in perspective of a frame with open-ended axially extending strut segments serving as commissural support members, according to some examples.
Figs. 10A-10D show stages of a method for covering an open-ended axially extending strut segment with a heat-shrink sleeve, followed by mounting a commissure assembly thereover, according to some examples.
Fig. 11 is a view in perspective of a frame with axially extending strut segments serving as commissural support members, bound between angled struts on both ends of each axially extending strut segment, according to some examples.
Figs. 12A-12D show stages of a method for covering an axially extending strut segment with a heat-shrink film, followed by mounting a commissure assembly thereover, according to some examples.
Figs. 13A-13C show stages of a method for covering an axially extending strut segment with a C-shaped heat-shrink tube, according to some examples.
Fig. 14A-B are views in perspective of a balloon-expandable valve having commissure windows, with and without soft components of the valve, respectively, according to some examples.
Fig. 15 is an enlarged view of a commissure window with heat-shrink wraps, according to some examples.
Figs. 16A-16B show a top view of a prosthetic valve in two opening configurations of the leaflets, according to some examples.
Figs. 17A-17B show two stages of a method for covering a strut segment with a pad, according to some examples.
Fig. 18 shows an enlarged cut-away view in perspective of a strut segment covered by a coated pad, according to some examples.
Fig. 19 is a view in perspective of a frame having some of its strut segments covered by a plurality of pads, according to some examples.
Fig. 20 is an enlarged view of a portion of a frame having both strut segments and junctions covered by corresponding pads, according to some examples.
Figs. 21A-21D shows views in perspective of a frame having various portions thereof covered in a coating, according to some examples.
Figs. 22A-22B shows views in perspective of a frame encapsulated between inner and outer coating layers, according to some examples.
Fig. 23A-23C shows cross-sectional views of coated frames disposed around mandrels, according to some examples.
Fig. 24 is an enlarged view of a portion of a frame covered by polymeric coating layers, according to some examples.

### DETAILED DESCRIPTION OF SOME EXAMPLES

For purposes of this description, certain aspects, advantages, and novel features of the examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present, or problems be solved. The technologies from any example can be combined with the technologies described in any one or more of the other examples. In view of the many possible examples to which the principles of the disclosed technology may be applied, it should be recognized that the illustrated examples are only preferred examples and should not be taken as limiting the scope of the disclosed technology.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the terms "have" or "includes" means "comprises." As used herein, "and/or" means "and" or "or," as well as "and" and "or".

Directions and other relative references may be used to facilitate discussion of the drawings and principles herein, but are not intended to be limiting. For example, certain terms may be used such as "inner," "outer," "upper," "lower," "inside," "outside,", "top," "bottom," "interior," "exterior," "left," right," and the like. Such terms are used, where applicable, to provide some clarity of description when dealing with relative relationships, particularly with respect to the illustrated examples. Such terms are not, however, intended to imply absolute relationships, positions, and/or orientations. For example, with respect to an object, an "upper" part can become a "lower" part simply by turning the object over. Nevertheless, it is still the same part and the object remains the same.

Throughout the figures of the drawings, different superscripts for the same reference numerals are used to denote different examples of the same elements. Examples of the disclosed devices and systems may include any combination of different examples of the same elements. Specifically, any reference to an element without a superscript may refer to any alternative example of the same element denoted with a superscript. In order to avoid undue clutter from having too many reference numbers and lead lines on a particular drawing, some components will be introduced via one or more drawings and not explicitly identified in every subsequent drawing that contains that component.

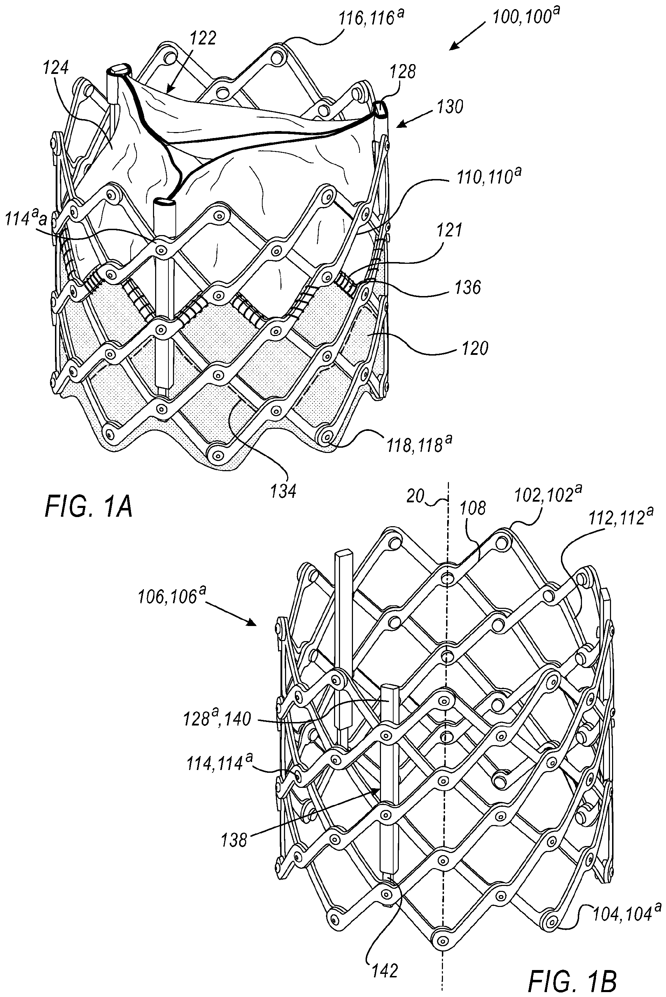

Figs. 1A and 1B show perspective views of an exemplary example of a prosthetic valve 100, with and without soft components (such as a skirt and a leaflet assembly), respectively. The term "prosthetic valve", as used herein, refers to any type of a prosthetic valve deliverable to a patient's target site over a catheter, which is radially expandable and compressible between a radially compressed, or crimped, state, and a radially expanded state. Thus, a prosthetic valve 100 can be crimped or retained by a delivery apparatus (not shown) in a compressed state during delivery, and then expanded to the expanded state once the prosthetic valve 100 reaches the implantation site. The expanded state may include a range of diameters to which the valve may expand, between the compressed state and a maximal diameter reached at a fully expanded state. Thus, a plurality of partially expanded states may relate to any expansion diameter between radially compressed or crimped state, and maximally expanded state. A prosthetic valve 100 of the current disclosure may include any prosthetic valve configured to be mounted within the native aortic valve, the native mitral valve, the native pulmonary valve, and the native tricuspid valve.

The term "plurality", as used herein, means more than one.

Figs. 1A-1B show a specific exemplary example of a mechanically expandable valve 100^{a}, illustrated in an expanded state. Mechanically expandable valves are a category of prosthetic valves that rely on a mechanical actuation mechanism for expansion. The mechanical actuation mechanism usually includes a plurality of expansion and locking assemblies, releasably coupled to respective actuation assemblies of a delivery apparatus, controlled via a handle for actuating the expansion and locking assemblies to expand the prosthetic valve to a desired diameter.

The terms coupled, engaged, connected and attached, as used herein, are interchangeable.

The terms "including" and/or "having", as used herein, are defined as comprising (i.e., open language).

The prosthetic valve 100 can comprise an inflow end 104 and an outflow end 102. In some instances, the outflow end 102 is the distal end of the prosthetic valve 100, and the inflow end 104 is the proximal end of the prosthetic valve 100. Alternatively, depending for example on the delivery approach of the valve, the outflow end can be the proximal end of the prosthetic valve, and the inflow end can be the distal end of the prosthetic valve.

The term "proximal", as used herein, generally refers to a position, direction, or portion of any device or a component of a device, which is closer to the user and further away from the implantation site.

The term "distal", as used herein, generally refers to a position, direction, or portion of any device or a component of a device, which is further away from the user and closer to the implantation site.

The term "outflow", as used herein, refers to a region of the prosthetic valve through which the blood flows through and out of the valve 100.

The term "inflow", as used herein, refers to a region of the prosthetic valve through which the blood flows into the valve 100.

The valve 100 comprises an annular frame 106 movable between a radially compressed configuration and a radially expanded configuration, and a leaflet assembly 122 mounted within the frame 106. The frame 106 can be made of various suitable materials, including plastically-deformable materials such as, but not limited to, stainless steel, a nickel based alloy (e.g., a cobalt-chromium or a nickel-cobalt-chromium alloy such as MP35N alloy), polymers, or combinations thereof. The frame can include a plurality of interconnected struts 110, wherein the struts can define strut segments 112 which are struts or segments of struts defined between junctions 114 of the frame 106.

According to some examples, the struts 110^{a} of a mechanically expandable valve 100^{a} are arranged in a lattice-type pattern. In the example illustrated in Fig. 1A-1B, the struts 110^{a} are positioned diagonally, or offset at an angle relative to, and radially offset from, the centerline 20 of the valve 100^{a}, when the valve 100^{a} is in an expanded state. It will be clear that the struts 110^{a} can be offset by other angles than those shown in Fig 1A-1B, such as being oriented substantially parallel to the centerline 20 of the valve 100^{a}.

According to some examples, the struts 110 are pivotably coupled to each other. In the exemplary example shown in Figs. 1A-1B, the end portions of the struts 110 are forming apices 116 at the outflow end 102 and apices 118 at the inflow end 104. The struts 110 can be coupled to each other at additional junctions 114 formed between the outflow apices 116 and the inflow apices 118. Both outflow and inflow apices 116, 118 constitute specific types of junctions 114, but for the sake of simplicity, reference to junction 114 throughout the current specification will refer to intermediate, non-apical junctions 114 positioned between the outflow apices 116 and the inflow apices 118, unless stated otherwise.

The junctions 114 can be equally spaced apart from each other, and/or from the apices 116, 118 along the length of each strut 110. Frame 106^{a} may comprise openings or apertures at the regions of apices 116^{a}, 118^{a} and junctions 114^{a} of the struts 110^{a}. Respective hinges can be included at locations where the apertures of struts 110^{a} overlap each other, via fasteners such as rivets or pins, which extend through the apertures. The hinges can allow the struts 110^{a} to pivot relative to one another as the frame 106^{a} is radially expanded or compressed.

In alternative examples, the struts 110 are not coupled to each other via respective hinges, but are otherwise pivotable or bendable relative to each other, so as to permit frame expansion or compression. For example, the frame can be formed from a single piece of material, such as a metal tube, via various processes such as, but not limited to, laser cutting, electroforming, and/or physical vapor deposition, while retaining the ability to collapse/expand radially in the absence of hinges and like. In some examples, the frame can comprise a braided structure braided from one or more metal wires.

The frame 106 further comprises a plurality of cells 108, defined between intersecting portions of struts 110. The shape of each cell 108, and the angle between intersecting portions of struts 110 defining the cell borders, vary during expansion or compression of the prosthetic valve 100. Further details regarding the construction of the frame and the prosthetic valve are described in U.S. Publication Nos. 2018/0153689; 2018/0344456; 2019/0060057 all of which are incorporated herein by reference.

The leaflet assembly 122 comprises a plurality of leaflets 124 (e.g., three leaflets), positioned at least partially within the frame 106, and configured to regulate flow of blood through the prosthetic valve 100 from the inflow end 104 to the outflow end 102. While three leaflets 124 arranged to collapse in a tricuspid arrangement similar to the native aortic valve, are shown in the exemplary example illustrated in Fig. 1A, it will be clear that a prosthetic valve 100 can include any other number of leaflets 124, such as two leaflets configured to collapse in a bicuspid arrangement similar to the native mitral valve, or more than three leaflets, depending upon the particular application. The leaflets 124 are made of a flexible material, derived from biological materials (e.g., bovine pericardium or pericardium from other sources), bio-compatible synthetic materials, or other suitable materials as known in the art and described, for example, in U.S. Pat. Nos. 6,730,118, 6,767,362 and 6,908,481, which are incorporated by reference herein.

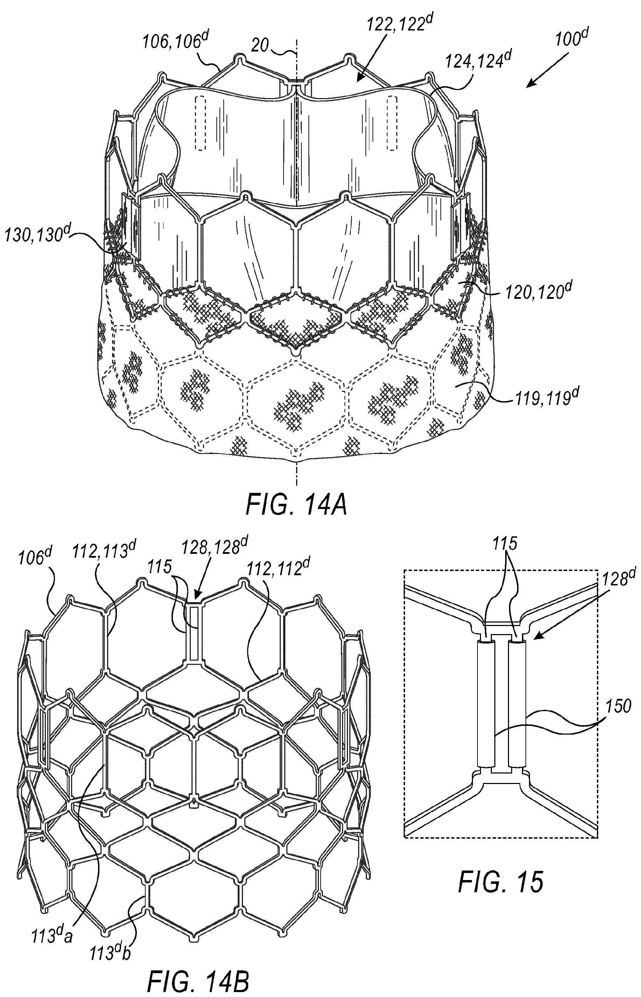

According to some examples, the prosthetic valve may further comprise at least one skirt or sealing member. An inner skirt 120 can be secured to the inner surface of the frame 106, configured to function, for example, as a sealing member to prevent or decrease perivalvular leakage. An inner skirt 120 can further function as an anchoring region for the leaflets 124 to the frame 106, and/or function to protect the leaflets 124 against damage which may be caused by contact with the frame 106, for example during valve crimping or during working cycles of the prosthetic valve 100. Additionally, or alternatively, the prosthetic valve 100 can comprise an outer skirt 119 (shown for example in Fig. 14A) mounted on the outer surface of the frame 106, configure to function, for example, as a sealing member retained between the frame 106 and the surrounding tissue of the native annulus against which the prosthetic valve is mounted, thereby reducing risk of paravalvular leakage past the prosthetic valve 100. Any of the inner skirt 120 and/or outer skirt 119 can be made of various suitable biocompatible materials, such as, but not limited to, various synthetic materials (e.g., PET) or natural tissue (e.g. pericardial tissue). In some examples, the inner skirt 120 comprises a single sheet of material that extends continuously around the inner surface of the frame 106.

The inner skirt 120 can comprise a tough, tear resistant material. The thickness of the skirt desirably is less than 6 mil or 0.15 mm, and desirably less than 4 mil or 0.10 mm, and even more desirably about 2 mil or 0.05 mm. In some examples, the inner skirt 120 can have a variable thickness, for example, the skirt 120 can be thicker at its edges than at its center. In one implementation, the inner skirt 120 can comprise a PET skirt having a thickness of about 0.07 mm at its edges and about 0.06 mm at its center. The thinner skirt can provide for better crimping performances while still providing good perivalvular sealing.

Each leaflet 124 comprises a pair of oppositely-directed tabs 126, wherein each tab 126 of each leaflet 124 is paired with a tab 126 of an adjacent leaflet 124 to form commissure assemblies 122 that can be secured, directly or indirectly, to the frame 106 of prosthetic valve 100, and more specifically, to commissural support members 128 that can be either integrally formed with the frame 106, or provided as separate components attached to the frame 106. In some examples, portions of the tabs 126 of the commissure assemblies 122 can be at least partially wrapped around commissural support members 128, and secured thereto, for example with one or more commissural attachment sutures 132.

When secured to two other leaflets 124 to form leaflet assembly 122, distal cusp edges of the leaflets 124 collectively form the scalloped shaped lower edge portion of the leaflet assembly 122, which can be secured to the frame 106, directly or indirectly, for example, by being sutured to an inner skirt 120 of the valve 100. The leaflet assembly 122 can be sutured to the inner skirt 120 along a scallop line 134 that tracks the lower edge of the leaflet assembly 122. Further details regarding prosthetic valves, including the manner in which leaflets may be mounted to their frames, are described in U.S. Patent Nos. 7,393,360, 7,510,575, 7,993,394 and 8,252,202, and U.S. Patent Application No. 62/614,299, all of which are incorporated herein by reference.

According to some examples, a prosthetic valve 100, which can be a mechanical prosthetic valve 100^{a}, comprises a plurality of expansion and locking assemblies 138, configured to facilitate expansion of the valve 100^{a}, and in some instances, to lock the valve 100^{a} at an expanded state, preventing unintentional recompression thereof. Although Figs. 1A-1B illustrate three expansion and locking assemblies 138, mounted to the frame 106^{a}, and optionally equally spaced from each other around an inner surface thereof, it should be clear that a different number of expansion and locking assemblies 138 may be utilized, that the expansion and locking assemblies 138 can be mounted to the frame around its outer surface, and that the circumferential spacing between expansion and locking assemblies 138 can be unequal.

Each of the expansion and locking assemblies 138 generally includes an inner member 142 that may partially extend through a lumen of an outer member 140. The outer member 140 can be attached to the frame 106^{a} at a first location, such as an outflow apex 116^{a} or another junction 114^{a} along the outflow end portion, such as the proximal-most non-apical junction 114^{a}a, as shown in Fig. 1A. The inner member 142 can be attached to the frame 106^{a} at a second location, spaced apart from the first location, such as an inflow apex 118^{a} or another junction 114^{a} along the inflow end portion, such as a distal-most non-apical junction 114^{a}.

The inner members 142 and the outer members 140 can be moveable longitudinally relative to each other in a telescoping manner to radially expand and contract the frame 106, as further described in U.S. Publication No. 2018/0153689 incorporated by reference above. In some implementations, the inner member 142 and outer member 140 can telescope relative to each other between a compressed configuration and an expanded configuration of the prosthetic valve 100^{a}. Movement of the inner member in a first direction, relative to the outer member, causes the frame 106 to foreshorten axially and expand radially. The first direction can be, for example, a proximally oriented direction.

Actuators (not shown) of the delivery assembly can be releasably coupled to components of the expansion and locking assemblies 138, such as inner members 142, such that when the actuators are pulled, the inner members 142 are pulled therewith, preferably while holding the outer members 140 in place, thereby approximating the inflow end 104^{a} to the outflow end 102^{a} to facilitate valve expansion.

According to some examples, the prosthetic valve 100 can include a plurality of commissural support members 128 having a fixed length, to which commissure assemblies can be mounted. According to some examples, the commissural support members 128 are commissural posts that can be attached to the frame 106 and extend axially along the outflow end portion 102 of the valve 100. In other examples, the commissural support members 128 can be formed as integral axially-oriented portions of the frame 106.

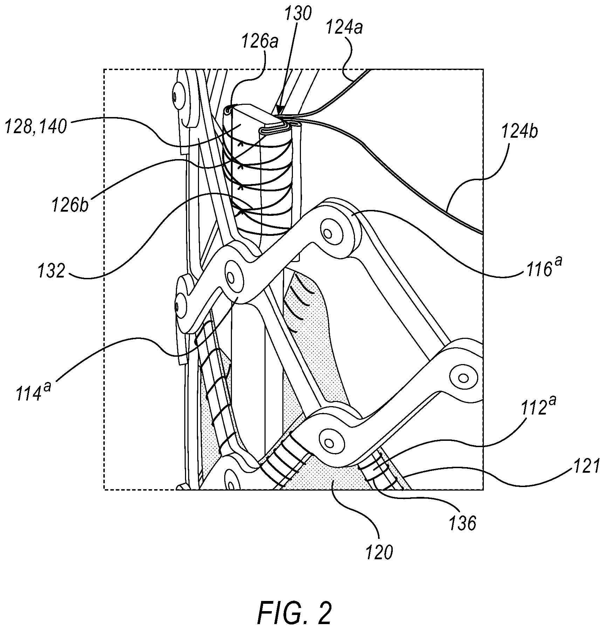

According to some examples, the outer members 140 of expansion and locking assemblies 138 of mechanically expandable valve 100^{a} further serve as commissural support members 128^{a}, as also shown in greater detail in Fig. 2. Commissural assemblies 130 can be secured to commissural support members 128, such as outer members 140, using suitable techniques and mechanisms. For example, tabs 126a and 126b of leaflets 124a and 124b, respectively, can be at least partially wrapped around a proximal portion of commissural support members 128^{a} (i.e., of housing 140) and secured thereto and to each other by a commissural attachment suture (one or more), as also shown in Fig. 2.

According to some examples, the outer member 140 is coupled to a proximal-most non-apical junction 114^{a}a, such that the portion of the outer member 140 extending in a first direction (e.g., a proximally) from the junction 114^{a}a, serves as the commissural support member 128^{a}, providing easy access for mounting the commissure assembly 130 thereto. This configuration may be advantageous since attachment of the outer member 140 to an outflow apex 116^{a}, for example, will result either in a portion of the housing 140 extending proximally therefrom to serve as a commissural support member 128^{a}, to protrude too far away from the outflow end 102. Similarly, either attaching the outer member 140 to another non-apical junction 114^{a}, which is distal to the proximal-most non-apical junction 114^{a}a, or attaching it to an outflow apex 116^{a} such that a portion of the housing that extends distally from the apex 116^{a} serves as the commissural support member, may be less convenient since in such cases, the designated commissural support member is bound between two junctions 114^{a}, or between a non-apical junction 114^{a} and an outflow apex 116^{a}, eliminating the advantageous ease of access for components of the commissure assembly 130 from above.

Nevertheless, in some implementations, a portion of the outer member 140 extending between two junctions, such as an outflow apex 116 and a non-apical junction 114 proximal thereto, can serve as a commissural support member 128 which is enclosed in both axial ends by such junctions.

The leaflets 124 of the leaflet assembly 122 constantly transition between a fully-open state, allowing blood flow through the valve, and a closed state, wherein the leaflet co-apt so as to prevent blood backflow. As such, the leaflet assembly 122 as a whole, and any portion thereof attached to the frame 106 directly or indirectly, such as the commissure assemblies 130 mounted on the commissural support members 128, may be subject to wear due to the relative ongoing movement of the soft components of the commissure assemblies 130 over the rigid, potentially metallic, commissural support members 128.

According to some examples, there is provided at least one protective cover configured to be disposed around various segments of the prosthetic valve 100, such as around at least a portion of a commissural support member 128 and/or around strut segments 112. According to some further examples, the protective cover comprises a heat-shrink wrap 150. According to some examples, there is provided at least one heat-shrink wrap 150 configured to be disposed around various segments of the prosthetic valve 100, such as around at least a portion of a commissural support member 128 and/or around strut segments 112.

As used herein, the term "heat-shrink wrap" refers to a shrinkable material that is configured to be disposed around various segments of a prosthetic valve 100, such as around at least a portion of a commissural support member 128 and/or around strut segments 112, in order to provide abrasion resistance and prolong long-term durability of soft components attached to such commissural support members 128 and/or around strut segments 112. The heat-shrink wrap can be configured to shrink radially inward from a substantially tubular form it may have, when heat is applied thereto, to a final diameter that ranges from about 50% to about 10% of its original diameter, thereby effectively tightly covering the commissural support member 128 and/or strut segment 112 it is shrunk over.

The term "tightly shrunk" or "tightly attached", with reference to a heat-shrink wrap, are interchangeable and refer to a heat-shrink wrap having at least a majority of an inner surface thereof in direct physical contact with a component covered thereby, such as a strut segment or a commissural support member.

As used herein, the term "about" modifies a particular value, by referring to a range equal to the particular value, plus or minus twenty percent (+/-10%). For any of the examples of flexible containers, disclosed herein, any disclosure of a particular value, can, in various alternate examples, also be understood as a disclosure of a range equal to about that particular value (i.e. +/-10%).

According to further examples, the protective cover comprises a pad 170. The term "pad", as used herein, refers to sheet of materials or a sleeve, which can be formed from a sheet of material, that does not include heat-shrinkable materials as described hereinabove. The sheet material for a pad 170 may be, for example only, a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof. Each possibility represents a different example. The pad 170 may be made of a biocompatible material. As mentioned, the sheet may be rolled into a suitable cylindrical shape to serve as a pad 170 on the frame 106 of the prosthetic valve 100. The pads 170 may be secured through such methods as sewing, ultrasonic welding, using adhesives, melting, a combination thereof or other attachment methods known in the art. Alternatively, the pads may be applied directly to the frame, such as by electrospinning or targeted coating, for example. In one example, the pad is TPU material applied through coating.

According to some examples, the protective cover is flexible and is provided in a shape selected from a sleeve, a tube, a hollow cylinder, a sheet, a tape, and combinations thereof. Each possibility represents a different example. According to some examples, the heat-shrink wrap 150 is flexible and is provided in a shape selected from a sleeve, a tube, a hollow cylinder, a sheet, a tape, and combinations thereof. Each possibility represents a different example. According to some examples, the pad 170 is flexible and is provided in a shape selected from a sleeve, a tube, a hollow cylinder, a sheet, a tape, and combinations thereof. Each possibility represents a different example.

According to some examples, the heat-shrink wrap 150 is semi-rigid and is in a shape selected from C-shape, L-shape (90° bend), U-shape, and variations thereof. Each possibility represents a different example.

According to some examples, the heat-shrink wrap 150 comprises a plurality of layers, wherein each layer can comprise different or the same materials. According to some examples, the heat-shrink wrap 150 can further comprise at least one additional layer, wherein said additional layer comprises at least one of an additive, adhesive, and combinations thereof. Each possibility represents a different example.

According to some examples, the heat-shrink wrap 150 is configured to shrink radially inward when heat is applied thereon, thereby to become tightly attached to the segment of the prosthetic valve 100 it surrounds. According to further examples, the heat-shrink wrap 150 does not shrink longitudinally. According to further examples, the heat-shrink wrap 150 is configured to shrink longitudinally, to a final length that ranges from about above 90% to about 99% of its original length.

According to some examples, the heat-shrink wrap 150 is configured to shrink radially inward when heat is applied thereon at an elevated temperature of above about 90°C, wherein the temperature is dependent on the type of materials that the heat-shrink layer comprises therein. According to some examples, the heat-shrink wrap 150 is configured to shrink radially inward when heat is applied thereon at an elevated temperature of above about 135°C. According to further examples, the elevated temperature is selected from the range of about 90°C to about 250 °C. According to further examples, the elevated temperature is selected from the range of about 135°C to about 200°C.

According to some examples, a heat source is configured to apply uniform controlled heat on the heat-shrink wrap 150, wherein said heat source is selected from an oven, a hot air gun, other sources of hot gas flow, and combinations thereof. Each possibility represents a different example.

According to some examples, the heat-shrink wrap 150 comprises at least one biocompatible thermoplastic polymeric material selected from but not limited to: polyolefins, fluoropolymers, polyvinyl chloride (PVC), polychloroprenes (neoprenes), silicone elastomers, Viton^{®}, and combinations and variations thereof. According to some examples, the polyolefin is a polymer produced from an olefin as a monomer (i.e., an alkene having the general formula CₙH₂ₙ), wherein the polyolefin is selected from but not limited to, polyethylene (PE), polypropylene (PP), polymethylpentene (PMP), polybutene-1 (PB-1), and combinations and variations thereof. Each possibility represents a different example. According to some examples, the fluoropolymer is a fluorocarbon-based polymer selected from but not limited to, fluorinated ethylene-propylene (FEP), polyvinylidene fluoride (PVDF), polytetrafluoroethylene (PTFE, Teflon^{®}), and combinations and variations thereof. Each possibility represents a different example.

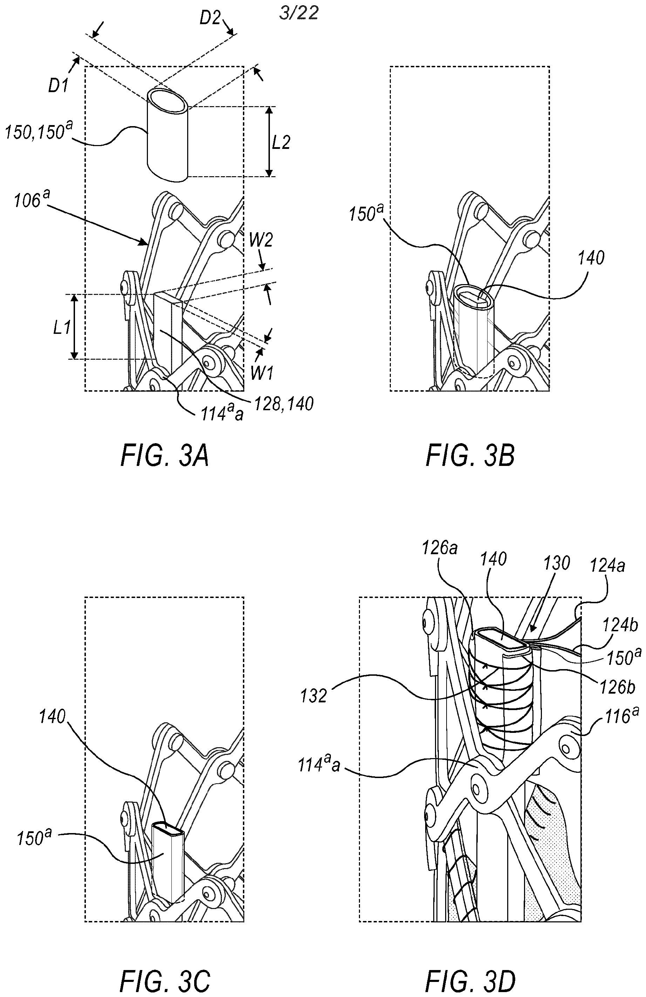

According to some examples, the heat-shrink wrap 150 is a heat-shrink sleeve 150^{a}, configured to be tightly shrunk over a commissure support member 128. Reference is now made to Figs. 3A-3D, showing stages of a method of assembling a prosthetic valve 100, and more specifically, stages of covering a commissural support member 128 with a heat-shrink sleeve 150^{a}, followed by mounting a commissure assembly 130 over the heat-shrink sleeve 150^{a} covering the commissural support member 128. The commissural support member 128 shown in Fig. 3A is formed by the portion of the outer member 140 extending proximally from a proximal-most non-apical junction 114^{a}a, having a length L1.

According to some examples, the heat-shrink sleeve 150^{a} has a length L2 that is at least as great as the length L1. The length L2 can be substantially equal to L1, such that once it is disposed over the commissural support member 128^{a} portion of outer member 140, it covers its entire length. In alternative implementations, the length L2 can be greater than L1, such that once it is disposed over the commissural support member 128^{a} portion of outer member 140, it extends beyond the proximal end of outer member 140, in which case it can be cut, for example by scissors, a knife, or any other suitable cutting means, at the level of the proximal end of outer member 140, resulting in the heat-shrink sleeve 150^{a} covering the exact length L1 of the commissural support member 128^{a} portion of outer member 140.

The term "substantially equal", as used herein, mean within a range of no more than ±10 percent of the referred measure. For example, the length L2 being substantially equal to the length L1, means that this length is in the range of 90% to 110% of L1.

In alternative examples, the heat-shrink sleeve 150^{a} has a length L2 that is shorter than the length L1, such that once it is disposed over the commissural support member 128^{a} portion of outer member 140, it covers only a portion thereof. Such examples may be applicable if the commissure assembly 140 is shorter than the length L1, or if a shorter length along which the heat-shrink sleeve 150^{a} covers the commissural support member 128^{a} is still considered to be enough to sufficiently reduce long-term wear of the soft components of the commissure assembly 140.

According to some examples, the inner cross-sectional dimensions of the heat-shrink sleeve 150^{a} are at least as great as, and preferably greater than, the outer cross-sectional dimensions of the commissural support member 128^{a} portion of outer member 140, so as to allow the heat-shrink sleeve 150^{a} to be easily disposed thereover. For example, the outer member 140 can have a maximal support member radial depth W1 and a maximal support member lateral width W2 as shown in Fig. 3A. For such cases, the heat-shrink sleeve 150^{a} can be provided with an elliptical cross-section having a sleeve first diameter D1, oriented in the radial direction, which is at least as great as, and preferably greater than W1, and a sleeve second diameter D2, oriented at the lateral direction, which is at least as great as, and preferably greater than W2.

The term "radial direction", as used herein, refers to a direction extending radially from the centerline 20 of the prosthetic valve 100 to the frame 106.

The term "lateral direction", as used herein, refers to a direction substantially orthogonal to the radial direction, for example along the circumference of the frame 106.

The sleeve diameters D1 and D2 refer to inner diameters defined by the inner surface of the heat-shrink sleeve 150^{a}. The support member radial depth W1 and lateral width W2 refer to external dimensions defined across the outer surface of the commissural support member 128^{a}.

According to some examples, the sleeve first diameter D1 is at least as great as 120% of the support member radial depth W1. According to some examples, the sleeve first diameter D1 is at least as great as 150% of the support member radial depth W1. According to some examples, the sleeve first diameter D1 is at least as great as 200% of the support member radial depth W1.

According to some examples, the sleeve second diameter D2 is at least as great as 120% of the support member lateral width W2. According to some examples, the sleeve second diameter D2 is at least as great as 150% of the support member lateral width W2. According to some examples, the sleeve second diameter D2 is at least as great as 200% of the support member lateral width W2.

The heat-shrink sleeve 150^{a} can be provided as a tubular sleeve having a circular cross section with a circular sleeve diameter De, that can be squeezed to an elliptical cross-sectional shape having the first and second diameters D1 and D2 as mentioned above.

It is to be understood that the dimensions L2, De, D1 and D2 of the heat-shrink sleeve 150^{a} relate to its initial, pre-shrunk state.

While the outer member 140 is shown in Figs. 3A-3D to have a rectangular cross-sectional shape, it is to be understood that this is shown by way of illustration and not limitation, and that the outer member 140 can have any other cross-sectional shape, such as square, triangular, pentagonal, hexagonal, octagonal, circular, elliptical, star-shaped, and the like. For example, the outer member can have an elliptical cross-section having a support member first diameter in a radial direction that is equal to the support member radial depth W1, and a support member second diameter in a lateral direction that is equal to the support member lateral width W1, wherein D1 is at least as great as, and preferably greater than W1, and wherein D2 is at least as great as, and preferably greater than W2, following the same relative dimensions described hereinabove.

In another example, the outer member 140 or other types of commissure posts serving as a commissural support member 128 can have a circular or elliptic outer cross-section defining a maximal support member diameter We, wherein a heat-shrink sleeve 150^{a} having a circular or elliptic cross-section defines a minimal sleeve diameter Dm which is at least as great as, and preferably greater than We.

If the commissural support member 128 has a circular cross-section defining an outer circular diameter, this circular diameter is the maximal support member diameter We. If the commissural support member 128 has an elliptical cross-section defining support member first and second diameters equal to W1 and W2 as mentioned above, the maximal support member diameter We is the larger of the two.

If the heat-shrink sleeve 150^{a} has a circular cross-section defining an inner circular sleeve diameter De, the minimal sleeve diameter Dm is the circular sleeve diameter De. If the heat-shrink sleeve 150^{a} has an elliptical cross-section defining the first and second diameters D1 and D2 as mentioned above, the minimal sleeve diameter Dm is the smaller of the two. Similarly, if the outer member 140 has a circular cross-section defining a circular diameter

According to some examples, the minimal sleeve diameter Dm is at least as great as 120% of the maximal support member diameter We. According to some examples, the minimal sleeve diameter Dm is at least as great as 150% of the maximal support member diameter We. According to some examples, the minimal sleeve diameter Dm is at least as great as 200% of the maximal support member diameter We.

As shown in Fig. 3B, the method includes a step of disposing the heat-shrink sleeve 150^{a} over the outer member 140. The configuration shown in Figs. 3A-3D, in which the outer member 140 has an exposed proximal end, advantageously allows the heat-shrink sleeve 150^{a} to be easily slid over the outer member 140 from above, preferably having inner dimensions that are sufficiently larger than the outer dimensions of the outer member 140 to enable it to be conveniently positioned over the outer member 140 with minimal effort. This advantage is similarly applicable for other types of commissural support member 128, such as various commissure posts, having an exposed proximal end.

The method further includes a step of applying heat to the heat-shrink sleeve 150^{a} utilizing a heat source as mentioned hereinabove, thereby causing it to shrink radially inward and to become tightly attached around the commissural support member 128^{a} portion of outer member 140, as shown in Fig. 3C. The commissure assembly 130 is then attached to the commissural support member 128^{a} covered by the heat-shrink sleeve 150^{a}, as shown in Fig. 3D. For example, the tabs 126a and 126b of adjacent leaflets 124a and 124b, respectively, can be at least partially wrapped around the heat-shrink sleeve 150^{a} covering the commissural support member 128^{a}, and commissural attachment sutures 132 can be utilized to stitch the tabs 126 to each other and to the commissural support member 128^{a} covered by the heat-shrink sleeve 150^{a}, as shown.

Advantageously, covering the commissural support member 128^{a} portion of outer member 140 with a heat-shrink wrap 150, such as heat-shrink sleeve 150^{a}, increases the long-term durability of the prosthetic valve 100 due to less wear from the commissural support members 128^{a} engaging soft components of the commissure assembly 130, such as tabs 126 of leaflets 124 and commissural attachment sutures 132.

While shown for an outer member 140 coupled to the frame 106 at a proximal-most non-apical junction 114^{a}a, such that the portion serving as the commissural support member 128^{a} extends proximally therefrom, it is to be understood that the method can be similarly applied to an outer member 140 coupled to the frame 106 at an outflow apex 116, in which case the portion serving as the commissural support member 128 extends proximally from the outflow apex 116. While such a commissural support member 128 extends proximally beyond the outflow end 102 of the frame 106, it's proximal end still remains open-ended, enabling the heat-shrink sleeve 150^{a} to be slid over the outer member 140 from above in a similar manner to that described hereinabove in conjunctions with Figs. 3A-3B.

While exemplified for an expansion and locking assembly 138 coupled to a frame 106 of a mechanically expandable valve 100, in which a portion of the outer member 140 serves as a commissural support member 128^{a}, it will be clear that the method can be similarly applied to other types of commissural support members 128 that may be coupled to the frame, such as commissure posts that can be attached to a frame of a prosthetic valve (100), which can be either a mechanically expandable valve, or any other type of prosthetic valve, including self-expandable valves and balloon expandable valves.

As mentioned above, the prosthetic valve can include an inner skirt 120 attached to the frame 106 such that it is disposed along the inner surface of the frame 106. The inner skirt 120 can be configured to serves as a flow barrier for preventing perivalvular leakage through cells 108 there-through into the lumen defined by the frame 106. The inner skirt can be further configured to serve as an attachment member for the undulating distal edges of the leaflet assembly 122 to the frame 106, by having them stitched thereto along the scallop line 134 as shown in Fig. 1, for example.

The inner skirt 120 can be sutured at its distal end to the inflow end 104, such as being stitch-locked to the inflow apices 118, and at its proximal end 121 to strut segments 112 of the frame 106, such as along a mid-region or any other region between the inflow end 104 and the outflow end 102.

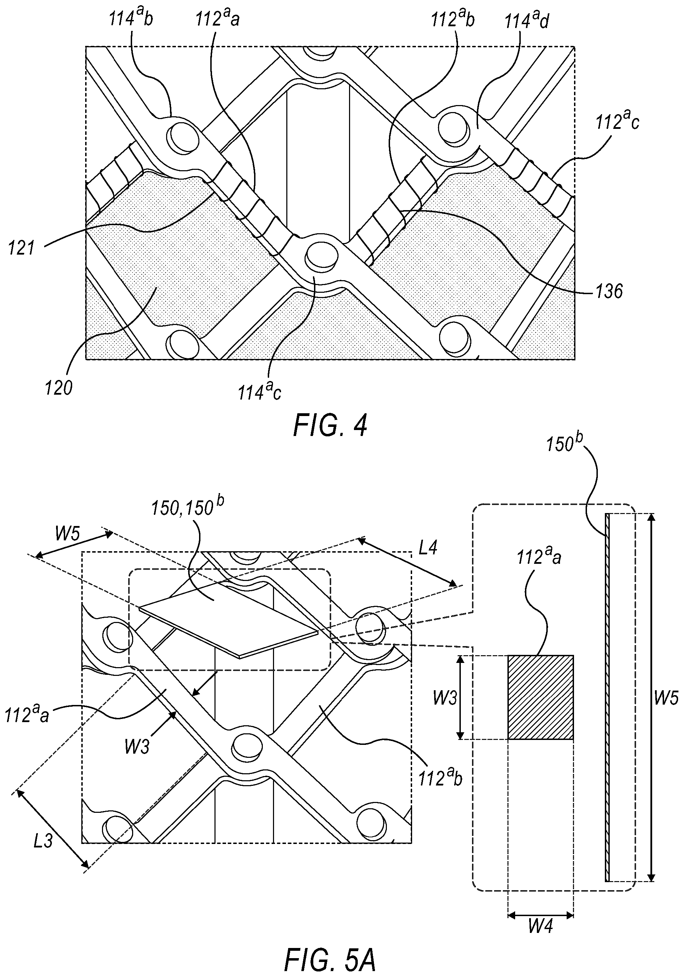

Fig. 4 shows an enlarged view of a portion of a region of the skirt 120 attached along its proximal end 121 to the frame 106. As shown, the skirt proximal end 121 can be sutured to struts segments 112 extending along the circumference of the frame 106 via at least one skirt attachment suture 136. The skirt proximal end 121 can be sutured to interconnected struts segments 112 following a zig-zag pattern, such as along strut segment 112^{a}a between junction 114^{a}b and junction 114^{a}c, then along struts segment 112^{a}b between junction 114^{a}c and junction 114^{a}d, continuing along strut segment 112^{a}c and so on, around the entire circumference of the frame 106. A single skirt attachment suture 136 can be stitched through the inner skirt 120 and along all of the strut segments 112 around the circumference of the frame 106, or a plurality of skirt attachment sutures 136 can be used, for example by stitching a separate suture 136 along each strut segment 112 between two junctions 114.

The leaflet assembly 122 can be attached to the inner skirt 120 along a scallop line 136 following an undulation pattern that is distal to the skirt proximal end 121. This separation between the leaflets 124 and the frame 106 may serve to protect leaflet material from direct contact with the frame 106, thereby reducing risk of leaflet abrasion against the struts 110. However, as the leaflet assembly 122 constantly transitions between closed and open states during diastolic and systolic phases, the leaflet assembly 122 may apply, via the scallop line 134, axial and/or radial forces to the skirt 120 that may be translated to the skirt's attachment to the frame 106, for example along the skirt attachment suture 136, which may similarly increase wear rates of the skirt attachment suture 136, as well as the skirt material along the skirt proximal end 121 that is in contact with the struts 110.

According to some examples, a plurality of heat-shrink wraps 150 cover the plurality of interconnected strut-segments 112 to which the inner skirt 120 it attached, configured to be tightly shrunk over the respective strut-segments 112. According to some examples, the heat-shrink wrap 150 is a heat-shrink film 150^{b}, configured to be disposed over and tightly shrunk around a strut segment 112. Reference is now made to Figs. 5A-5C, showing stages of a method of covering a strut segment 112 with a heat-shrink film 150^{b}, followed by suturing the skirt 120 over the heat-shrink film 150^{b} covering the strut segments 112. As shown in Fig. 5A, each strut segment 112, such as strut segment 112^{a}a, can have a length L3 and a strut width W3. In some implementations, strut segment 112 can have a uniform strut segment width W3. In other implementations, strut segment 112 can have a varying width along its length, wherein strut segment width W3 represents the maximal width thereof.

The terms "film" or "sheet", as used herein, are interchangeable and refer to a flat flexible structure of the protective cover, such as a flat flexible structure of the heat-shrink material or a flat flexible structure of the pad material.

A plurality of heat-shrink films 150^{b} can be provided, matching the number of strut segments 112 to which the inner skirt 120 should be sutured along the circumference of the frame 106. Each heat-shrink film 150^{b} can be provided as a rectangular film of sheet configured to cover a single strut segment 112, having a film length L4 and a film width W5. The film length L4 is not greater than the strut segment length L3. In some examples, the film length L4 is substantially equal to the strut segment length L3, such that once it is disposed over the strut segment 112, it covers it along its entire length.

In some examples, the film length L4 can be shorter than the strut segment length L3, such that once it is disposed over the strut segment 112, it covers only a portion thereof. Such examples may be applicable if the commissure assembly 130 is shorter than the length L3, or if a shorter length along which the heat-shrink film 150^{b} covers the strut segment 112 is still considered to enough to sufficiently reduce long-term wear of the skirt 120 and/or suture 136.

The term "and/or" is inclusive here, meaning "and" as well as "or". For example, "skirt 120 and/or suture 136" encompasses, skirt 120, suture 136, and skirt 120 with suture 136; and, such "skirt 120 and/or suture 136" may include other elements as well.

As further shown in Fig. 5A, each strut segment 112 can have a strut thickness W4, representing its thickness in the radial direction (i.e., toward the centerline 20 of the frame 106). In some cases, the strut segment 112 can be provided with a uniform thickness W4 along its entire length. In other cases, the strut segment 112 can be provided with a varying thickness, in which case the strut thickness W4 is representative of the maximal thickness of the strut segment 112.

While strut segment 112 is shown in Figs. 5A-5C to have a rectangular cross-sectional shape, it is to be understood that this is shown for the sake of illustration and not limitation, and that a strut segment 112 can have any other cross-sectional shape, such as square, triangular, pentagonal, hexagonal, octagonal, circular, elliptical, star-shaped, and the like. For example, the strut segment 112 can have an elliptical cross-section having a first diameter in one direction, that is equal to the linear strut width W3 illustrated in Fig. 5A, and a second diameter in a radial direction, what is equal to the linear strut thickness W4 illustrated in Fig., wherein the first direction of the first diameter is substantially orthogonal to the radial direction. In another example, the strut segment 112 can have a circular cross-section having a circular strut diameter Wc, such that W3 = W4 = Wc.

Thus, any reference to a strut width W3 hereinbelow or in the claims is for either a rectangular strut segment having a linear strut width W3, an elliptic strut segment having a first diameter with the same magnitude of W3, or a circular strut segment having a circular diameter Wc that is equal to W3. Similarly, any reference to a strut thickness W4 hereinbelow or in the claims is for either a rectangular strut segment having a linear strut thickness W4, an elliptic strut segment having a second diameter with the same magnitude of W4, or a circular strut segment having a circular diameter Wc that is also equal to W4.

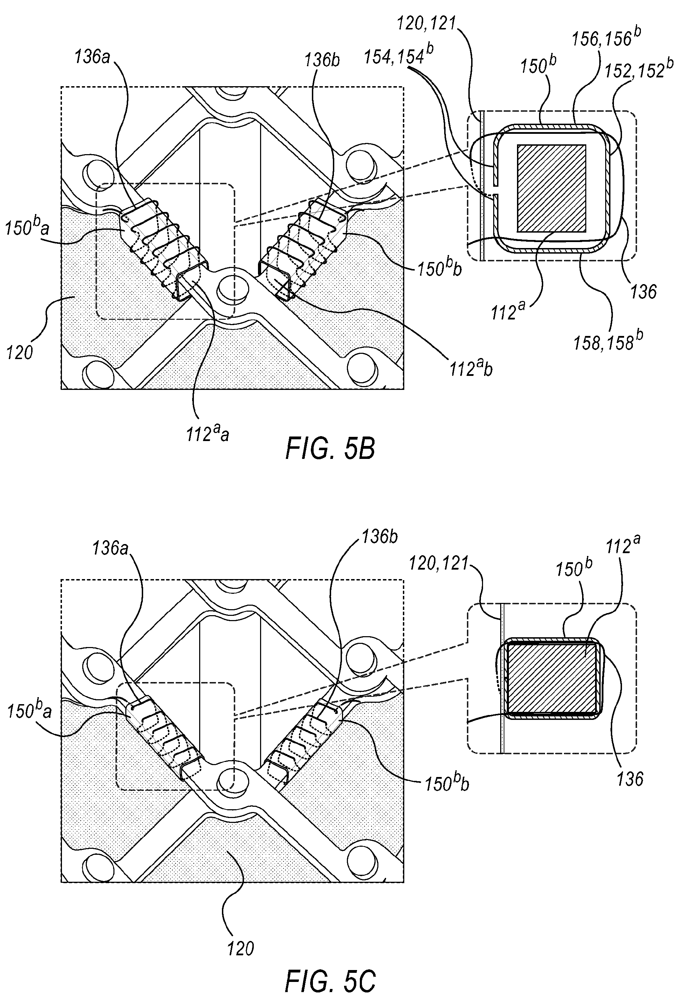

According to some examples, the film width W5 is greater than the strut width W3 plus twice the strut thickness W4, that it to say, W5 > W3 + 2W4. This enables the heat-shrink film 150^{b}, which is approximated to the strut segment 112 as shown in Fig. 5A, to be folded over the strut segment 112 as shown in Fig. 5B in a manner that will completely cover at least three edges of the strut 112, and at least a portion of the fourth remaining edge (shown as the inner edge in Fig. 5B).

It is to be understood that the dimensions W5 and L4 of the heat-shrink film 150^{b} relate to its initial, pre-shrunk state.

A heat-shrink wrap 150 can be folded in a manner that defines a heat-shrink outer section 152, heat-shrink proximal and distal sections 156 and 158, respectively, substantially orthogonal to and continuously extending from both sides of the heat-shrink outer section 152, and opposing heat-shrink inner sections 154 substantially orthogonal to and continuously extending from the heat-shrink proximal and distal sections 156 and 158.

The term "substantially orthogonal", as used herein, refers to an angle between two sections that is in the range of 70° to 110°.

As shown in Fig. 5B, the method includes a step of folding the heat-shrink film 150^{b} over the corresponding strut segment 112^{a}, such that the folded heat-shrink film 150^{b} defines an outer section 152^{b} disposed radially outward to the strut segment 112^{a}, heat-shrink proximal and distal sections 156^{b} and 158^{b} folded over the proximal and distal edges of the strut segment 112^{a}, and two opposing heat-shrink inner sections 154^{b} folded therefrom and disposed radially inward to the strut segment 112^{a}.

According to some examples, the film width W5 is selected such that when the heat-shrink film 150^{b} is folded over the strut segment 112^{a}, a gap remains between the opposite ends of both heat-shrink inner section 154^{b} as shown in the example illustrated in Fig. 5B, which can be reduced or eliminated upon application of heat serving to shrink the heat-shrink film 150^{b} such that the shrunk film 150^{b} fully encloses the strut segment 112^{a} as shown in the example illustrated in Fig. 5C.

The terms "example" and "exemplary" are used herein to mean "serving as an example, instance or illustration". Any example described as an "example" or "exemplary" is not necessarily to be construed as preferred or advantageous over other examples and/or to exclude the incorporation of features from other examples.

According to some examples, the film width W5 is greater than the twice the strut width W3 plus twice the strut thickness W4, that it to say, W5 > 2W3 + 2W4. According to some examples, the film width W5 is selected such that when the heat-shrink film 150^{b} is folded over the strut segment 112^{a}, it fully encircles the strut segment 112^{a} with no gaps formed between the ends heat-shrink inner section 154^{b}, which can contact each other or even overlap over each other to some extent, even prior to heat application thereto.

According to some examples, the method further comprises a step of attaching the inner skirt 120 to the heat-shrink film 150^{b} which is folded over the strut segment 112, for example along the skirt proximal end 121. The attachment can be accomplished by suturing a skirt attachment suture 136, as shown in Fig. 5B, through the inner skirt 120 and the folded heat-shrink film 150^{b}, for example by looping the suture 136 in a whip stitch pattern around the strut segment 112.

Suturing in a whip stitch pattern can include passing the skirt attachment suture 136, for example with a needle, through the inner skirt 120 and a first heat-shrink inner section 154^{b}, extending the suture between the strut segment 112 and the heat-shrink distal section 158^{b} toward and through the heat-shrink outer section 152^{b}, folding the suture over the outer side of the heat-shrink outer section 152^{b} and passing it back through the heat-shrink outer section 152^{b} such that it further extends between the strut segment 112 and the heat-shrink proximal section 156^{b}, forming a substantially U-shaped configuration around the strut segment 112. The suture 136 is then passed back through the second (i.e., opposite to the first) heat-shrink inner section 154^{b} and the skirt 120, thereby forming a looped portion of the suture 136 around the strut segment 112, attaching the skirt 120 to the heat-shrink film 150^{b} folded over the strut segment 112, wherein the suture can extend laterally, for example along the inner side of the inner skirt 120, to form a following loop in a continuous manner.

The term "inner side", as used herein, refers to a side of a component, such as a skirt 120, a heat shrink wrap 150, a strut 110 and the like, facing the centerline 20 of the frame 106. The term "outer side", as used herein, refers to a side of a component, that it opposite to the "inner side", facing away from the centerline 20 of the frame 106.

The above mentioned stitch pattern results in two sections of the skirt attachment suture 136 extending substantially parallel to each other within each loop formed by the stitching pattern, between the strut segment 112 and opposite sections of the heat-shrink film 150^{b}, such as one section of the suture 136 extending between the strut segment 112 and the heat-shrink distal section 158^{b}, and another section extending parallel thereto between the strut segment 112 and the heat-shrink proximal section 156^{b}, while the section of the suture 136 extending between these two parallel sections, is disposed over the outer side of the heat-shrink outer section 152^{b}.

While the method of suturing disclosed hereinabove with respect to Fig. 5B refers to passing the suture 136 from the inner side of the skirt 120 over the outer side of the heat-shrink outer section 152^{b}, and back toward the skirt 120, it is to be understood that these steps can be actually reversed, such that the suture is passed through the heat-shrink outer section 152^{b}, toward and through a first heat-shrink inner section 154^{b} and the skirt 120, folded to extend over an inner side of the inner skirt 120, and back through the skirt 120 and the second heat-shrink inner section 154^{b}, passing it back through the heat-shrink outer section 152^{b}, and optionally extending the suture 136 laterally, for example along the outer side of the heat-shrink outer section 152^{b}, to form a following loop in a continuous manner.

While the methods of suturing disclosed hereinabove with respect to Fig. 5B and alternatives thereof, refer to passing the suture 136 through the first heat-shrink inner section 154^{b} and extending it between the strut segment 112 and the heat-shrink distal section 158^{b}, performed prior to extending the suture 136 parallelly between the strut segment 112 and the heat-shrink proximal section 156^{b}, it is to be understood that the series of steps can be reversed such that the suture is first extended between the strut segment 112 and the heat-shrink proximal section 156^{b}, and a follow-up step includes extending it between the strut segment 112 and the heat-shrink distal section 158^{b}.

The suturing methods can include forming locking knots at both ends of the suture, adjacent to the opposite junctions 114 on both ends of the strut segment 112.

The term "adjacent" as used herein has the meaning, being next to or proximate another object. Two objects may be adjacent if they are in contact with each other or if they are merely in proximity to each other without actual contact.

While the methods of suturing disclosed herein refer, in some examples, to formation of whip stitch pattern, it is to be understood that any other method for formation of stitching patterns known in the art for suturing components around a longitudinal member, such as the strut segment 112, are contemplated.

The attachment of the skirt 120 to the heat-shrink films 150^{b}, for example by any of the suturing methods described hereinabove, can be repeated for each of the plurality of heat-shrink films 150^{b} following the appropriate strut segments 112 around the entire circumference of the frame 106, to which the skirt 120 is to be attached. This series of attachments (e.g., suturing) can follow a zig-zag pattern, for example in the case of a mechanically expandable valve 100^{a}, such as illustrated in Fig. 5B showing the skirt proximal end 121 sutured to a heat-shrink film 150^{b}a folded over strut segment 112^{a}a, then sutured to an adjacent heat-shrink film 150^{b}b folded over strut segment 112^{a}b, and so on, following the zig-zag pattern shown.

The method of covering a strut segments 112 with a heat-shrink film 150^{b} further includes a step of applying heat to the heat-shrink films 150^{b} utilizing a heat source as mentioned hereinabove, thereby causing them to shrink radially inward and to become tightly attached to and around the corresponding strut segments 112, as shown in Fig. 5C.

Since two suture sections of every suture loop along the whip stitch pattern have been parallelly extended between the strut segment 112 and the heat-shrink film 150^{b} (e.g., between the strut segment 112 and heat-shrink distal section 158^{b} and between the strut segment 112 and the heat-shrink outer and inner sections 152^{b} and 154^{b}, respectively), shrinking the heat-shrink films 150^{b} over the strut segments 112 advantageously serves to tighten the skirt attachment sutures 136 between the heat-shrink films 150^{b} and the corresponding struts 112, as also shown in Fig. 5C.

Advantageously, covering the strut segments 112 to which the inner skirt 120 is attached with heat-shrink wraps 150, such as heat-shrink films 150^{b}, increases the long-term durability of the prosthetic valve 100 due to less wear from the strut segments 112 engaging the inner skirt 120, as well as less wear to the skirt attachment sutures 136, due to being at least partially extended over portions of the heat-shrink wraps 150, as well as being tightened thereby, thus reducing relative movement of the sutures 136 and the skirt 120 against the struts 110 during the cycling motion of the leaflet assembly 122.

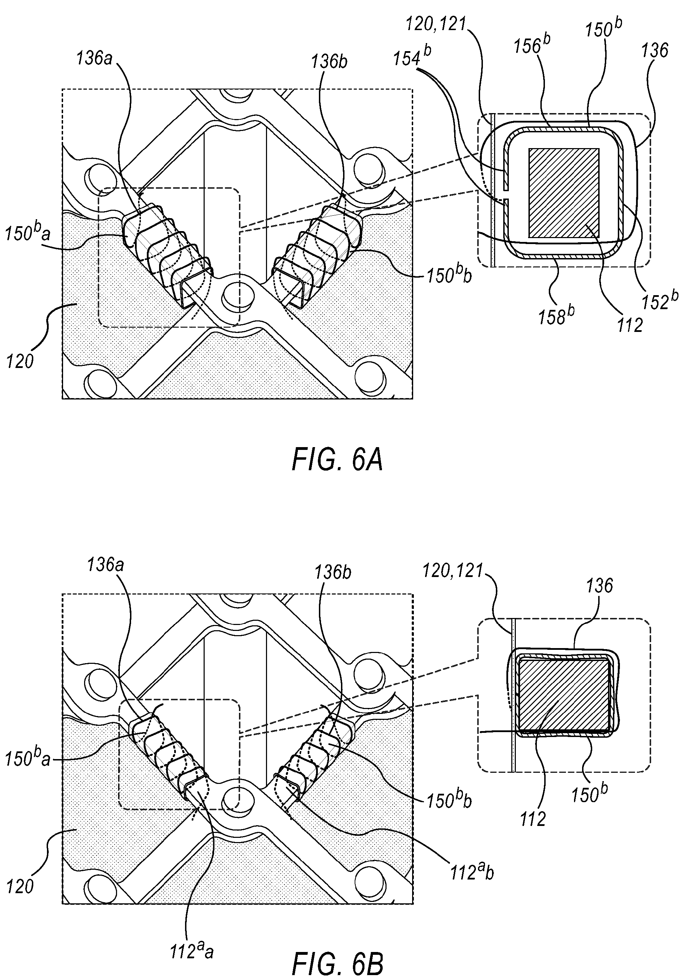

Reference is now made to Figs. 6A-6B, showing stages of another method of covering a strut segments 112 with a heat-shrink film 150^{b}, followed by suturing the skirt 120 over the heat-shrink film 150^{b} covering the strut segments 112. The stages of the method illustrated in Figs. 6A-6B are similar to the method described hereinabove in conjunction with Figs. 5A-5C, with the exception of the stitching pattern as will be elaborated below.

The types and dimensions of heat-shrink film 150^{b} and the stage of folding the heat-shrink film 150^{b} around a corresponding strut segment 112 can be identical to any example described hereinabove in conjunction with Fig. 5A. The configuration of a suture 136 looped through the skirt 120 and the heat-shrink film 150^{b} folded over the strut segment 112 shown in Fig. 6A is similar to that described above and illustrated in Fig. 5B, except that the suture 136 includes only one section extending between the strut segment 112 and the heat-shrink film 150^{b}, while the opposite, parallel section of the suture 136 is disposed over the external side of the heat-shrink film 150^{b}.

The term "internal side", as used herein with respect to a heat-shrink film 150^{b}, refers to a side of the heat-shrink film 150^{b} facing a respective strut segment 112 it is folded over. The term "external side", as used herein with respect to a heat-shrink film 150^{b}, refers to a side of the heat-shrink film 150^{b} that is opposite to the "internal side", facing away from the respective strut segment 112 it is folded over.

Thus, any reference to a section of the suture 136 described hereinabove to extend or be disposed over the outer side of the heat-shrink outer section 152^{b}, may be similarly termed to extend or be disposed over the external side of the heat-shrink outer section 152^{b}. Moreover, the sections of the suture 136 described hereinabove in conjunction with Fig. 5A-5C to extend between the strut segment 112 and the heat-shrink proximal section 156^{b}, or between the strut segment 112 and the heat-shrink distal section 158^{b}, may be similarly termed to extend specifically between the strut segment 112 and the internal side of heat-shrink proximal section 156^{b}, or between the strut segment 112 and the internal side of heat-shrink distal section 158^{b}, respectively.

Suturing in a whip stitch pattern, as shown in Fig. 6A, can include passing the skirt attachment suture 136 through the inner skirt 120 and a first heat-shrink inner section 154^{b}, extending the suture between the strut segment 112 and the heat-shrink distal section 158^{b} toward and through the heat-shrink outer section 152^{b}, folding the suture over the outer side of the heat-shrink outer section 152^{b} and then folding it back to extend over the external side of the heat-shrink proximal section 156^{b}, forming a substantially U-shaped configuration around the strut segment 112. The suture 136 is then passed back through the inner skirt 120, thereby forming a looped portion of the suture 136 around the strut segment 112, attaching the skirt 120 to the heat-shrink film 150^{b} folded over the strut segment 112, wherein the suture can extend laterally, for example along the inner side of the inner skirt 120, to form a following loop in a continuous manner.

The above mentioned stitch pattern results in two sections of the skirt attachment suture 136 extending substantially parallel to each other within each loop formed by the stitching pattern, wherein one section extends between the strut segment 112 and an inner side of a section of the heat-shrink film 150^{b}, such as between the strut segment 112 and the inner side of the heat-shrink distal section 158^{b}, and another section extends parallel thereto over an external side of a section of the heat-shrink film 150^{b}, such as along the external side of the heat-shrink proximal section 156^{b}, while the section of the suture 136 extending between these two parallel sections, is disposed over the outer side of the heat-shrink outer section 152^{b}.

While the method of suturing disclosed hereinabove with respect to Fig. 6A refers to passing the suture 136 from the inner side of the skirt 120 over the outer side of the heat-shrink outer section 152^{b}, and back toward the skirt 120, it is to be understood that these steps can be actually reversed, such that the suture is passed through the heat-shrink outer section 152^{b}, toward and through a first heat-shrink inner section 154^{b} and the skirt 120, folded to extend over an inner side of the inner skirt 120, and back through the skirt 120 and over an external side of a section of the heat-shrink film 150^{b}, such as along the external side of the heat-shrink proximal section 156^{b}, and optionally extending the suture 136 laterally, for example along the outer side of the heat-shrink outer section 152^{b}, to form a following loop in a continuous manner.

While the methods of suturing disclosed hereinabove with respect to Fig. 6A and alternatives thereof, refer to passing the suture 136 through the first heat-shrink inner section 154^{b} and extending it between the strut segment 112 and an internal side of the heat-shrink distal section 158^{b}, performed prior to extending the suture parallelly over the outer side of the heat-shrink outer section 152^{b}, it is to be understood that the series of steps can be reversed such that the suture is first extended over the outer side of the heat-shrink outer section 152^{b}, and a follow-up step includes extending it between the strut segment 112 and the internal side of heat-shrink distal section 158^{b}.

The method of covering a strut segments 112 with a heat-shrink film 150^{b} further includes a step of applying heat to the heat-shrink films 150^{b} utilizing a heat source as mentioned hereinabove, thereby causing them to shrink radially inward and to become tightly attached around the corresponding strut segments 112, as shown in Fig. 6B.

Since a suture section of every suture loop along the whip stitch pattern has been extended between the strut segment 112 and the heat-shrink film 150^{b} (e.g., between the strut segment 112 and heat-shrink distal section 158^{b} and between the strut segment 112 and an internal side of the heat-shrink distal section 158^{b}), shrinking the heat-shrink films 150^{b} over the strut segments 112 advantageously serves to tighten the skirt attachment suture 136 between the heat-shrink films 150^{b} and the corresponding struts 112, as also shown in Fig. 6B.

The configuration shown in Figs. 6A-6B includes a single section of the suture 136 disposed between one side of the strut segment 112 and the heat-shrink film 150^{b}, which may result in the suture 136 being tightened to a lesser degree than that offered by the configuration shown in Figs. 5B-5C, for example, wherein two parallel sections of the suture 136 are disposed between opposite sides of the strut segment 112 and the heat-shrink film 150^{b}. However, the stitching configuration described in conjunction with Figs. 6A-6B may be advantageous over the configuration described in conjunction with Figs. 5B-5C in that it may be less labor-intensive and simpler for execution during the assembly procedures, since extending portions of the suture 136 over a folded heat-shrink film 150^{b} (such as over heat-shrink proximal section 156^{b}) is simpler and easier than stitching through two opposite sections thereof (such as through 152^{b} and 154^{b}, respectively) while extending the suture between the heat-shrink film 150^{b} and the strut segment 112. Thus, the configuration described in conjunction with Figs. 6A-6B can represent a potential trade-off between the degree of desired tightening of the suture 136 achieved during shrinking of heat-shrink film 150^{b}, and the simplicity of suturing during the assembly procedure.

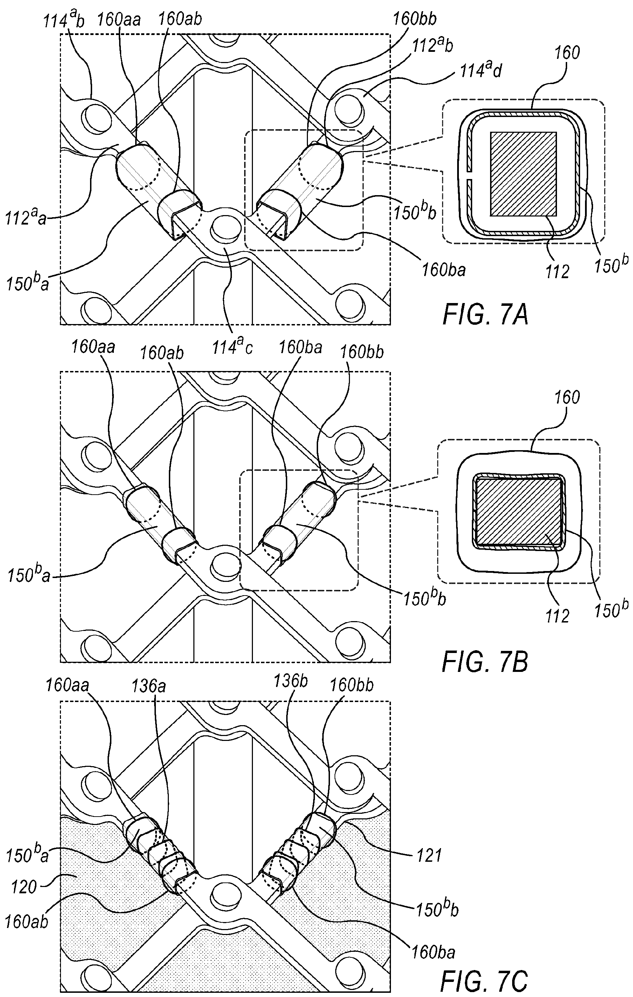

Reference is now made to Figs. 7A-7C, showing stages of another method of covering a strut segments 112 with a heat-shrink film 150^{b}, followed by suturing the skirt 120 over the heat-shrink film 150^{b} covering the strut segments 112. The types and dimensions of heat-shrink film 150^{b} and the stage of folding the heat-shrink film 150^{b} around a corresponding strut segment 112 can be identical to any example described hereinabove in conjunction with Fig. 5A.

According to some examples, at least one suture loop 160 is formed around a heat-shrink film 150^{b} folded over a corresponding strut segment 112. According to some examples, at least one suture loop 160 includes two suture loops 160 positioned at opposite end portions of the heat-shrink film 150^{b}, for example adjacent each of the opposite junctions 114 on both sides of the corresponding strut segment 112 covered by the heat-shrink film 150^{b}.

Fig. 7A shows an example of two heat-shrink films 150^{b}a and 150^{b}b folded over respective strut segments 112^{a}a and 112^{a}b, wherein two suture loops 160 are disposed around opposite ends of each of the heat-shrink films 150^{b}. Specifically, suture loop 160aa is disposed over an end portion of heat-shrink film 150^{b}a adjacent junction 114^{a}b, and suture loop 160ab is disposed over an opposite end portion of heat-shrink film 150^{b}a adjacent junction 114^{a}c. Similarly, suture loop 160ba is disposed over an end portion of heat-shrink film 150^{b}b adjacent junction 114^{a}c, and suture loop 160bb is disposed over an opposite end portion of heat-shrink film 150^{b}b adjacent junction 114^{a}d.

According to some examples, the perimeter of each suture loop 160 are at least as great as, and preferably greater than, the width W5 of the heat-shrink film 150^{b}, enabling the loop 160 to conveniently encircle the folded heat-shrink film 150^{b}.

Each suture loop 160 can be formed by surrounding a suture around the heat-shrink film 150^{b} which is folded over the corresponding strut segment 112, and potentially tying a knot between the free end of the suture to form the loop and preventing it from being spontaneously unraveled.

The role of the suture loops 160 are to temporarily retain the heat-shrink film 150^{b} in a folded configuration over the strut segment 112, prior to applying heat thereto. While two suture loops 160 disposed over two opposite end portions of each heat-shrink film 150^{b} are shown in Figs. 7A-7C, it is to be understood that this is shown for illustration and not limitation, and that any other number of suture loops 160 can be disposed at any other position. For example, a single suture loop 160 can be disposed over each heat-shrink film 150^{b}, and can be positioned over a middle-portion of the heat-shrink film 150^{b}. In another example, two or more suture loops 160 can be disposed along the heat-shrink film 150^{b} at various positions, which are not necessarily the end portions of the heat-shrink film 150^{b}. A plurality of suture loops 160 can be equally spaced from each other along a corresponding heat-shrink film 150^{b}, or spaced at varying distances from each other.

The method of covering a strut segments 112 with heat-shrink films 150^{b} further includes a step of applying heat to the heat-shrink films 150^{b} utilizing a heat source as mentioned hereinabove, thereby causing them to shrink radially inward and to become tightly attached around the corresponding strut segments 112, as shown in Fig. 7B. Since the suture loops 160 illustrated in Figs. 7A-7B are not passed through the heat-shrink films 150^{b}, but are rather surrounding the external sides thereof, they are not tightened as the heat-shrink films 150^{b} are shrunk, and remain loosely disposed therearound, as further shown in Fig. 7B. At this point the suture loops 160 are no longer required, and may be either cut away and removed from around the shrunken heat-shrink films 150^{b}, or alternatively the suture loops 160 can remain thereover.

As shown in Fig. 7C, the method further includes a step of attaching the inner skirt 120, for example along the skirt proximal end 121, to the strut segments 112 covered by the shrunken heat-shrink films 150^{b}. This attachment can be performed by suturing the skirt 120 to the strut segments 112 covered by the shrunken heat-shrink films 150^{b} with skirt attachment sutures 136, for example following whip stitch patterns, or any other stitching patterns suitable for attaching a skirt 120 to longitudinal members such as strut segments 112. This results in a configuration in which the skirt attachment suture 136 is sutured over the external surface of the shrunken heat-shrink film 150^{b}, without extending through the heat-shrink film 150^{b}. If the temporary suture loops 160 have not been yet cut away, they can be cut and removed from around the shrunken heat-shrink films 150^{b} after suturing the skirt 120 to the covered strut segments 112, or alternatively the suture loops 160 can remain thereover.

The method described in conjunction with Figs. 7A-7C may be advantageous over the methods described in conjunction with Figs. 5B-5C and 6A-6B in that it takes advantage of the fact that the heat-shrink films 150^{b} need to remain folded over the corresponding strut segments 112 mainly during a limited period of time, before heat is applied thereto, and that after application of heat, the heat-shrink films 150^{b} are tightly attached to the struts 112 due to being shrunk thereover, and no longer requires external means of keeping them in position over the strut segments 112. Thus, while the skirt 120 being sutured to the folded heat-shrink films 150^{b} prior to heat application, according to any of the methods described hereinabove in conjunction with Figs. 5B-5C and 6A-6B, served to retain the heat-shrink films 150^{b} in position and prevent them from unfolding prior to being shrunk, such suturing steps may be labor-intensive and prolong overall assembly procedures.

In comparison, the temporary suture loops 160 formed around the heat-shrink films 150^{b} instead of extending therethrough, may be formed in a much simpler and quicker manner, serving to keep the heat-shrink films 150^{b} in a folded configuration for a limited time period, such that once the heat-shrink films 150^{b} are shrunk over the corresponding strut segments 112, the inner skirt 120 can be sutured to the strut segments 112 in the same manner conventionally performed for skirt attachment to frames 106 without heat-shrink films 150^{b}, which may be much simpler and quicker, and require fewer adjustments to conventional assembly procedures.

While the method described hereinabove in conjunction with Fig. 7A-7C may lack the advantages of tightening the sutures 136 during the shrinking of the heat-shrink films 150^{b}, since the skirt 120 is sutured with the sutures 136 passing over and around the covered strut segments 112, it may, on the other hand, provide an alternative advantage of improved wear-resistance to the skirt attachment sutures 136, since specific sections of the sutures 136 extending between the strut segments 112 and the heat-shrink films 150^{b} according to the methods described hereinabove in conjunction with Figs. 5B-5C and 6A-6B, remain in contact with the strut segments 112 and may experience a certain degree of wear due to such contact, while the configuration described in conjunction with Fig. 7A-7C results in the sutures 136 extending over the heat-shrink films 150^{b} along their entire lengths, such that no portion thereof is in contact with the strut segments 112 and may be better protected from potential wear by the layers of the heat-shrink films 150^{b}.

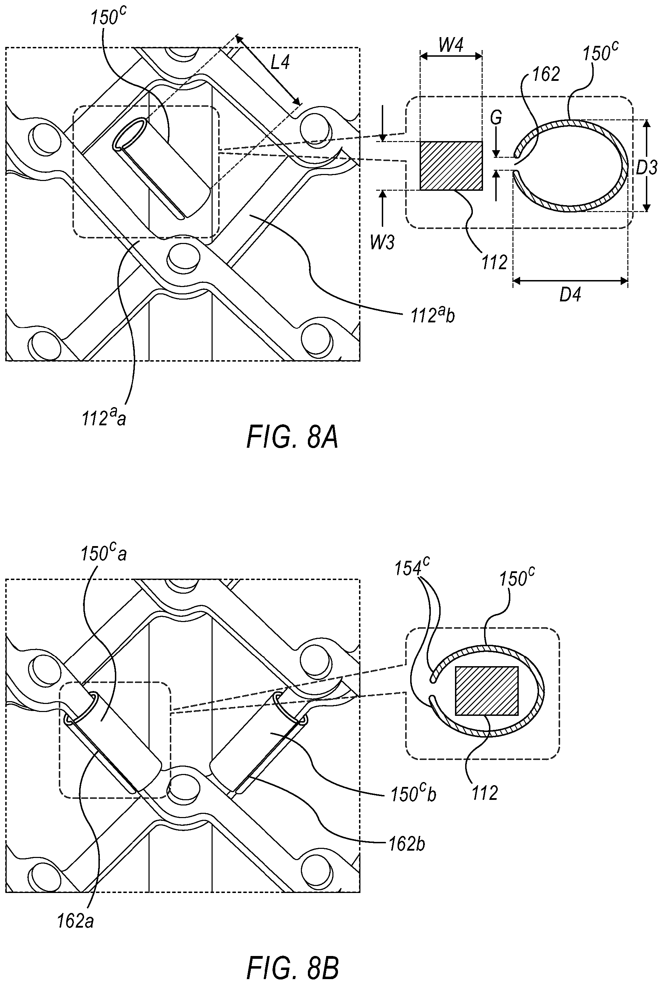

According to some examples, the heat-shrink wrap 150 is a semi-rigid C-shaped heat-shrink tube 150^{c}, configured to be tightly disposed around strut segment 112. Reference is now made to Figs. 8A-8D, showing stages of a method of covering a strut segment 112 with a C-shaped heat-shrink tube 150^{c}, followed by suturing the skirt 120 over the heat-shrunk tube 150^{c} covering the strut segments 112. Each strut segment 112, such as strut segment 112^{a}a, can have a strut segment length L3, a strut width W3 and a strut thickness W4 as defined hereinabove with respect to Fig. 5A, for example.

The C-shaped heat-shrink tube 150^{c} can have a tube length L4 similar to the length L4 of a heat-shrink films 150^{b} defined hereinabove with respect to Fig. 5A, for example. According to some examples, the inner dimensions of the C-shaped heat-shrink tube 150^{c} are at least as great as, and preferably greater than, the outer dimensions of corresponding strut segment 112 so as to allow the C-shaped heat-shrink tube 150^{c} to be easily disposed thereover. For example, the C-shaped heat-shrink tube 150^{c} can be provided with a tube first diameter D3, oriented in a first direction, which is at least as great as, and preferably greater than W3, and a tube second diameter D4, oriented in a second direction, which is at least as great as, and preferably greater than W4. The second direction of D4 can be a radial direction, substantially parallel to the direction of W4, while the first direction can be substantially orthogonal to the radial direction.

According to some examples, the tube first diameter D3 is at least as great as 120% of the strut width W3. According to some examples, the tube first diameter D1 is at least as great as 150% of the strut width W3. According to some examples, the tube first diameter D1 is at least as great as 200% of the strut width W3.

According to some examples, the tube second diameter D4 is at least as great as 120% of the strut thickness W4. According to some examples, the tube second diameter D4 is at least as great as 150% of the strut thickness W4. According to some examples, the tube second diameter D4 is at least as great as 200% of the strut thickness W4.

The C-shaped heat-shrink tube 150^{c} can have a circular cross section with a tube circular diameter Dc, that can be squeezed to an elliptical cross-sectional shape having the first and second diameters D3 and D4 as mentioned above.

It is to be understood that the dimensions L4, Dc, D3 and D4 of the C-shaped heat-shrink tube 150^{c} relate to its initial, pre-shrunk state.

As mentioned above, the strut segment 112 can have any cross-sectional shape, including a circular cross-section having a uniform diameter Wc, such that W3 = W4 = Wc. In such cases, a circular C-shaped heat-shrink tube 150^{c} will have a diameter Dc which is at least as great as, and preferably greater than Wc. An elliptic C-shaped heat-shrink tube 150^{c} will have each of the first and second diameters D3 and D4 at least as great as, and preferably greater than Wc.

As shown in Fig. 8A, the cross-sectional profile of the C-shaped heat-shrink tube 150^{c} includes a gap 162 defined between the ends of two opposing side arms 154^{c} thereof, having a gap width G in a free, unbiased state thereof. The side arms 154^{c} on both sides of the gap 162 are resiliently expandable away from each other, allowing a strut segment 112 to be passable through the gap 162.

In use, the C-shaped heat-shrink tube 150^{c} may be pushed to cover a corresponding strut segment 112. The gap width G is smaller than the smallest edge of the strut segment 112, for example being smaller than strut width W3. The sidewalls of the strut segment 112 may apply a force sufficient to expand the side arms 154^{c} of the C-shaped heat-shrink tube 150^{c} away from each other, so as to expand the gap width G and allow passing of the C-shaped heat-shrink tube 150^{c} over the strut segment 112. Once the strut segment 112 is completely accommodated within the C-shaped heat-shrink tube 150^{c}, and in the absence of further expanding force applied to its side arms 154^{c}, the side arms 154^{c} resiliently snap back toward each other to return the gap 162 to the original free gap width G, resulting in the C-shaped heat-shrink tube 150^{c} retained over the corresponding strut segment 112, as shown in Fig. 8B.

While the gap 162 is described hereinabove facing the side of the strut segment 112 having a width W3, allowing the C-shaped heat-shrink tube 150^{c} to be pushed there-against, it is to be understood that the gap 162 can be similarly facing the side of the strut segment 112 having a thickness W4, G being for example smaller than W4, allowing the C-shaped heat-shrink tube 150^{c} to be pushed there-against.

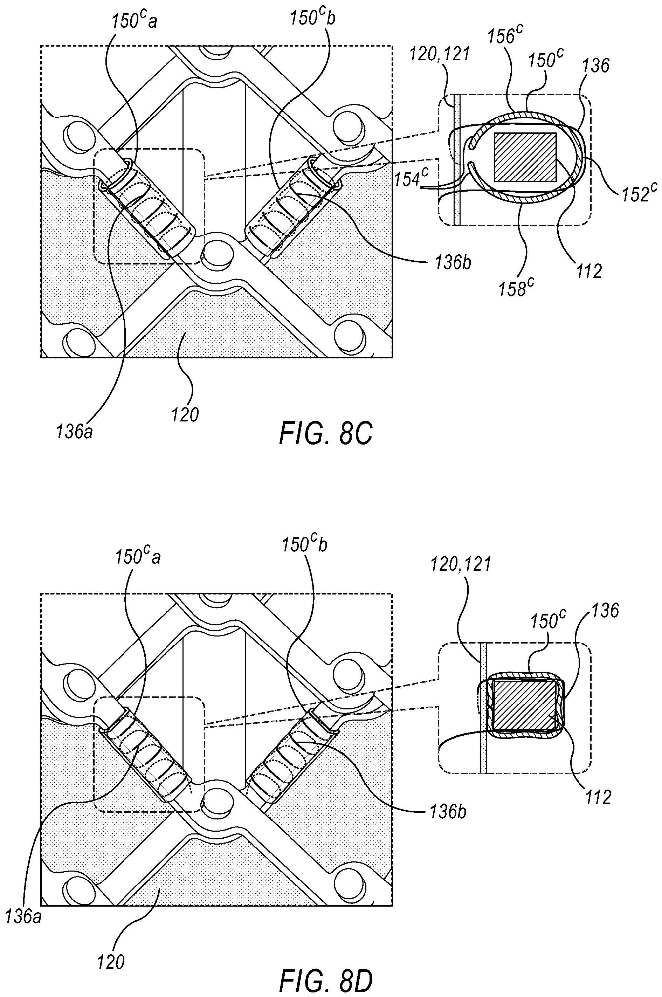

According to some examples, the method further comprises a step of attaching the inner skirt 120 to the C-shaped heat-shrink tube 150^{c} disposed over the strut segment 112, for example along the skirt proximal end 121. The attachment can be accomplished by suturing a skirt attachment suture 136, as shown in Fig. 8C, through the inner skirt 120 and the C-shaped heat-shrink tube 150^{c}, for example by looping the suture 136 in a whip stitch pattern.

Suturing in a whip stitch pattern can be performed in a similar manner to that described hereinabove in conjunction with Fig. 5B, and include passing the skirt attachment suture 136, through the inner skirt 120 and a first side arm 154^{c}, extending the suture 136 between one side of the strut segment 112 and the heat-shrink distal section 158^{c} toward and through the heat-shrink outer section 152^{c}, folding the suture over outer side of the heat-shrink outer section 152^{c} and passing it back through the heat-shrink outer section 152^{c} such that it further extends between the strut segment 112 and the heat-shrink proximal section 156^{c}, forming a substantially U-shaped configuration around the strut segment 112. The suture 136 is then passed back through the second (i.e., opposite to the first) side arm 154^{c} and the skirt 120, thereby forming a looped portion of the suture 136 around the strut segment 112, attaching the skirt 120 to the heat-shrink tube 150^{c} folded over the strut segment 112, wherein the suture can extend laterally, for example along the inner side of the inner skirt 120, to form a following loop in a continuous manner.

Similar to the configuration described and shown in Fig. 5B, the above mentioned stitch pattern results in two sections of the skirt attachment suture 136 extending substantially parallel to each other within each loop formed by the stitching pattern, between the strut segment 112 and opposite sections of the C-shaped heat-shrink tube 150^{c}, such as one section of the suture 136 extending between the strut segment 112 and the heat-shrink distal section 158^{c}, and another section extending parallel thereto between the strut segment 112 and the heat-shrink proximal section 156^{c}, while the section of the suture 136 extending between these two parallel sections, is disposed over the outer side of the heat-shrink outer section 152^{c}.

While the method of suturing disclosed hereinabove with respect to Fig. 8C refers to passing the suture 136 from the inner side of the skirt 120 toward and over the outer side of the heat-shrink outer section 152^{c}, and back toward the skirt 120, it is to be understood that these steps can be actually reversed, such that the suture is passed through the outer side of the heat-shrink outer section 152^{c}, toward and through a first side arm 154^{c} and the skirt 120, folded to extend over an inner side of the inner skirt 120, and back through the skirt 120 and the second side arm 154^{c}, passing it back through the heat-shrink outer section 152^{c}, and optionally extending the suture 136 laterally, for example along the outer side of the heat-shrink outer section 152^{c}, to form a following loop in a continuous manner.

While the methods of suturing disclosed hereinabove with respect to Fig. 8C and alternatives thereof, refer to passing the suture 136 through the first side arm 154^{c} and extending it between the strut segment 112 and the heat-shrink distal section 158^{c}, performed prior to extending the suture 136 parallelly between the strut segment 112 and the heat-shrink proximal section 156^{c}, it is to be understood that the series of steps can be reversed such that the suture is first extended between the strut segment 112 and the heat-shrink proximal section 156^{c}, and a follow-up step includes extending it between the strut segment 112 and the heat-shrink distal section 158^{c}.

The suturing methods can include forming locking knots at both ends of the suture, adjacent to the opposite junctions 114 on both ends of the strut segment 112.

While the methods of suturing disclosed herein refer, in some examples, to formation of whip stitch pattern, it is to be understood that any other method for formation of stitching patterns known in the art for suturing components around a longitudinal member, such as the strut segment 112, are contemplated.

The attachment of the skirt 120 to the C-shaped heat-shrink tubes 150^{c}, for example by any of the suturing methods described hereinabove, can be repeated for each of the plurality of semi-rigid C-shaped heat-shrink tubes 150^{c} following the appropriate strut segments 112 around the entire circumference of the frame 106, to which the skirt 120 is to be attached. This series of attachment can follow a zig-zag pattern, for example in the case of a mechanically expandable valve 100^{a}, such as illustrated in Fig. 8A-8C showing the skirt proximal end 121 sutured to a C-shaped heat-shrink tube 150^{c}a folded over strut segment 112^{a}a, then sutured to an adjacent C-shaped heat-shrink tube 150^{c}b folded over strut segment 112^{a}b, and so on, following the zig-zag pattern shown.

According to some examples, the method of covering a strut segments 112 with a C-shaped heat-shrink tube 150^{c} further includes a step of applying heat to the C-shaped heat-shrink tubes 150^{c} utilizing a heat source as mentioned hereinabove, thereby causing them to shrink radially inward and to become tightly attached to and around the corresponding strut segments 112, as shown in Fig. 5C.

Since two suture sections of every suture loop along the whip stitch pattern have been parallelly extended between the strut segment 112 and the C-shaped heat-shrink tube 150^{c} (e.g., between the strut segment 112 and heat-shrink distal section 158^{c} and between the strut segment 112 and the heat-shrink outer sections 152^{c} and side arms 154^{c}), shrinking the C-shaped heat-shrink tubes 150^{c} over the strut segments 112 advantageously serves to tighten the skirt attachment sutures 136 between the heat-shrink tubes 150^{c} and the corresponding struts 112, as also shown in Fig. 8D.

It is to be understood that the term "C-shaped heat-shrink tube" refers to a semi-rigid heat-shrink tube having a C-shaped cross-section in an initial, pre-heated/pre-shrunk state, and that once heat is applied thereto, the heat-shrink tube no longer retains its original C-shape, but is rather tightly covering the strut segment 112 following its cross-sectional shape. The gap 162 can be either shortened in this state, or completely closed to result in the heat-shrink tube 150^{c} completely encircling the entire circumference of the corresponding strut segment 112, as shown in Fig. 8D.

The term "semi rigid", as used herein, refers to materials or articles that hold a shape without external support, but exhibit higher flexibility when external forces are exerted on the structure. Thus, the term "semi rigid" used with reference to a C-shaped heat-shrink tube 150^{c}, refers to a heat-shrink tube, provided in a pre-heated/pre-shrunk state, having a rigidity that is sufficient to retain it in a pre-shaped C-shaped configuration, in the absence of external forces applied thereto, yet flexible enough to allow the side arms at both sides of the gap 162 to flex away from each other when force is applied thereto, and resiliently return to their original configuration when force is no longer applied thereto.

While Fig. 8C-8D illustrate a stitching pattern comparative to that illustrated in Fig. 5B-5C, *mutatis mutandis,* an alternative stitching patten of the skirt 120 to the C-shaped heat-shrink tubes 150^{c} can be adapted to follow steps similar to those described and illustrated in conjunction with Fig. 6A-6B, *mutatis mutandis,* wherein the suture 136 includes only one section extending between the strut segment 112 and the C-shaped heat-shrink tubes 150^{c}, while the opposite, parallel section of the suture 136 is disposed over an external side of the C-shaped heat-shrink tubes 150^{c}.

For example, suturing in a whip stitch pattern through C-shaped heat-shrink tubes 150^{c} can include passing the skirt attachment suture 136, through the inner skirt 120 and a first side arm of the C-shaped heat-shrink tube 150^{c}, extending the suture 136 between one side of the strut segment 112 and the heat-shrink tube 150^{c} toward and through the opposite side (e.g., opposite to the gap 162), folding the suture 136 over the outer side of the heat-shrink outer section 152^{b} and backward, forming a substantially U-shaped configuration around the strut segment 112. The suture 136 is then passed back through the inner skirt 120, thereby forming a looped portion of the suture 136 around the strut segment 112, attaching the skirt 120 to the C-shaped heat-shrink tube 150^{c} disposed over the strut segment 112, wherein the suture can extend laterally, for example along the inner side of the inner skirt 120, to form a following loop in a continuous manner.

The above mentioned stitch pattern results in two sections of the skirt attachment suture 136 extending substantially parallel to each other within each loop formed by the stitching pattern, wherein one section extends between the strut segment 112 and an inner side of a section of the C-shaped heat-shrink tube 150^{c}, and another section extending parallel thereto over an external side of an opposite section of the C-shaped heat-shrink tube 150^{c}.

Any example of variation of the method described hereinabove for suturing through a heat-shrink film 150^{b} in conjunction with Figs. 6A-6B is similarly applicable to suturing through a C-shaped heat-shrink tube 150^{c}, *mutatis mutandis.*

Utilization of C-shaped heat-shrink tubes 150^{c} instead of heat-shrink films 150^{b} may be advantageous in that the heat-shrink tubes 150^{c} can be easily positioned over the strut segments 112 and retain their position thereover while suture 136 are passed therethrough, without requiring application of an external force to keep the films 150^{b} from unfolding prior to and during passing of sutures 136 therethrough.

Alternative examples of a method of covering a strut segment 112 with a C-shaped heat-shrink tube 150^{c}, followed by suturing the skirt 120 over the heat-shrunk tube 150^{c} covering the strut segments 112, does not include extending a suture 136 through the C-shaped heat-shrink tubes 150^{c} prior to heat application thereto, but rather includes a step of applying heat to the C-shaped heat-shrink tubes 150^{c} utilizing a heat source as mentioned hereinabove, after the step of disposing the C-shaped heat-shrink tubes 150^{c} over the corresponding strut segments 112 shown in Fig. 8B, thereby causing them to shrink radially inward and to become tightly attached to and around the corresponding strut segments 112.

The method further includes a step of attaching the inner skirt 120, for example along the skirt proximal end 121, to the strut segments 112 covered by the shrunken heat-shrink tubes 150^{c}. This attachment can be performed by suturing the skirt 120 to the strut segments 112 covered by the shrunken heat-shrink tubes 150^{c} with skirt attachment sutures 136, for example following whip stitch patterns, or any other stitching patterns suitable for attaching a skirt 120 to longitudinal members such as strut segments 112.

The method described above may be advantageous over any of the other methods described herein, in that it takes advantage of the fact that the C-shaped heat-shrink tubes 150^{c} can be easily placed over the corresponding strut segments 112 and retain their position up to and during the heating phase, after which the inner skirt 120 can be sutured to the covered strut segments 112 in the same manner conventionally performed for skirt attachment to frames 106 without heat-shrink wraps 150 of any kind. This in turn can allow easy adaptation of the proposed method with almost no change required to conventional suturing methods of the skirt 120, wherein the additional step of placing the C-shaped heat-shrink tubes 150^{c} over the corresponding strut-segments 112 and applying heat thereto can be achieved in a relatively simple manner, without requiring folding of the heat-shrink wraps 150, stitching sutures 136 therethrough, or forming suture loops 160 thereover.

Some types of prosthetic valves 100 include leaflet assemblies 122 in which the leaflets 124 are directly sutured to the frame 106, for example along the scallop line 134, instead of being stitched to an inner skirt 120 which is in turn attached to the frame 106. While described and shown for strut segments 112 to which an inner skirt 120 is attached, it is to be understood that any of the configurations described above, for covering strut segment 112 with various types of heat-shrink wraps 150, such as head-shrink films 150^{b} or C-shaped heat-shrink tubes 150^{c}, are similarly applicable for covering strut segments to which the leaflets 124 are directly attached, wherein any reference to attachment of the skirt 120 to the heat-shrink wraps 150 and/or the strut-segments 112 disclosed hereinabove, can be replaced with a reference to the scalloped end of the leaflet 124, *mutatis mutandis.*

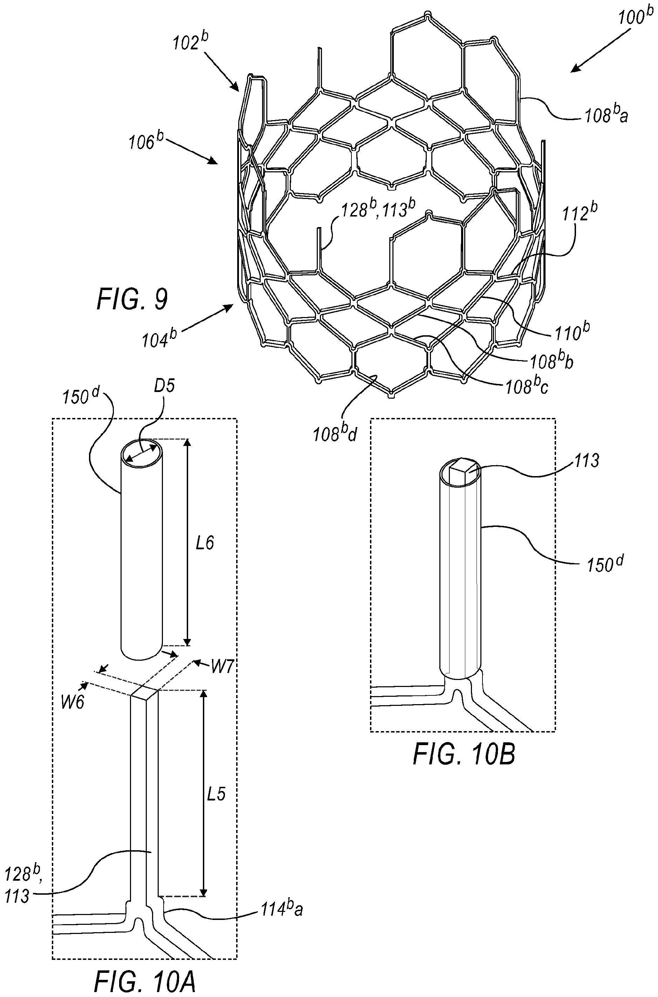

Reference is now made to Fig. 9, showing an example of a frame 106^{b} of a prosthetic valve 100^{b} which does not necessarily include expansion and locking assemblies (138) or any other type of attachable commissural posts. The prosthetic valve 100^{b} can be a type of a self-expandable or a balloon expandable valve. Balloon expandable valves generally involve a procedure of inflating a balloon within a prosthetic valve, thereby expanding the prosthetic valve (100) within the desired implantation site. Once the valve is sufficiently expanded, the balloon is deflated and retrieved along with the delivery apparatus. Self-expandable valves include a frame that is shape-set to automatically expand as soon an outer retaining capsule, which may be also defined as the distal portion of an outer shaft of the delivery assembly, is withdrawn proximally relative to the prosthetic valve.

According to some examples, the frame 106^{b} can include a plurality of angled strut segments 112^{b} as well as axially extending strut segments 113^{b}. The angled strut segments 112^{b} may be pivotable or bendable relative to each other, so as to permit frame expansion or compression. For example, the frame 106^{b} can be formed from a single piece of material, such as a metal tube, via various processes such as, but not limited to, laser cutting, electroforming, and/or physical vapor deposition, while retaining the ability to collapse/expand radially in the absence of hinges and like.

It is to be understood that any reference to "strut segments 112" throughout the current specification, may refer to either "angled strut segments 112^{x}" and/or "axially extending strut segments 113" or "axially extending strut segments 113^{x}" (superscript ^{x} may be any specific superscript relating to examples of frame 106).

The frame 106^{b} can include a plurality of rows of cells 108^{b}, wherein the cells 108^{c} can be differently shaped and define different internal openings. For example, Fig. 9 illustrates an exemplary frame 106^{b} with four rows of cells: a first row of axially elongated cells 108^{b}a along the outflow portion of the valve, two intermediate rows of cells 108^{b}b and 108^{b}c, and a final row of axially elongated cells 108^{b}d along the inflow portion of the valve, wherein the axial length of the cells 108^{b}d can be shorter than the axial length of the cells 108^{b}a.

As further shown, the upper row of cells along the outflow portion of the valve 100^{b} can include a plurality of axially extending strut segments 113^{b}, each extending proximally from a junction 114^{b}, disposed between two adjacent closed cells 108^{b}a. Such axially extending strut segments 113^{b} can serve as commissural support members 128^{b}, which can be integrally formed with the frame 106^{b}, configured to allow commissure assemblies 130 to be mounted thereto. According to some examples, the frame 106^{b} includes three axially extending strut segments 113^{b} serving as commissural support members 128^{b}.

The axially extending strut segments 113^{b} shown in Fig. 9 are open ended, meaning that they have exposed proximal end allowing unrestricted access for sliding a heat-shrink sleeve 150^{d} from above, in a similar manner to that described hereinabove for sliding a heat-shrink sleeve 150^{a} over a commissural support member 128^{a} of an outer member 140.

Reference is now made to Figs. 10A-10D, showing stages of a method of covering a commissural support member 128^{b} with a heat-shrink sleeve 150^{d}, followed by mounting a commissure assembly 130 over the heat-shrink sleeve 150^{d} covering the commissural support member 128. The commissural support member 128^{b} shown in Fig. 10A is formed by an axially extending strut segments 113^{b} extending proximally from a proximal-most non-apical junction 114^{b}a, having a support member length L5.

According to some examples, the heat-shrink sleeve 150^{d} has a sleeve length L6 that is at least as great as the length L5. The sleeve length L6 can be substantially equal to L5, such that once it is disposed over the commissural support member 128^{b}, it covers its entire length. In alternative implementations, the sleeve length L6 can be greater than L5, such that once it is disposed over the commissural support member 128^{b}, it extends beyond the proximal end of the commissural support member 128^{b}, in which case it can be cut, for example by scissors, a knife, or any other suitable cutting means, at the level of the proximal end of the commissural support member 128^{b}, resulting in the heat-shrink sleeve 150^{d} covering the exact length L5 of the commissural support member 128^{b}.

In alternative examples, the heat-shrink sleeve 150^{d} has a sleeve length L6 that is shorter than the support member length L5, such that once it is disposed over the commissural support member 128^{b}, it covers only a portion thereof. Such examples may be applicable if the commissure assembly 140 is shorter than the support member length L5, or if a shorter length along which the heat-shrink sleeve 150^{d} covers the commissural support member 128^{b} is still considered to be enough to sufficiently reduce long-term wear of the soft components of the commissure assembly 140.

According to some examples, the inner dimensions of the heat-shrink sleeve 150^{d} are at least as great as, and preferably greater than, the outer dimensions of the commissural support member 128^{b}, so as to allow the heat-shrink sleeve 150^{d} to be easily disposed thereover. For example, the commissural support member 128^{b} can have a support member radial depth W6 and a support member lateral width W7 as shown in Fig. 10A. For such cases, the heat-shrink sleeve 150^{d} can be provided as a cylindrical sleeve having a uniform sleeve circular diameter D5 that is greater than each of W5 and W6.

Alternatively, the heat-shrink sleeve 150^{d} can be provided with an elliptical cross-section, similar to that shown for heat-shrink sleeve 150^{a} in Fig. 3A, and have a first diameter oriented in the radial direction, which is at least as great as, and preferably greater than W6, and a second diameter, oriented at the lateral direction, which is at least as great as, and preferably greater than W7. Alternatively, the heat-shrink sleeve 150^{d} can have a circular cross section with a diameter D5 that can be squeezed to form a first diameter oriented in the radial direction, which is at least as great as, and preferably greater than W6, and a second diameter, oriented at the lateral direction, which is at least as great as, and preferably greater than W7.

It is to be understood that the dimensions D5 and/or the first and second diameters of the heat-shrink sleeve 150^{d} relate to its initial, pre-shrunk state.

While the axially extending strut segments 113^{b} serving as the commissural support member 128^{b} is shown in Figs. 10A-10D to have a rectangular cross-sectional shape, it is to be understood that this is shown by way of illustration and not limitation, and that the axially extending strut segments 113^{b} can have any other cross-sectional shape, such as square, triangular, pentagonal, hexagonal, octagonal, circular, elliptical, star-shaped, and the like. For example, the outer member can have an elliptical cross-section having a support member first diameter in a radial direction that is equal to the linear radial depth W6, and a support member second diameter in a lateral direction that is equal to the linear lateral width W7, wherein D5 or the sleeve first diameter of the heat-shrink sleeve 150^{d} is at least as great as, and preferably greater than W5, and wherein D5 or the sleeve second diameter of the heat-shrink sleeve 150^{d} is at least as great as, and preferably greater than W7.

In another example, the axially extending strut segment 113^{b} can have a circular cross-section having a uniform support member circular diameter Ws, such that W6 = W7 = Wc. A circular heat-shrink sleeve 150^{d} may have in such case a sleeve circular diameter D5 which is at least as great as, and preferably greater than Ws, and an elliptic heat-shrink sleeve 150^{d} will have each of the sleeve first and second diameters at least as great as, and preferably greater than Ws.

Thus, any reference to a support member radial depth W6 hereinbelow or in the claims is for either a rectangular commissural support member having a linear radial depth W6, an elliptic commissural support member having a support member first diameter with the same magnitude of W6, or a circular commissural support member having a support member circular diameter Ws that is equal to W6. Similarly, any reference to a support member lateral width W7 hereinbelow or in the claims is for either a rectangular commissural support member having a linear lateral width W7, an elliptic commissural support member having a support member second diameter with the same magnitude of W7, or a circular commissural support member having a support member circular diameter Wc that is also equal to W7

According to some examples, the sleeve circular diameter D5 is at least as great as 120% of the support member radial depth W6 and/or of the support member lateral width W7. According to some examples, the sleeve circular diameter D5 is at least as great as 150% of W6 and/or of W7. According to some examples, the sleeve circular diameter D6 is at least as great as 200% of W6 and/or of W7.

As shown in Fig. 10B, the method includes a step of disposing the heat-shrink sleeve 150^{d} over the commissural support member 128^{b}. The configuration shown in Figs. 10A-10D, in which the commissural support member 128^{b} has an exposed proximal end, advantageously allows the heat-shrink sleeve 150^{d} to be easily slid over the commissural support member 128^{b} from above, preferably having inner dimensions that are sufficiently larger than the outer dimensions of the commissural support member 128^{b} to enable it to be conveniently positioned over the commissural support member 128^{b} with minimal effort.

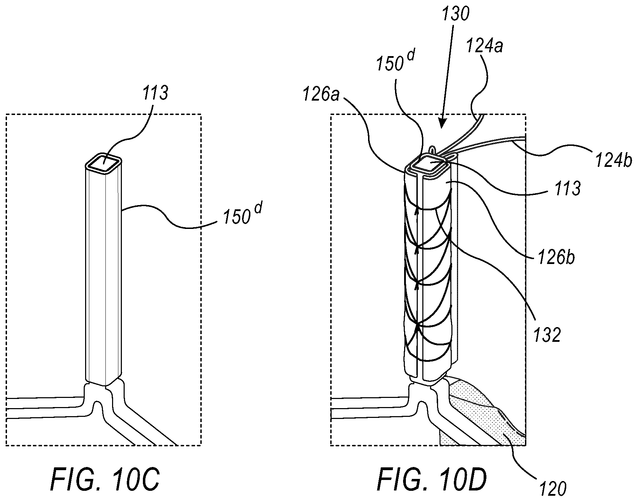

The method further includes a step of applying heat to the heat-shrink sleeve 150^{d} utilizing a heat source as mentioned hereinabove, thereby causing it to shrink radially inward and to become tightly attached around the commissural support member 128^{b}, as shown in Fig. 10C. The commissure assembly 130 is then attached to the commissural support member 128^{b} covered by the heat-shrink sleeve 150^{d}, as shown in Fig. 10D. For example, the tabs 126a and 126b of adjacent leaflets 124a and 124b, respectively, can be folded over the heat-shrink sleeve 150^{d} covering the commissural support member 128^{b}, and commissural attachment sutures 132 can be utilized to stitch the tabs 126 to each other and to the commissural support member 128^{b} covered by the heat-shrink sleeve 150^{d}, as shown.

Advantageously, covering the commissural support member 128^{b} with a heat-shrink wrap 150, such as heat-shrink sleeve 150^{d}, increases the long-term durability of the prosthetic valve 100^{b} due to less wear from the commissural support members 128^{b} engaging soft components of the commissure assembly 130, such as tabs 126 of leaflets 124 and commissural attachment sutures 132.

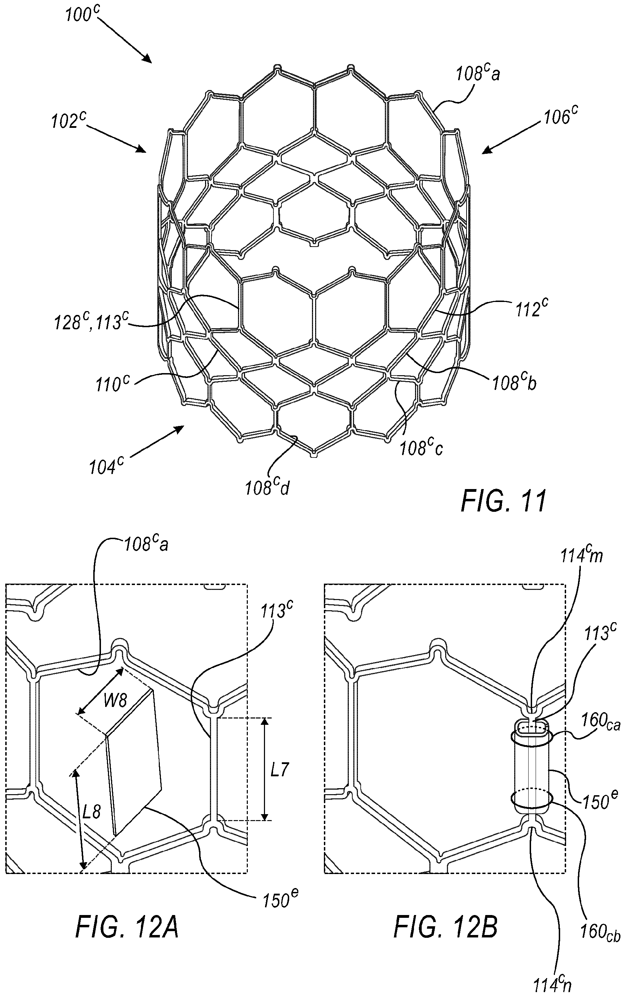

Reference is now made to Fig. 11, showing an example of a frame 106^{c} of a prosthetic valve 100^{c} which can be similar to any example disclosed above for prosthetic valve 100^{b}, except that the upper row of cells along the outflow portion of the frame 106^{c} includes only axially elongated closed cells 108^{c}a defined by two lower angled strut segments 112^{c}, two upper angled strut segments 112^{c}, and two axially extending strut segments 113^{c} extending between corresponding lower and upper angled strut segments 112^{c}. According to some examples, some of the axially extending strut segments 113^{c} of the cells 108^{c}a formed along the row at the outflow portion of the valve 100^{c} serve as commissural support members 128^{c}, which can be integrally formed with the frame 106^{c}, configured to allow commissure assemblies 130 to be mounted thereto. According to some examples, three axially extending strut segments 113^{b} of the frame 106^{c} are configured to serve as commissural support members 128°.

Unlike the commissural support members 128^{b} shown in Figs. 9-10D, each commissural support member 128^{c} is bound between angled strut segments 112°, restricting access thereto such that it may not be possible to slide a heat-shrink sleeve (150^{d}) thereover.

According to some examples, the heat-shrink wrap 150 is a heat-shrink film 150^{e}, configured to be tightly disposed around a commissural support member 128°. Reference is now made to Figs. 12A-12D, showing stages of a method of covering a commissural support member 128^{c} with a heat-shrink film 150^{e}, followed by mounting a commissure assembly 130 over the heat-shrink film 150^{e} covering the commissural support member 128°. As shown in Fig. 10A, the commissural support member 128^{c} can have a support member length L7. The commissural support member 128^{c} can also have a support member radial depth W6 and a support member lateral width W7 as described hereinabove for commissural support member 128^{b} in conjunction with Fig. 10A.

A plurality of heat-shrink films 150^{e} can be provided, matching the number of commissural support member 128^{c} to which commissure assemblies 130 should be mounted. According to some examples, three heat-shrink films 150^{e} are provided to cover three commissural support member 128^{c} disposed around the circumference of the frame 106^{c}.

Each heat-shrink film 150^{e} can be provided as a rectangular film of sheet configured to cover a single strut segment 112, having a film length L8 and a film width W8. The film length L8 is not longer than the commissural support member length L7. In some examples, the film length L8 is substantially equal to the commissural support member length L7, such that once it is disposed over the commissural support member 128°, it covers it along its entire length.

In some examples, the film length L8 can be shorter than the commissural support member length L7, such that once it is disposed over the commissural support member 128°, it covers only a portion thereof. Such examples may be applicable if the commissure assembly 130 is shorter than the length L7, or if a shorter length along which the heat-shrink film 150^{e} covers the commissure support member 128^{c} is still considered to enough to sufficiently reduce long-term wear of soft components of commissure assembly 130.

The axially extending strut segment 113^{c} forming the commissural support member 128^{c} can be shaped and dimensioned according to any of the examples described for axially extending strut segment 113^{b}, including an optional circular cross-section having a uniform diameter Ws.

According to some examples, the film width W8 is greater than the support member lateral width W7 plus twice the support member radial depth W6, that it to say, W8 > W7 + 2W6. This enables the heat-shrink film 150^{e}, which is approximated to the commissural support member 128^{c} as shown in Fig. 12A, to be folded over the commissural support member 128^{c} as shown in Fig. 12B in a manner that will completely cover at least three edges of the commissural support member 128^{c}, and at least a portion of the fourth remaining edge.

It is to be understood that the dimensions W8 and L8 of the heat-shrink film 150^{e} relate to its initial, pre-shrunk state.

The method can include a step of folding the heat-shrink film 150^{e} over the corresponding commissural support member 128^{c}, such that the folded heat-shrink film 150^{e} defines an outer section 152^{e} disposed radially outward to the commissural support member 128^{c}, heat-shrink proximal and distal sections 156^{e} and 158^{e} folded over the proximal and distal edges of the commissural support member 128^{c}, and two heat shrink inner sections 154^{e} folded therefrom and disposed radially inward to the commissural support member 128^{c} (folded sections of heat-shrink film 150^{e} not shown separately, but similar to the configuration shown in Fig. 5B).

According to some examples, the film width W8 is selected such that when the heat-shrink film 150^{e} is folded over the commissural support member 128^{c}, a gap remains between the opposite ends of both heat-shrink inner section 154^{e}, which can be reduced or eliminated upon application of heat serving to shrink the heat-shrink film 150^{e} such that the shrunk film 150^{e} fully encloses the commissural support member 128^{c}.

According to some examples, the film width W8 is greater than twice the lateral width W7 plus twice the radial depth W6, that it to say, W8 > 2W7 + 2W6. According to some examples, the film width W8 is selected such that when the heat-shrink film 150^{e} is folded over the commissural support member 128^{c}, it fully encircles the commissural support member 128^{c} with no gaps formed between the ends heat-shrink inner section 154^{e}, which can contact each other or even overlap over each other to some extent, even prior to heat application thereto.

According to some examples, at least one suture loop 160 is formed around a heat-shrink film 150^{e} folded over a corresponding commissural support member 128^{c}. According to some examples, at least one suture loop 160 includes two suture loops 160 positioned at opposite end portions of the heat-shrink film 150^{e}, for example adjacent each of the opposite junctions 114^{c} on both sides of the commissural support member 128^{c} covered by the heat-shrink film 150^{e}.

Fig. 12B shows an example of two suture loops 160 disposed around opposite ends of each of a heat-shrink films 150^{e}. Specifically, suture loop 160ca is disposed over an end portion of heat-shrink film 150^{e} adjacent junction 114^{c}m, and suture loop 160cb is disposed over an opposite end portion of heat-shrink film 150^{e} adjacent junction 114^{c}n.

According to some examples, the external dimensions of each suture loop 160 are at least as great as, and preferably greater than, the width W8 of the heat-shrink film 150^{e}, enabling the loop 160 to conveniently encircle the folded heat-shrink film 150^{e}.

Each suture loop 160 can be formed by surrounding the heat-shrink film 150^{e} which is folded over the commissural support member 128^{c}, and potentially tying a knot to form the loop and preventing it from being spontaneously unraveled.

The role of the suture loops 160 are to temporarily retain the heat-shrink film 150^{e} in a folded configuration over the commissural support member 128^{c}, prior to applying heat thereto. While two suture loops 160 disposed over two opposite end portions of each heat-shrink film 150^{e} are shown in Figs. 12B-12C, it is to be understood that this is shown for illustration and not limitation, and that any other number of suture loops 160 can be disposed at any other position. For example, a single suture loop 160 can be disposed over a heat-shrink film 150^{e}, and can be positioned over a middle-portion of the heat-shrink film 150^{e}. In another example, two or more suture loops 160 can be disposed along the heat-shrink film 150^{e} at various positions, which are not necessarily the end portions of the heat-shrink film 150^{e}. A plurality of suture loops 160 can be equally spaced from each other along a corresponding heat-shrink film 150^{e}, or spaced at varying distances from each other.

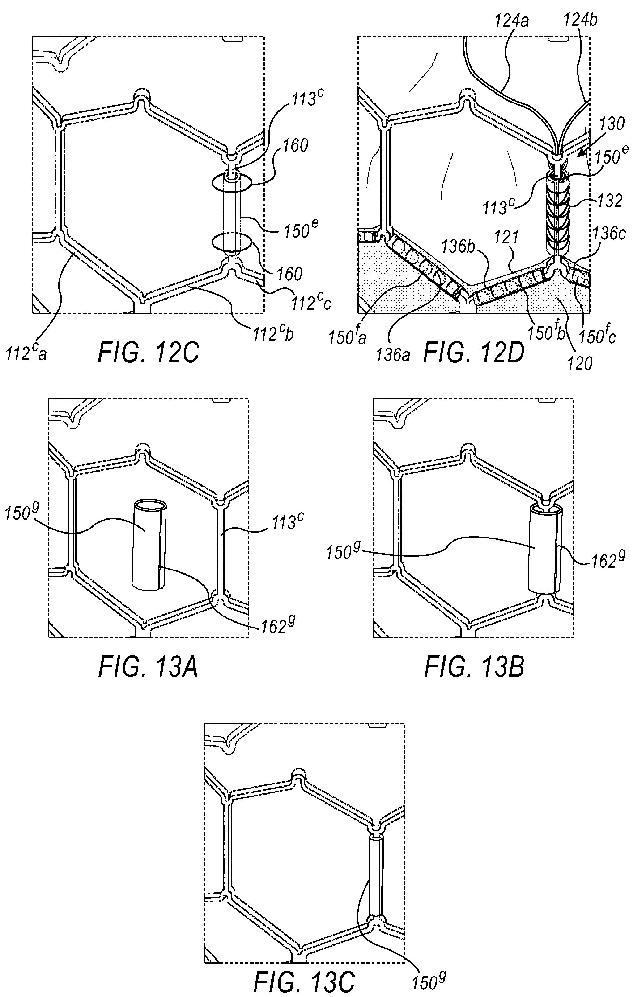

The method of covering a commissural support member 128^{c} with a heat-shrink film 150^{e} further includes a step of applying heat to the heat-shrink films 150^{e} utilizing a heat source as mentioned hereinabove, thereby causing it to shrink radially inward and to become tightly attached around the commissural support member 128^{c}, as shown in Fig. 12C. Since the suture loops 160 illustrated in Figs. 12B-12C are not passed through the heat-shrink film 150^{e}, but are rather surrounding the external sides thereof, they are not tightened as the heat-shrink film 150^{e} is shrunk, and remain loosely disposed therearound, as further shown in Fig. 12C. At this point the suture loops 160 are no longer required, and may be either cut away and removed from around the shrunken heat-shrink film 150^{e}, or alternatively the suture loops 160 can remain thereover.

The commissure assembly 130 is then attached to the commissural support member 128^{c} covered by the heat-shrink film 150^{e}, as shown in Fig. 12D. For example, the tabs 126a and 126b of adjacent leaflets 124a and 124b, respectively, can be folded over the heat-shrink film 150^{e} covering the commissural support member 128^{c}, and commissural attachment sutures 132 can be utilized to stitch the tabs 126 to each other and to the commissural support member 128^{c} covered by the heat-shrink film 150^{e}, as shown.

While utilizing temporary suture loops 160, alternative methods can include passing commissural attachment sutures 132 utilized during formation of a commissure assembly 130, through sections of the folded heat-shrink film 150^{e}, for example following stitching patterns that can be similar to those described hereinabove in conjunctions with Figs. 5B-C or 6A-B, *mutatis mutandis.* Such configurations can be advantageous in that shrinking of the heat-shrink films 150^{e} may further serve to tighten the commissural attachment sutures 132.

According to some examples, the heat-shrink wrap 150 is a semi-rigid C-shaped heat-shrink tube 150^{g}, configured to be tightly disposed around commissural support member 128^{c}. Reference is now made to Figs. 13A-13C, showing stages of a method of covering a commissural support member 128^{c} with a C-shaped heat-shrink tube 150^{g}, in a similar manner to that described hereinabove for covering strut segment 112 with semi-rigid C-shaped heat-shrink tube 150^{c} in conjunction with Figs. 8A-8D, *mutatis mutandis.*

The C-shaped heat-shrink tube 150^{g} can have a length L8 similar to the length L8 of a heat-shrink films 150^{e} defined hereinabove with respect to Fig. 12A, for example. According to some examples, the inner dimensions of the C-shaped heat-shrink tube 150^{g} are at least as great as, and preferably greater than, the outer dimensions of corresponding commissural support member 128^{c} so as to allow the C-shaped heat-shrink tube 150^{g} to be easily disposed thereover. For example, the C-shaped heat-shrink tube 150^{g} can be provided with a tube first diameter oriented in a first direction, which is at least as great as, and preferably greater than W6, and a tube second diameter oriented in a second direction, which is at least as great as, and preferably greater than W7. The first direction can be a radial direction, substantially parallel to the direction of W6, while the second direction can be orthogonal to the first direction, and substantially parallel to the direction of W7.

The C-shaped heat-shrink tube 150^{g} can have a circular cross section with a diameter D5, that can be squeezed to an elliptical cross-sectional shape having the first and second diameters as mentioned above.

It is to be understood that the dimensions D5 and/or the first and second diameters of the C-shaped heat-shrink tube 150^{g} relate to its initial, pre-shrunk state.

As mentioned above, the commissural support member 128^{c} can have any cross-sectional shape, including a circular cross-section having a uniform diameter Ws, such that W6 = W7 = Ws. In such cases, a circular C-shaped heat-shrink tube 150^{g} will have a diameter D5 which is at least as great as, and preferably greater than Ws. An elliptic C-shaped heat-shrink tube 150^{g} will have each of the first and second diameters at least as great as, and preferably greater than Ws.

The C-shaped heat-shrink tube 150^{g} shown in Fig. 13A can be structured similarly to any of the examples disclosed hereinabove for a C-shaped heat-shrink tube 150^{c}, and include a gap 162 defined between the ends of two opposing side arms 154^{g} thereof (not indicated separately in Figs. 13A-13C, but similar to side arms 154^{c} shown in Figs. 8B-8C), having a gap width G in a free, unbiased state thereof. The side arms 154^{g} on both sides of the gap 162 are resiliently expandable away from each other, allowing a commissural support member 128^{c} to be passable through the gap 162.

In use, the C-shaped heat-shrink tube 150^{g} may be pushed to cover a commissural support member 128^{c}. The gap width G is smaller than the smallest edge of the commissural support member 128^{c}, for example being smaller than lateral width W7. The sidewalls of the commissural support member 128^{c} may apply a force sufficient to expand the side arms 154^{g} of the C-shaped heat-shrink tube 150^{g} away from each other, so as to expand the gap width G and allow passing of the C-shaped heat-shrink tube 150^{g} over the commissural support member 128^{c}. Once the commissural support member 128^{c} is completely accommodated within the C-shaped heat-shrink tube 150^{g}, and in the absence of further expanding force applied to its side arms 154^{g}, the side arms 154^{g} resiliently snap back toward each other to return the gap 162 to the original free gap width G, resulting in the C-shaped heat-shrink tube 150^{g} retained over the commissural support member 128^{c}, as shown in Fig. 13B.

While the gap 162 is described hereinabove facing the side of the commissural support member 128^{c} having a lateral width W7, allowing the C-shaped heat-shrink tube 150^{g} to be pushed there-against, it is to be understood that the gap 162 can be similarly facing the side of the commissural support member 128^{c} having the radial thickness W6, G being for example smaller than W6, allowing the C-shaped heat-shrink tube 150^{g} to be pushed there-against.

The method can further include a step of applying heat to the C-shaped heat-shrink tube 150^{g} utilizing a heat source as mentioned hereinabove, thereby causing it to shrink radially inward and to become tightly attached to and around the commissural support member 128^{c}, as shown in Fig. 13C. The commissure assembly 130 can then be attached to the commissural support member 128^{c} covered by the heat-shrink tube 150^{g}, in a manner similar to that illustrated in Fig. 12D. For example, the tabs 126a and 126b of adjacent leaflets 124a and 124b, respectively, can be folded over the heat-shrink tube 150^{g} covering the commissural support member 128^{c}, and commissural attachment sutures 132 can be utilized to stitch the tabs 126 to each other and to the commissural support member 128^{c} covered by the heat-shrink tube 150^{g}.

It is to be understood that alternative methods can include passing commissural attachment sutures 132 utilized during formation of a commissure assembly 130, through sections of the C-shaped heat-shrink tube 150^{g} after it is passed over the commissural support member 128^{c} as shown in Fig. 13B, for example following stitching patterns that can be similar to those described hereinabove in conjunctions with Figs. 5B-C or 6A-B, *mutatis mutandis.* Such configurations can be advantageous in that shrinking of the heat-shrink tubes 150^{g} may further serve to tighten the commissural attachment sutures 132.

According to some examples, heat-shrink films 150^{b} or C-shaped heat-shrink tubes 150^{c} can be utilized for attachment of a skirt 120 to angled strut sections 112^{b} or 112^{c} of prosthetic valves 100^{b} or 100^{c}, respectively, following any of the attachment examples described hereinabove in conjunction with Figs. 5A-5C, 6A-6B, 7A-7C, or 8A-8D, *mutatis mutandis,* as shown for example in Fig. 12D, showing a plurality of heat-shrink wraps 150^{f}, such as heat shrink wrap 150^{f}a, 150^{f}b and 150^{f}c, tightly shrunk over corresponding strut sections 112^{c}a, 112^{c}b and 112^{c}c, utilized to attached the inner skirt 120, for example along skirt proximal end 121, thereto, via skirt attachment sutures 136a, 136b and 136c. A heat shrink wrap 150^{f} can be implemented as any of the heat shrink film 150^{b} or the C-shaped heat-shrink tube 150^{c} described hereinabove with respect to any of the Figs. 5A-5C, 6A-6B, 7A-7C, or 8A-8D.

It is to be understood that any configuration for covering commissural support members 128 with heat-shrink wraps 150 described hereinabove, such as covering a commissural support members 128^{a} portion of an outer members 140, or other commissural posts attachable to frames 106, with heat-shrink sleeves 150^{a}, as well as covering commissural support members 128^{b} or 128^{c} that can be integrally formed with the frame 106 with heat-shrink sleeves 150^{d}, heat-shrink films 150^{e} or C-shaped heat-shrink tubes 150^{g}, can be combined with any configuration for covering strut segments 112 of such frames with heat-shrink wraps 150 described hereinabove, such as covering strut segments 112^{a}, 112^{b} or 112^{c} with heat-shrink films 150^{b} or C-shaped heat-shrink tubes 150^{c}.

One such example is illustrated in Fig. 12D, showing a single prosthetic valve 100^{c} having its commissural support members 128^{c} covered by heat-shrink wraps 150 such as heat-shrink films 150^{e}, over which commissure assemblies 130 are mounted, as well as having struts sections 112^{c} surrounding its circumference, potentially in a zig-zag pattern, covered by heat-shrink wraps 150^{f}, that can be, for example, a series of heat shrink films 150^{b} or C-shaped heat-shrink tubes 150^{c}, through which or over which the inner skirt 120 is sutured, for example along the skirt proximal end 121.

In some examples, the dimensions of the commissural support members 128 and the strut segments 112 are substantially equal, enabling a single type of heat-shrink wrap 150 to utilized to cover both the commissural support members 128 and the strut segments 112 of the same frame. For example, a prosthetic valve such as prosthetic valve 100^{b} can be provided with axially extending strut segments 113^{b} having substantially equal length, width and thickness as angled strut segments 112^{b}, allowing utilization of heat-shrink films 150^{e} to be utilized to cover both the plurality of axially extending strut segments 113^{b} and attaching commissure assemblies 130 thereto, as well as utilized to cover the plurality of angled strut segments 112^{b} to which the inner skirt 120 it attached. This may advantageously simplify inventory management, including a single type of heat-shrink wrap 150, as well as simplify assembly procedures and efficiency thereof, as attachment of the heat-shrink wraps 150 to the commissural support members 128 and the strut segments 112 can follow similar procedural steps.

Reference is now made to Fig. 14A-14B, showing perspective views of an exemplary example of a balloon-expandable prosthetic valve 100^{d}, with and without soft components, respectively, such as inner skirt 120^{d}, outer skirt 119^{d}, and leaflet assembly 122^{d}. The frame 106^{d} of prosthetic valve 100^{d}, which is shown in Fig. 14B without inner skirt 120^{d}, outer skirt 119^{d}, and leaflet assembly 122^{d} (all of which are shown in Fig. 14A), can be similar to any example disclosed above for prosthetic valve 100^{c}, except that it comprises a plurality of commissural support members 128 in the form of commissure windows 128^{d} (e.g., three are illustrated in the example).

Each commissure window 128^{d} can be in the form of a window or slot defined between two axially extending window strut segments 115, through which portions of the commissural assemblies 130, such as leaflets tabs 126, can extend, so as to couple the commissural segments 130 to the commissure window 128^{d}. The frame 106^{d} can include a plurality of angled strut segments 112^{d} as well as axially extending strut segments 113^{d}, wherein the strut segments 113^{d} are disposed between the commissure windows 128^{d}. Each axially extending window strut segments 115 can be dimensioned similarly to any of the axially extending strut segments 113^{d}, though other configurations are also contemplated.

According to some segments, a heat shrink wrap 150, is tightly disposed around each axially extending window strut segment 115 of a commissure window 128^{d}. Fig. 15 shows an enlarged view of a single commissure window 128^{d}, with two heat shrink wraps 150 tightly disposed around both axially extending window strut segments 115. The heat shrink wrap 150 disposed around each corresponding axially extending window strut segment 115 can be implemented according to any of the various examples described hereinabove. Moreover, alternative or additional heat shrink wraps 150, according to any of the examples described hereinabove, can be utilized to cover any other portions of the frame 106^{d}, such as axially extending strut segments 113^{d} or any of the angled strut segments 112^{d}, which in the interest of brevity will not be further described.

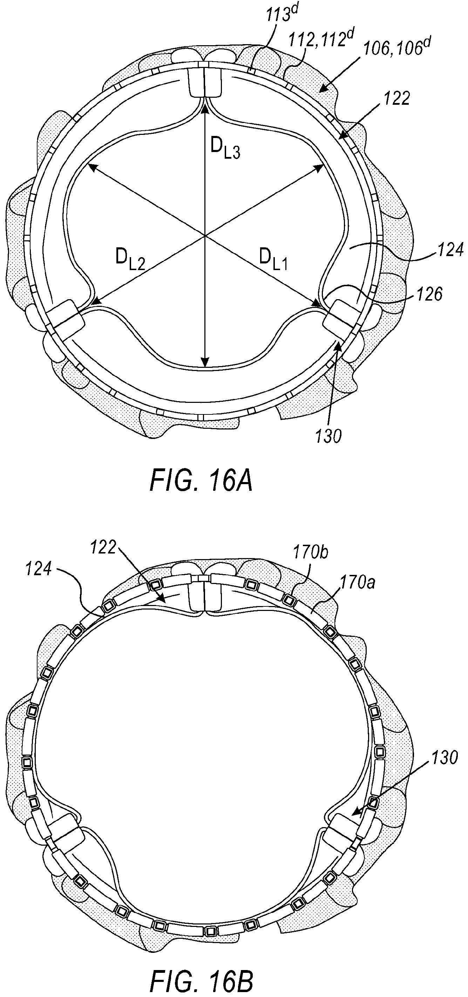

Reference is now made to Figs. 16A-B, showing a comparison between two opening configurations of the leaflets 124, according to some examples. Prosthetic valves are conventionally designed to prevent or minimize contact between the movable portions of the leaflets and the inner surface of the frame. Repeated contact between the movable portions of the leaflets and the metal frame during operation of a prosthetic valve can cause premature wear and eventual failure of the leaflets. Fig. 16A shows an example of a maximal opening of the leaflets during systole of a conventional prosthetic valve, illustrating a gap between the fully-open leaflets 124 and the frame 106.

During valve cycling, the leaflets 124 can articulate at the inner most edges of the tabs 126 of the commissural assemblies 130 as shown in Fig. 16A, which helps space the leaflets 124 away from the frame 106 during normal operation of the prosthetic valve, so as to prevent leaflet contact or abrasion when the leaflets fully open under the flow of blood. The effective full-opening diameter D_{FA} at the outflow of the prosthetic valve shown in Fig. 16A can be estimated or calculated by measuring the distance D_{L} between the articulating end of each commissural assembly 130 and the mid-point of the opposite leaflet, and averaging these distances. For example, three distances D_{L1}, D_{L2} and D_{L3}, illustrated in Fig. 16A, can be averaged to derive the effective full-opening diameter D_{FA}. In other cases, the effective full-opening diameter D_{FA} can be chosen as the widest point of the outflow end of the leaflets structure 122, instead of averaging. The effective full-opening diameter D_{FA} (according to any of the estimation methods described above) can be divided by the frame's inner diameter (in the same expanded configuration) to derive the full-opening diameter ratio R_{FA}.

The effective orifice area (EOA) can be approximated as the area of a circular orifice having an effective full-opening diameter D_{FA} as shown in Fig. 16A. The full-opening diameter ratio R_{FA} of certain types of conventional prosthetic valves (indicative of the EOA) can be in the range of 55-75%. This EOA, which is measured at the valve's outflow, and is narrower than the orifice at the inflow, may result in eddies and turbulence downstream at the outlet of the prosthetic valve, which can produce a relatively high-pressure gradient across the prosthetic valve when the leaflets are open and blood is flowing through the prosthetic valve.

In some examples, prosthetic valve 100 comprises protective covers, such as pads 170 disposed over strut segments 112 of the frame 106, to allow leaflets 124 to open further, as shown in Fig. 16B, optionally to the point of being in contact with the pads 170 on the frame 106. That is, in the fully-open state of the leaflet assembly 122, at least some portions of the leaflets 124 contact at least some of the pads 170 (or other types of protective covers) without contacting the frame 106. The protective covers, such as pads 170, act to protect the leaflets 124 from damage as they open against the frame 106.

The leaflets 124 of the prosthetic valve 100 of the example illustrated in Fig. 6B, comprising protective covers in the form of pads 170, can open to a wider diameter than the leaflets in a design without the pads, illustrated for example in Fig. 16A. The protective covers, such as pads 170, allow the leaflets 124 to open nearly the full inner diameter of the frame 106 at the widest points of the leaflet assembly 122, until portions of the leaflets touch the protective covers (e.g., pads 170). The protective covers, such as pads 170, prevent the leaflets 124 from contacting the metallic or other material of the frame 106 itself. In an examples without pads 170 or any other type of protective covers, the leaflets are designed to open to a narrower diameter, substantially less than the inner diameter of the frame, such that there is an open space between the outflow end of the leaflets 124 and the frame, as described above with reference to Fig. 16A. This protects the edges around the EOA of the leaflet assembly 122 from hitting the frame in a repeated way, such that the leaflets become worn or damaged over time.

In some examples, the protective covers, such as pads 170, allow the leaflets to open to an outflow full-opening diameter D_{FA} of approximately 25% wider than the outflow D_{FA} of a design without the pads. As one non-limiting example, a prosthetic valve having an inner diameter of 25 mm, pads with a thickness of 1 mm each, and a leaflet thickness of 0.25 mm may, in one example, permit the leaflets to open in the outflow end area to a D_{FA} of at least 22.5 mm. In this example, the full-opening diameter ratio R_{FA} is about 90% or greater.

As another non-limiting example, a prosthetic valve having an inner diameter of 18 mm with pads 170 with a thickness of 1 mm and leaflet thickness of 0.25 mm, may permit the D_{FA} to be about 15.5 mm or more, resulting in an R_{FA} of more than about 86% when the leaflets 124 contact the pads 170 that are on the frame 106, with limited or no contact with the frame 106 itself during normal operation of the prosthetic valve 100.

In a further example, a prosthetic valve with an outer diameter of 28.75mm and inner diameter of 27.5mm has a leaflet opening of about 19.5mm in a traditional/conventional design such as illustrated in Fig. 16A . That is, R_{FA} is about 71%. By adding pads to the frame and allowing the leaflets to open to the pads such as in Fig. 16B, the leaflet opening increases to 24.5 mm, resulting in an R_{FA} of about 89%. Consequently, in this example the design of Fig. 16B increases D_{FA} by about 25% compared to a previous design in which the leaflets were constrained from opening to the frame.

In some examples, allowing leaflets 124 to open to pads 170 on a frame 106 may increase the EOA substantially. Based on testing of one 23mm size valve in different configurations and conditions (aortic, mitral, and pulmonic), in one example the EOA is increased by about 25%. Improvement in EOA may vary for different sizes and valve designs.

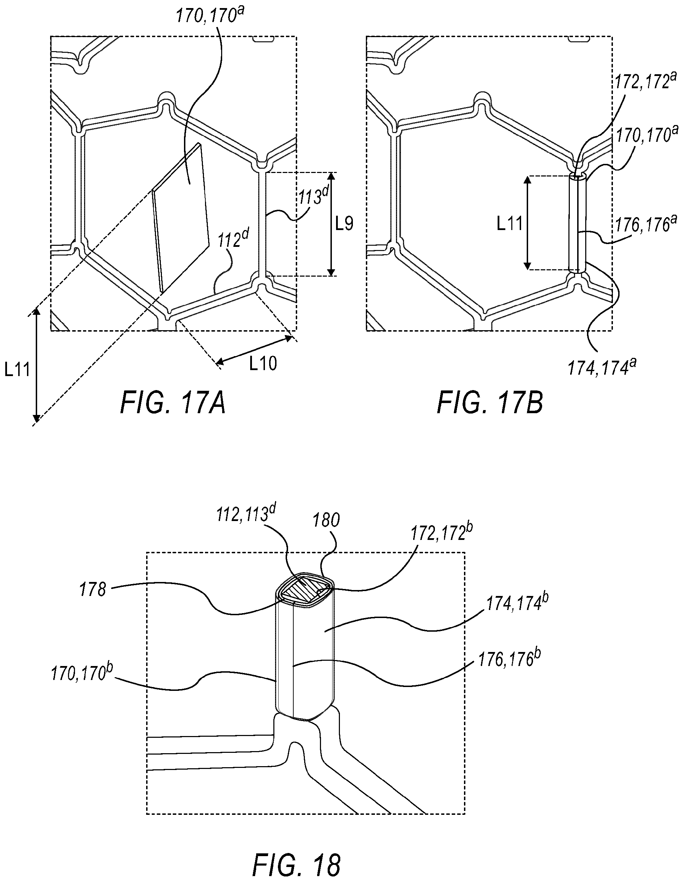

In some examples, a plurality of pads 170 cover the plurality of strut segments 112, such as any of a plurality of angled struts segments 112^{d} and/or a plurality of axially extending strut segments 113^{d}. According to some examples, the pad 170^{a} is provided as a pad sheet, configured to be disposed around and cover a strut segment 112. Reference is now made to Fig. 17A-B. Fig. 17A shows a pad sheet 170^{a} positioned in the vicinity of a strut segment 112 (axially extending strut segment 113^{d} in the illustrated example), and Fig. 17B shows the sheet rolled into a pad 170^{a} covering the strut segment 112. The sheet may be made of, but is not limited to, a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof. Each possibility represents a different example. The sheet may be made of a biocompatible and biodurable material.

As illustrated in Figs. 17A-B, the sheet may be rolled into a suitable cylindrical shape to serve as a pad 170^{a} on the frame of the prosthetic valve. The pads 170^{a} may be secured through such methods as sewing, ultrasonic welding, using adhesives, melting, a combination thereof or other attachment methods known in the art. The adhesives can be biocompatible adhesives. Melting can be performed utilizing laser systems. Ultrasonic welding utilizes highfrequency ultrasonic acoustic vibrations which are directly applied to materials (e.g., plastics) being held together under pressure, to create a solid-state weld. Advantages of ultrasonic welding can include the absence of connective bolts, nails, sutures, or adhesives necessary to join/connect materials together. Additionally, in ultrasonic welding there is no significant temperature increase of the joined materials, thus preventing any unwanted properties which may arise from such temperature increases.

As shown, the pad 170 has an inner surface 172 and an outer surface 174, wherein the inner surface 172 is configured to face the strut segment 112 (e.g., angled strut segment 112^{d} or axially extending strut segment 113^{d}) it is attached to, and at least a portion of the outer surface 174 is configured to face the leaflets 124 (i.e., radially inward, toward the centerline 20 of the frame 106). In some examples, two opposing edges of the sheet are attached to each other to form the rolled configuration of the pad 170^{a} in a manner that forms an attachment line 176, which can be a weld line in the case of ultrasonic welding or melting, a suture line in the case of stitching, and the like. In some examples, where the pads 170 comprise attachment lines 176, each pad 170 is attached to a strut segment 112 such that the attachment line 176 is facing radially outward from the frame 106 (away from the centerline 20 of the frame), leaving a relatively smooth portion of the outer surface 174 facing radially inward (toward the leaflets). This is important to prevent the leaflets 124 from contacting such attachment lines 176 when present, to avoid repetitive abrasion there-against.

The shape of the pad 170 can be different from the shape of the strut segment 112 covered thereby. For example, substantially tubular pads 170 can be utilized to cover struts segments having a rectangular cross-section, as illustrated. In such cases, Each pad 170 is preferably tight enough over the strut segment 112 so as to prevent spontaneous rotation or angular displacement of the pad 170 over the strut covered thereby, to maintain the attachment line 176 oriented at a fixed position facing outward, away from centerline 20.

In some examples, the pad 170 comprises more than one layer, such as a base layer 178 and an outer layer 180 coating the base layer 178, as shown for an example of a coated pad 170^{b} illustrated in Fig. 18. In such examples, the outer layer 180, which can be also referred to as a coating layer, defines the outer surface 174. The base layer 178 can define the inner surface 174^{b}, or can be alternatively coated by another, inner layer (not shown), which can define the inner surface 174^{b}. In some examples, the outer layer 180 and/or the outer surface 174 may be made of, but is not limited to, a polycarbonate, a polyamide, a polyester, polytetrafluoroethylene (PTFE), ePTFE, UHMWPE, a polyolefin, a polyether, a polyurethane, a thermoplastic polyurethane (TPU), or combinations and copolymers thereof. Each possibility represents a different example. The outer layer 180 and/or the outer surface 174 may be made of a biocompatible and biodurable material. The coated pad 170^{b} can be attached to the strut segment 112 by any of the above-mentioned methods, including a sheet rolled in the manner described above with respect to pad 170^{a}, or being directly applied to the frame.

In some examples, a coated pad 170^{b} may be directly applied to the frame by coating the strut segments 112 with base layers 178, and then overcoating the base layers 178 with outer layers 180. In other examples, a base layer 178 can be provided as a sheet and roller over a strut segment 112, in a manner similar to that described for pad 170^{a} (e.g., via ultrasonic welding, stitching, melting, using adhesives, and the like), followed by coating of the rolled base layer 178 by an outer layer 180.

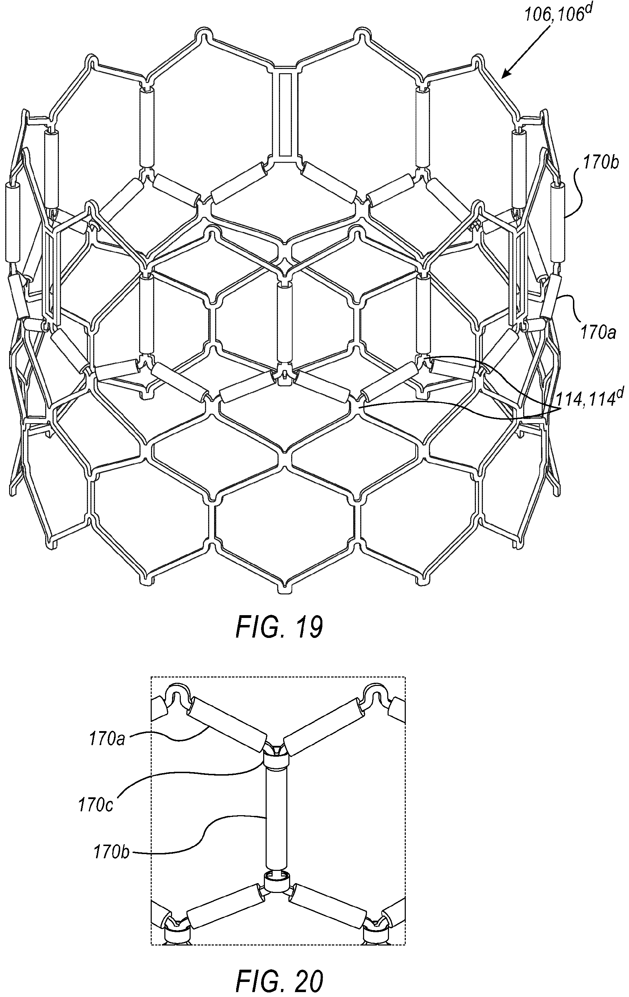

Fig. 19 shows an example of a frame 106^{d} with pads 170a covering angled strut segments 112^{d} and pads 170b covering axially extending strut segments 113^{d}. It is to be understood that examples can include only angled strut segments 112^{d} covered by pad 170a, only axially extending strut segments 113^{d} covered by pads 170b, or both. The number and position of the padded strut segments is preferably selected to cover any struts or strut segments that may be contacted by the leaflets 124 in their fully-open state, as shown in Fig. 16B. For example, in some implementations, only strut segments proximal to the scallop line 134 may be covered by pads 170.

In some examples, the prosthetic valve further includes protective covers disposed over and covering at least some junctions, or portions of junctions. Fig. 20 shows an examples of a frame 106^{d} with pads 170c covering junctions 114^{d}, on top of pads 170a covering angled strut segments 112^{d} and/or pads 170b covering axially extending strut segments 113^{d}. The length of a pad 170c covering a junction 114 can be significantly smaller than the length L11 of any of the pads 170a or 170b, due to the smaller dimensions of the junctions 114 with respect to any type of strut segments 112. A junction-covering pad 170c can be further dimensioned so as to properly cover the junction in a manner that yet is not too tight, so as to allow movement of the immediate portions extending from the junction between the compressed and expanded configurations. A valve provided with several types of junctions can include several types of pads 170c to match the size and/or shape of each type of junction. While shown in Fig. 20 only for non-apical junctions, it is to be understood that pads 170c can similarly cover apices of the frame, such as outflow apices 116.

With reference back to Fig. 17A, an axially extending strut segment 113^{d} to which the pad 170b may be coupled, can have a axially extending strut length L9, which can be similar, in some examples, to the support member length L7 described for a support member in conjunction with Fig. 10A. Similarly, an angled strut segment 112^{d} can have a length L10, which can be similar to length L3 described in conjunction with Fig. 5A. Lengths L9 and L10 can be similar or different from each other. A single frame 106 can have several groups of strut segment, each provided with a different length. For example, frame 106^{d} shown in Fig. 14B can have upper axially extending strut segments 113^{d}a having a length L9 that is greater than that of lower axially extending strut segments 113^{d}b. While lower axially extending strut segments 113^{d}b will not necessarily be covered by protective covers (such as pads 170), as they are positioned at regions of the frame 106^{d} that may not necessarily be contacted by the leaflets 124 during cycling of the leaflet assembly 124, this may serve to illustrate optional variance in strut segment lengths. Similarly, a frame 106 can include several lengths L10 of angled strut segments (not shown).

Each pad 170^{a} (or 170^{b}) can be provided as a rectangular sheet configured to cover a single strut segment 112, having a sheet length L11 as shown in Fig. 17A, which can be similar to lengths L4 or L8 described in conjunctions with Figs. 5A and 12A, respectively. The sheet length L11 is not longer than the length of the strut segment it is supposed to cover. In some examples, the sheet length L11 is substantially equal to the length L9 of the axially extending strut segment 113^{d} it is configured to cover, such that once it is disposed over the axially extending strut segment 113^{d}, it covers it along its entire length. In some examples, the sheet length L11 is substantially equal to the length L10 of the angled strut segment 112^{d} it is configured to cover, such that once it is disposed over the angled strut segment 112^{d}, it covers it along its entire length. In some examples, the valve includes more than one type of pad 170, each type having a different sheet length L11, which is not longer than the length of the strut segment it covers when attached thereto.

While pads 170 are illustrated and described throughout Figs. 16A-18 in combination with prosthetic valve 100^{d} illustrated in Figs. 14A-B, it is to be understood that the pads 170 can be coupled to strut segments of any other type of frame 106 of a prosthetic valve 100, such as frame 106^{a}, frame 106^{b}, frame 106^{c}, as well as other types of frames of any other type of a prosthetic valves that comprises a leaflet assembly mounted within its frame.

In some examples, the protective covers attached to strut segment 112 configured to reduce abrasive damage to the leaflets 124, are heat shrink wraps 150 of any of the types described hereinabove, (including, for example, heat-shrink films 150^{b}, semi-rigid C-shaped heat-shrink tubes 150^{c}, heat-shrink films 150^{e}, heat shrink wraps 150^{f} and/or C-shaped heat-shrink tubes 150^{g}). Such heat-shrink wraps 150 can be attached to axially extending strut segments and/or angled strut segments according to any of the attachment methods described above in conjunction with Figs. 5A-13C, *mutatis mutandis,* instead of, or in addition to, pad 170, and may serve to protect the leaflets 124 from damage as the open against the frame 106.

In some examples, the pad 170 or at least a portion thereof (e.g., the outer surface 174 and/or the outer layer 180) is characterized by having certain surface or material features, wherein said features are configured to reduce abrasive damage which may be caused to the leaflets 124 when opened against the frame so as to contact the pads 170, as disclosed hereinabove. In further such examples, the above-mentioned portion of the pad 170, such as the outer surface 174, is characterized by having the desired features, and is configured to at least partially face the leaflets 124 (i.e., radially inward, toward the centerline 20 of the frame 106). The desired features of the pad 170 or a portion thereof can be attributed to a pad sheet (e.g., pad sheet 170^{a}) prior to becoming disposed around a strut segment 112, and/or to the pad 170 or a portion thereof already covering a strut segment 112. The desired features can be selected from, but not limited to, Shore hardness, surface roughness, abrasion resistance, coefficient of friction, water absorption, vicat softening temperature, melt flow index, flexural strength, tensile strength, tensile strain at the failure point, secant tensile modulus, number average molecular weight (Mn), weight average molecular weight (Mw), and combinations thereof. Each option represents a different example.

Hardness of plastics can be determined by a Shore (Durometer) Hardness test, which measures the resistance of a plastic material (e.g., elastomer) toward indentation. Shore hardness is typically categorized on either a Shore A or Shore D scale by using a durometer apparatus, which penetrates the sample material. The Shore A Hardness Scale measures the hardness of flexible rubbers that range in hardness from very soft and flexible (having the lower values of the scale), to medium and somewhat flexible, and even hard or semi-rigid with almost no flexibility at all (having the top values of the scale). The Shore D Hardness Scale measures the hardness of hard rubbers, semi-rigid plastics and hard plastics. Each scale results in a value between 0 and 100, with higher values indicating a harder material. Certain materials may have corresponding or overlapping values of both Shore A and D scales.

In some examples, the pad 170 or a portion thereof (i.e., the outer surface 174 and/or the outer layer 180) is characterized by having a durometer hardness ranging from about 40 Shore A to about 100 Shore A. According to further examples, the durometer hardness of the pad 170 or a portion thereof ranges from about 40 Shore A to about 98 Shore A. According to still further examples, the durometer hardness of the pad 170 or a portion thereof ranges from about 40 Shore A to about 95 Shore A. According to some examples, the pad 170 or a portion thereof is characterized by having a durometer hardness of from about 0 Shore D to about 55 Shore D. According to further examples, the durometer hardness of the pad 170 or a portion thereof ranges from about 0 Shore D to about 50 Shore D. According to some examples, the pad 170 or a portion thereof is characterized by having a durometer hardness ranging from about 40 Shore A to about 98 Shore A, and/or from about 0 Shore D to about 55 Shore D.

Surface roughness is a component of surface texture. It is quantified by the deviations in the direction of the normal vector of a real surface from its ideal form. If these deviations are large, the surface is considered rough, and if they are small, the surface is considered smooth. Therefore, the term "smooth", as used herein refers to a surface having minor deviations in the direction of the normal vector of a real surface from its ideal form. Smooth surfaces are substantially unitary/continuous surfaces, free from irregular voids. The term "smooth" is not intended to be limited to the narrow meaning of a substantially planar surface devoid of surface irregularities.

Surface roughness is typically calculated by a method termed "Ra" or roughness average, which represents the arithmetic average of a set of individual measurements of surfaces peaks and valleys (e.g., normal vectors), relative to a mean line (e.g., a real surface), wherein low Ra values represents smooth surfaces.

In some examples, the pad 170 or a portion thereof (i.e., the outer surface 174 and/or the outer layer 180) is characterized by having a Ra value of about 0.2 µm or less, which corresponds to a roughness grade number of N4 (for example, as indicated by ISO 1302:1992). In further examples, the Ra value of the pad 170 or a portion thereof is below about 0.2 µm. In further examples, the outer surface 174 is characterized by having a smooth surface having a Ra value of about 0.2 µm or less.

In some examples, the pad 170 or a portion thereof (i.e., the outer surface 174 and/or the outer layer 180) is characterized by having a Ra value that is 90% or less of the Ra value of a surface of the frame 106. In further examples, the pad 170 or a portion thereof has a Ra value that is 80% or less of the Ra value of the surface of the frame 106. In still further examples, the pad 170 or a portion thereof has a Ra value that is 50% or less of the Ra value of the surface of the frame 106.

For example, if the surface of the frame 106 has a Ra value of 0.5 µm, and the outer surface 174 of the pad has a Ra value that is 80% than the Ra value of the frame 106, then the outer surface 174 will have a Ra value of 0.4 µm. Similarly, if the outer surface 174 of the pad has a Ra value that is 50% than the Ra value of the frame 106, then the outer surface 174 will have a Ra value of 0.25 µm.

Moreover, for example, if the surface of the frame 106 has a Ra value of 0.3 µm, and the outer surface 174 of the pad has a Ra value that is 80% than the Ra value of the frame 106, then the outer surface 174 will have a Ra value of 0.24 µm. Similarly, if the outer surface 174 of the pad has a Ra value that is 50% than the Ra value of the frame 106, then the outer surface 174 will have a Ra value of 0.15 µm.

Resistance to abrasion is defined as the ability of a material to withstand mechanical action such as rubbing, scraping, erosion, and the like. Resistance to abrasion can be evaluated by estimating the resistance of transparent plastics to one kind of surface abrasion by measuring the change in optical properties between an abraded and unabraded specimen, utilizing a Taber abrase for 1000 cycles (For example, as indicated by active standard: ASTM D1044). Low abrasion resistance values are correlative to smooth surfaces.

In some examples, the pad 170 or a portion thereof (i.e., the outer surface 174 and/or the outer layer 180) is characterized by having an abrasion resistance value of less than about 0.1%, preferably less than about 0.05%. In further examples, the outer surface 174 is characterized by having a smooth surface having an abrasion resistance value of less than about 0.05%.

The kinetic coefficient of friction is a value which is related to the slip properties of plastic films, and it can be tested by sliding one surface (e.g., plastic films or sheets) over itself or over another surface (for example, as indicated by active standard: ASTM D-1894). Low coefficients can be correlative to smooth surfaces.

In some examples, the pad 170 or a portion thereof (i.e., the outer surface 174 and/or the outer layer 180) is characterized by having a kinetic coefficient of friction in the range of about 0.1-2. In further examples, the outer surface 174 is characterized by having a smooth surface having a kinetic coefficient of friction in the range of about 0.3-1.7.

Water absorption is used to determine the amount of water absorbed into plastics under specified conditions, and can provide information regarding the performance of the materials in water, aqueous or humid environments (for example, as indicated by active standard: ASTM D570). Factors affecting water absorption into plastics include: type of material, additives used, temperature and length of exposure, etc. Typically, a material having a low water absorption capacity will exhibit reduced deformation or shape distortion as a result of water absorption within an aquas environment.

In some examples, the pad 170 or a portion thereof (i.e., the outer surface 174 and/or the outer layer 180) is characterized by having a water absorption capacity of less than 5%. In further examples, the water absorption capacity of less than 3%. In still further examples, the water absorption capacity of less than 1.5%. In yet still further examples, the water absorption capacity of less than 0.5%.

Vicat softening temperature (or point) is the temperature at which a flat-ended needle penetrates a polymer material to the depth of 1 mm under a specific load (for example, as indicated by active standard: ASTM D1525). The Vicat softening temperature reflects the point of softening to be expected when a material is used in an elevated temperature application. For example, the Vicat softening temperature can reflect a temperature or a range of temperatures under which a pad 170 and/or a pad sheet (e.g., pad sheet 170^{a}) can be manufactured and/or processed (e.g., to become disposed around, cover and attached to a strut segment 112) to have desired qualities or characteristics.

In some examples, the pad 170, a portion of pad 170 (i.e., the outer surface 174 and/or the outer layer 180), or a pad sheet (e.g., pad sheet 170^{a}) is characterized by having a Vicat softening temperature selected from the range of 130-200 °C. In further examples, the Vicat softening temperature is selected from the range of 150-180 °C.

Melt Flow Index (MFI) measures the flow rate of a melted thermoplastic polymer material via extrusion. It is defined as the mass of polymer, in grams, flowing in ten minutes through an extruder's orifice at a prescribed temperature and load (for example, as indicated by active standard: ASTM D1238). Typically, melt flow rate is an indirect measure of molecular weight, with high melt flow rate corresponding to low molecular weight, and can provide an indication of the desired qualities or characteristics of the final polymeric product.

In some examples, the pad 170, a portion of pad 170 (i.e., the outer surface 174 and/or the outer layer 180), or a pad sheet (e.g., pad sheet 170^{a}) is characterized by having an MFI in the range of 4-8 g/10 min, wherein the MFI is indicative of the extrusion rate of the material through an orifice at a temperature of about 210 °C and a load of 2.172 kg. In further examples, the MFI is in the range of 5-6 g/10 min.

Flexural strength test measures the force required to bend a polymer material under three point loading conditions. Typically, the tested material lies on a support span and the load is applied to the center by a loading nose producing three point bending at a specified rate, wherein the test is stopped when the tested material reaches 5% deflection or the tested material breaks before 5% (for example, as indicated by active standard: ASTM D790). Flexural strength modulus is used as an indication of a material's stiffness when flexed. For example, the flexural strength can provide an indication for the desired strength of a pad sheet (e.g., pad sheet 170^{a}) configured to be wrapped around a strut segment 112.

In some examples, the pad 170, a portion of pad 170 (i.e., the outer surface 174 and/or the outer layer 180), or a pad sheet (e.g., pad sheet 170^{a}) is characterized by having a flexural strength in the range of about 1,000-20,000 psi. In further examples, the flexural strength in the range of about 4,000-15,000 psi. In still further examples, the flexural strength in the range of about 5,000-10,000 psi.

The tensile properties of a polymer material define the ability of a material to resist failure under tension (for example, as indicated by active standard: ASTM D638). A stress-strain curve for a polymeric material represents the relationship between stress and strain, and is obtained by gradually applying a load to a test material and measuring the deformation, from which the stress and strain can be determined. Typically, this curve can reveal many of the properties of a material, such as the tensile strength, tensile strain, secant modulus, and the like. Tensile strength (TS) is the maximum stress that a material can withstand while being stretched or pulled before reaching failure (through deformation, rupture or breaking). Nominal strain at break is the tensile strain at the tensile strength at the failure point. Secant tensile modulus value is the slope of a line connecting the point of zero strain to a point on the stress-strain curve at a specified strain.

In some examples, the pad 170, a portion of pad 170 (i.e., the outer surface 174 and/or the outer layer 180), or a pad sheet (e.g., pad sheet 170^{a}) is characterized by having a tensile strength in the range of about 5,000-10,000 psi. In further examples, the tensile strength is in the range of about 6,000-9,000 psi.

In some examples, the pad 170, a portion of pad 170 (i.e., the outer surface 174 and/or the outer layer 180), or a pad sheet (e.g., pad sheet 170^{a}) is characterized by having a tensile strain at the failure point of about 200-500 %.

In some examples, the pad 170, a portion of pad 170 (i.e., the outer surface 174 and/or the outer layer 180), or a pad sheet (e.g., pad sheet 170^{a}) is characterized by having a secant tensile modulus in the range of about 500-3,000 psi, for a tensile strain of about 100 % or 300 %. In further examples, the secant tensile modulus is in the range of about 700-2,400 psi, for a tensile strain of about 100 % or 300 %.

Gel permeation chromatography (GPC) is a type of size exclusion chromatography that can separate analytes on the basis of size, typically used for the analysis of polymer materials which can be characterized by a number average molecular weight (Mn), a weight average molecular weight (Mw), and more.

In some examples, the pad 170 or a pad sheet (e.g., pad sheet 170^{a}) is characterized by having a number average molecular weight (Mn) selected from the range of 50,000-100,000. In further examples, the number average molecular weight (Mn) is selected from the range of 70,000-90,000.

In some examples, the pad 170 or a pad sheet (e.g., pad sheet 170^{a}) is characterized by having a weight average molecular weight (Mw) selected from the range of 150,000-350,000. In further examples, the weight average molecular weight (Mw) is selected from the range of 200,000-300,000.

It is contemplated that some of the desired surface or material features as presented hereinabove are correlative to the smoothness and/or hardness levels of the pad 170 or a portion thereof facing the leaflets 124. Advantageously, in some examples, a pad 170 and/or an outer surface 174 having at least one or a combination of the desired surface or material features as presented hereinabove, can effectively reduce abrasive damage which may be caused to the leaflets 124 during the utilization of the heart valve. For example, a pad 170 and/or an outer surface 174 having a smooth surface (which faces the leaflets 124) is considered to be preferable, since it can further reduce any abrasive damage as disclosed above.

In some examples, at least one of the pad 170, the outer surface 174, and the pad sheet 170^{a} is characterized by having at least one property selected from: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a flexural strength in the range of about 5,000-10,000 psi; a tensile strength in the range of about 6,000-9,000 psi; a tensile strain at the failure point of about 200-500 %; a secant tensile modulus in the range of about 700-2,400 psi, for a tensile strain of about 100 % or 300 %; a number average molecular weight (Mn) selected from the range of 70,000-90,000; a weight average molecular weight (Mw) selected from the range of 200,000-300,000, and combinations thereof.

In some examples, at least one of the pad 170, the outer surface 174, and the pad sheet 170^{a} is characterized by being biodurable.

As used herein, the term "biodurable" refers to preservation of one or more physical properties, one or more mechanical properties, one or more chemical properties, or any combination thereof, during exposure to a biological environment or a biologically similar environment over a period of time. The biodurable materials of the present invention are characterized by being able to preserve at least 80% of their properties as disclosed herein, during exposure to the biological environment over a minimal period of time. A minimal period of time can comprise about 6 months, about 1 year, about 5 years, or more. In some examples, biodurable materials as presented herein are configured to preserve at least 80% of their properties after a predetermined number of test cycles (such as for example, 400 or 600 million cycles) within a system that simulates the physiological conditions of a patient's body.

For example, following the implantation of a prosthetic valve within the implantation site as disclosed herein, the biodurable materials of pads 170 can maintain at least 80% of their Ra value (of about 0.2 µm or less), for a minimal period of time such as, for example, at least one year, at least five years, or more.

In some examples, at least one of the pad 170, the outer surface 174, and the pad sheet 170^{a} is biodurable, adapted to preserve at least 80%, at least 90%, or more, of one or more properties thereof during exposure to biological environment over a minimal period of time. The preserved properties can include at least one of: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a flexural strength in the range of about 5,000-10,000 psi; a tensile strength in the range of about 6,000-9,000 psi; a tensile strain at the failure point of about 200-500 %; a secant tensile modulus in the range of about 700-2,400 psi, for a tensile strain of about 100 % or 300 %; a number average molecular weight (Mn) selected from the range of 70,000-90,000; a weight average molecular weight (Mw) selected from the range of 200,000-300,000, and combinations thereof.

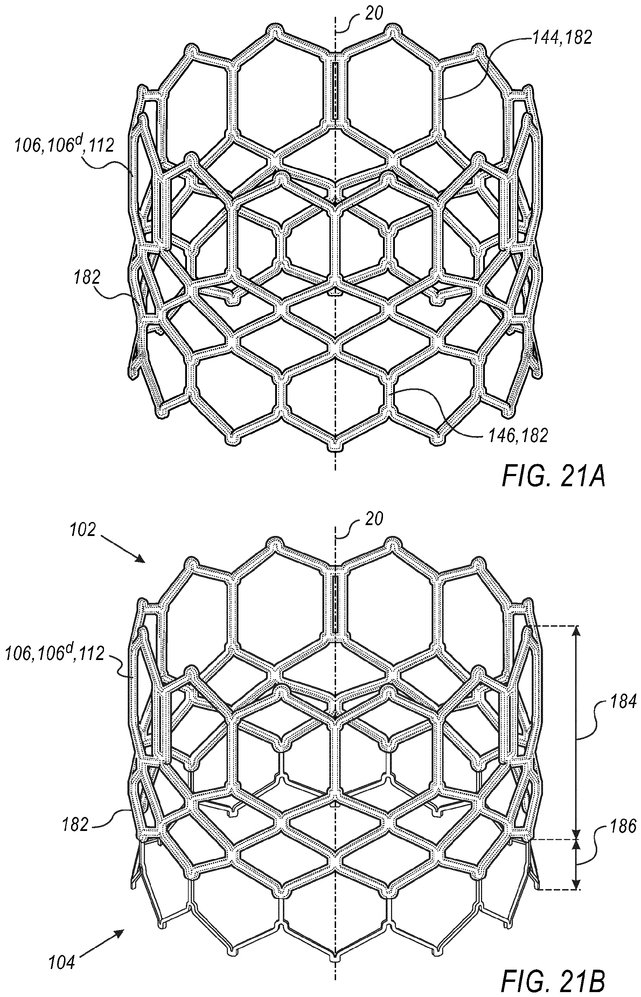

Reference is now made to Figs. 21A-D, showing views in perspective of a frame 106 (e.g., frame 106^{d}) having various portions thereof coated by a coating 182. For ease of illustration, coating 182 is drawn disproportionately thick relative to the thickness of frame 106 and/or strut segments 112.

According to an aspect of the invention, there is provided a coating 182, which is configured to be applied to the frame 106 or to a portion thereof by a coating technique selected from, but not limited to, electrospinning, brushing, spray-coating, dip coating, combinations thereof, or any other suitable coating technique. Each possibility represents a different example.

In some examples, the frame 106 (e.g., frame 106^{d}) has an inner frame surface 144 and an outer frame surface 146, wherein the inner frame surface 144 is facing toward the centerline 20 of the valve, and the outer frame surface 146 is facing outward (i.e., in the opposite direction, away from the centerline 20).

In some examples, the frame 106 comprises a coating 182, wherein the coating covers both of the inner frame surface 144 and the outer frame surface 146, as illustrated in Fig. 21A.

In some examples, a portion of the frame 106, defining a portion of the inner frame surface 144 and a corresponding portion of the outer frame surface 146, comprises the coating 182, wherein said coated frame portion is preferably a portion that may be contacted by the leaflets 124 in their fully-open state, as illustrated in Fig. 21B. In further examples, the coating 182 covers a portion of the inner frame surface 144 and a corresponding portion of the outer frame surface 146, wherein the leaflet assembly 122 is configured to be secured to the frame such that the leaflets 124 may contact only the portion of the inner frame surface 144 comprising the coating 182, in their fully-open state.

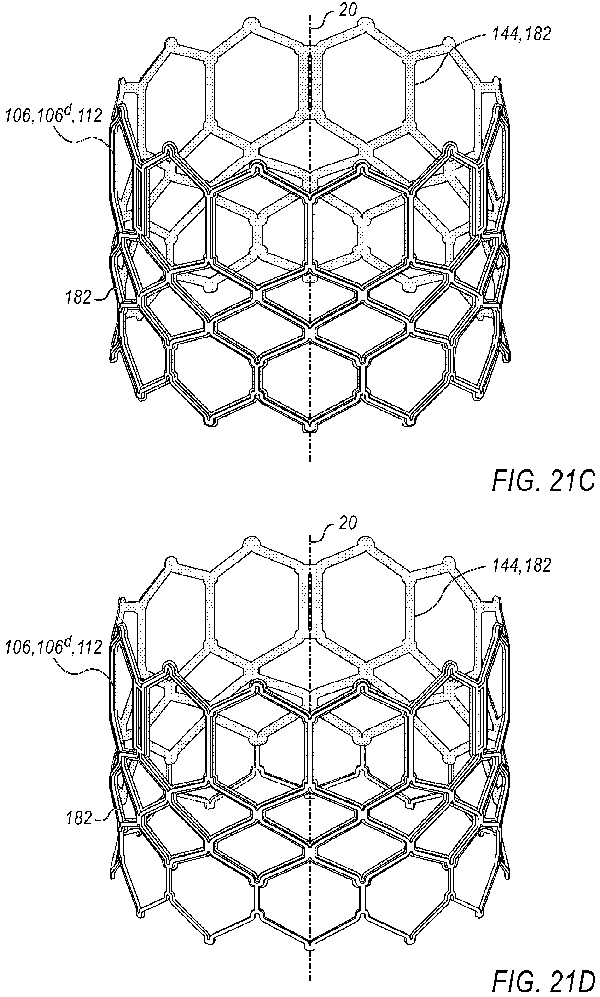

In some examples, the frame 106 comprises the coating 182, wherein the coating covers solely the inner frame surface 144, as illustrated in Fig. 21C.

In some examples, a portion of the frame 106 comprises the coating 182 disposed solely over the inner frame surface 144, wherein said coated frame portion is a portion that may be contacted by the leaflets 124 in their fully-open state, as illustrated in Fig. 21D. In further examples, the coating 182 covers a portion of the inner frame surface 144, wherein the leaflet assembly 122 is configured to be secured to the frame such that the leaflets 124 may contact only the portion of the inner frame surface 144 comprising the coating 182, in their fully-open state.

The coating 182 can be made of various suitable biocompatible synthetic materials, such as, but not limited to, a thermoplastic material. Suitable thermoplastics biocompatible materials are selected from, but not limited, polyamides, polyesters, polyethers, polyurethanes, polyolefins (such as polyethylene and/or polypropylenes), polytetrafluoroethylenes, and combinations and copolymers thereof. Each possibility represents a different example. In further examples, the thermoplastic material comprises a thermoplastic elastomer (TPE). In still further examples, the thermoplastic elastomer is selected from the group consisting of: thermoplastic polyurethane (TPU), styrene block copolymers (TPS), Thermoplastic polyolefinelastomers (TPO), thermoplastic vulcanizates (TPV), thermoplastic copolyester (TPC), thermoplastic polyamides (TPA), and combinations and variations thereof. Each possibility represents a different example. According to some examples, the coating 182 comprises a thermoplastic elastomer comprising TPU.

In some examples, the coating 182 comprises at least one material selected from a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof. Each possibility represents a different example.

In some examples, the coating 182 comprises TPU. In some examples, the coating 182 comprises TPU and at least one biocompatibility-enhancing polymer. In some examples, the coating 182 comprises TPU which is laminated with a UHMWPE layer for surface modification.

As used herein, the term "biocompatibility-enhancing" refers to a polymer which can enhance/improve biocompatible properties of other polymers.

In some examples, the coating 182 comprises polycarbonate urethane (PCU). In some examples, the coating 182 does not comprise poly(ether urethane) and/or poly(ether urethane urea).

In some examples, the coating 182 comprises a polyester layer which can be coated with at least one biocompatibility-enhancing polymer.

In some examples, the coating 182 is non-biodegradable.

In some examples, the coating 182 is non-porous. In some examples, the coating 182 is a uniform, continuous, smooth coating.

In some examples, the coating 182 covers the frame 106 or a portion thereof, thereby forming a polymeric covering layer attached thereto, which can be characterized by having certain surface features. Said features are configured to reduce abrasive damage which may be caused to the leaflets 124 when opened against the frame, as disclosed herein above similarly in context of pad 170. The desired features can be selected from, but not limited to, Shore hardness, surface roughness, abrasion resistance, coefficient of friction, water absorption, vicat softening temperature, melt flow index, tensile strength, tensile strain at the failure point, secant tensile modulus, and combinations thereof. Each option represents a different example.

In some examples, the coating 182 is applied to the frame 106 prior to the attachment of the leaflet assembly 122 thereto, thus enabling to form the polymeric covering layer attached to the frame 106 or a portion thereof having the desired surface features as disclosed herein.

In some examples, the coating 182 forms a polymeric covering layer attached to the frame 106 or a portion thereof, and is characterized by having a durometer hardness ranging from about 40 Shore A to about 100 Shore A. According to further examples, the durometer hardness of the coating 182 ranges from about 40 Shore A to about 98 Shore A. According to still further examples, the durometer hardness of the coating 182 ranges from about 40 Shore A to about 95 Shore A. According to some examples, the coating 182 is characterized by having a durometer hardness of from about 0 Shore D to about 55 Shore D. According to further examples, the durometer hardness of the coating 182 ranges from about 0 Shore D to about 50 Shore D. According to some examples, the coating 182 is characterized by having a durometer hardness ranging from about 40 Shore A to about 98 Shore A, and/or from about 0 Shore D to about 55 Shore D.

In some examples, the coating 182 forms a polymeric covering layer attached to the frame 106 or a portion thereof, and is characterized by having a Ra value of about 0.2 µm or less, which corresponds to a roughness grade number of N4. In further examples, the Ra value of the coating 182 is below about 0.2 µm.

In some examples, the coating 182 forms a polymeric covering layer attached to the frame 106 or a portion thereof, and is characterized by having a Ra value that is 90% or less of the Ra value of the frame 106. In further examples, the coating 182 forms a polymeric covering layer having a Ra value that is 80% or less of the Ra value of the surface of the frame 106. In still further examples, the coating 182 forms a polymeric covering layer having a Ra value that is 50% or less of the Ra value of the surface of the frame 106. For example, if the surface of the frame 106 has a Ra value of 0.5 µm, and the coating 182 forms a polymeric covering layer having a Ra value that is 80% than the Ra value of the frame 106, then the polymeric covering layer will have a Ra value of 0.4 µm. Similarly, if the coating 182 forms a polymeric covering layer having a Ra value that is 50% than the Ra value of the frame 106, then the polymeric covering layer will have a Ra value of 0.25 µm. Moreover, for example, if the surface of the frame 106 has a Ra value of 0.3 µm, and the coating 182 forms a polymeric covering layer having a Ra value that is 80% than the Ra value of the frame 106, then the polymeric covering layer will have a Ra value of 0.24 µm. Similarly, if the coating 182 forms a polymeric covering layer having a Ra value that is 50% than the Ra value of the frame 106, then the polymeric covering layer will have a Ra value of 0.15 µm.

In some examples, the coating 182 is characterized by having an abrasion resistance value of less than about 0.1%, preferably less than about 0.05%.

In some examples, coating 182 is characterized by having a kinetic coefficient of friction in the range of about 0.1-2. In further examples, the coating 182 is characterized by having a smooth surface having a kinetic coefficient of friction in the range of about 0.3-1.7.

In some examples, the coating 182 is characterized by having a water absorption capacity of less than 5%. In further examples, the water absorption capacity is less than 3%. In still further examples, the water absorption capacity is less than 1.5%. In yet still further examples, the water absorption capacity is less than 0.5%.

In some examples, the coating 182 is characterized by having a Vicat softening temperature selected from the range of 130-200 °C. In further examples, the Vicat softening temperature is selected from the range of 150-180 °C.

In some examples, the coating 182 forms a polymeric covering layer attached to the frame 106 or a portion thereof, and is characterized by having an MFI in the range of 4-8 g/10 min, wherein the MFI is indicative of the extrusion rate of the material through an orifice at a temperature of about 210 °C and a load of 2.172 kg. In further examples, the MFI is in the range of 5-6 g/10 min.

In some examples, the coating 182 forms a polymeric covering layer attached to the frame 106 or a portion thereof, and is characterized by having a tensile strength in the range of about 5,000-10,000 psi. In further examples, the tensile strength is in the range of about 6,000-9,000 psi.

In some examples, coating 182 forms a polymeric covering layer attached to the frame 106 or a portion thereof, and is characterized by having a tensile strain at the failure point of about 200-500 %.

In some examples, coating 182 forms a polymeric covering layer attached to the frame 106 or a portion thereof, and is characterized by having a secant tensile modulus in the range of about 500-3,000 psi, for a tensile strain of about 100 % or 300 %. In further examples, the secant tensile modulus is in the range of about 700-2,400 psi, for a tensile strain of about 100 % or 300 %.

It is contemplated that some of the desired surface features as presented hereinabove are correlative to the smoothness and/or hardness levels of the coating 182 or portions thereof which faces the leaflets 124. Advantageously, in some examples, a coating 182 having at least one or a combination of the desired surface features as presented herein above, can effectively reduce abrasive damage which may be caused to the leaflets 124 during utilization of the heart valve. For example, a coating 182 having a smooth surface (which faces the leaflets 124) is considered to be preferable, since it can further reduce any abrasive damage as disclosed above.

In some examples, the coating 182 forms a polymeric covering layer attached to the frame 106 or a portion thereof, and is characterized by having at least one property selected from: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

In some examples, the coating 182 is biodurable, adapted to preserve at least 80%, at least 90%, or more, of one or more properties thereof during exposure to biological environment over a minimal period of time. The preserved properties can include at least one of: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a flexural strength in the range of about 5,000-10,000 psi; a tensile strength in the range of about 6,000-9,000 psi; a tensile strain at the failure point of about 200-500 %; a secant tensile modulus in the range of about 700-2,400 psi, for a tensile strain of about 100 % or 300 %; a number average molecular weight (Mn) selected from the range of 70,000-90,000; a weight average molecular weight (Mw) selected from the range of 200,000-300,000, and combinations thereof.

**In** some examples, coating 182 is applied to the frame 106 or a portion thereof by a dip coating process comprising: (a) providing a frame 106 of a prosthetic valve 100 as disclosed above at an expanded configuration, prior to attachment of leaflet assembly 122 thereto.

**In** further examples, the dip coating process further comprises: (b) immersing the frame 106 of step (a) in a solution comprising a coating material, and maintaining the frame 106 therein. In some examples, the immersion is performed at a constant speed, thereby reducing the risk of forming uneven or deformed shapes on the surface of the immersed frame 106. The frame 106 of step (a) can be pre-heated prior to its immersion during step (b). Alternatively or additionally, the coating material solution can be heated prior to the immersion of the frame 106 therein.

**In** further examples, the dip coating process further comprises: (c) extracting the frame 106 from within the coating material solution, wherein a thin coating layer forms on the surface of the frame 106 during its extraction. In some examples, the extraction is performed at a constant speed, thereby reducing the risk of forming uneven coating on the surface of the immersed frame 106.

**In** further examples, the dip coating process further comprises: (d) solidifying the coating layer formed on the surface of the frame 106, thereby forming the coating 182 thereon. The solidification can include draining excess liquid from the surface of the solidified coating 182, and/or evaporating unwanted solvents therefrom.

**In** some examples, the coating 182 covers the entire frame 106, including both of the inner frame surface 144 and the outer frame surface 146.

In some alternative examples, the coating 182 covers a portion of the frame 106, such as a portion of the inner frame surface 144 and optionally a corresponding portion of the outer frame surface 146. In further such examples, step (a) further comprises masking a portion of the frame 106, so that the frame 106 is provided having a first unmasked portion 184 and a second masked portion 186. In further such examples, the first unmasked portion 184 is preferably the portion of the frame 106 configured to be contacted at least partially by leaflets 124 in their fully-open state, such as a portion of the frame 106 proximal to the scallop line 134, wherein the second masked portion 186 can be along a portion of the frame 106 distal to the scallop line 134, such as a region between the scallop line 134 and the inflow end 104 of the frame 106. Masking can be performed by attaching a masking layer (e.g., a removable adhesive layer) to the second masked portion 186.

In further such examples, step (c) comprises forming a thin coating layer on the first unmasked portion 184 and the second masked portion 186 comprising the masking layer, during the extraction of the frame 106 from within the coating material solution. In still further such examples, step (d) further comprises removing the masking layer from the second masked portion 186, following the formation of the solidified coating 182 on the first unmasked portion 184, thereby exposing an unmasked portion of the frame 106. Thus, step (d) results in the formation of a thin coating layer (i.e., coating 182) on the first unmasked portion 184 and an uncoated second masked portion 186 of the frame 106, as illustrated for example in Fig. 21B.

By utilizing a masking layer, portions of frame 106 which are not required to become coated by coating 182 can remain uncoated, and therefore the dip coating process as presented herein can be utilized as a targeted coating process for covering desired portions of the frame 106 or any other portion of the valve that can be contacted by the leaflets 124 in their fully-open state.

In some examples, coating 182 is applied to the frame 106 or a portion thereof by a spray-coating process comprising: (a) providing a frame 106 of a prosthetic valve 100 as described above at an expanded configuration, prior to attachment of leaflet assembly 122 thereto.

In some examples, the spray-coating process further comprises: (b) spray-coating the inner frame surface 144, and optionally the outer frame surface 146 of frame 106, utilizing at least one coating nozzle, thereby forming a coating layer thereon.

In some examples, the coating nozzle is a spray nozzle, which is configured to facilitate the dispersion of a coating liquid into a spray (i.e., to cause atomization), in order to enable the formation of the coating layer on the desired surface(s) of frame 106, during the spray-coating process as disclosed herein. In further examples, step (b) comprise spray-coating the inner frame surface 144, and optionally the outer frame surface 146, utilizing a spray nozzle, wherein the spray-coating technique is selected from electrical charge (electrostatic) coating, ultrasound (ultrasonic) coating, gas spray coating, and the like. In still further examples, the spray nozzle is selected from an ultrasonic nozzle, rotary atomizer, or any other suitable spray nozzle known in the art.

In some examples, the spray-coating process further comprises: (c) solidifying the coating layer formed on the inner frame surface 144, and optionally on the outer frame surface 146, thereby forming the coating 182 thereon. The solidification can include evaporating unwanted solvents, drying the coating layer via heat, etc.

In some examples, the spray-coating process is configured to form a continuous, uniform, controllable thickness of the coating layer formed on the inner frame surface 144, and optionally the outer frame surface 146, thereby forming a continuous, uniform, thin-layered coating 182 thereon.

In some examples, the coating 182 covers the inner frame surface 144 (as illustrated in Fig. 21C), or a portion of the inner frame surface 144 (as illustrated in Fig. 21D). In further such examples, step (b) comprises positioning at least one spray nozzle internally within the frame 106, in parallel to or along the centerline 20, during the coating process. It is to be understood that any reference to a nozzle placed internally within frame 106 throughout the specification and the claims, refers to positioning of the nozzle within the space defined by the frame (i.e., the space between the centerline 120 and the inner frame surface 144).

In some other examples, the coating 182 is disposed over the entire frame 106, covering both of the inner frame surface 144 and the outer frame surface 146. In further such examples, step (b) comprises positioning at least one spray nozzle externally to the outer frame surface 146 of frame 106, during the coating process, in order to coat the outer frame surface 146 and optionally inner frame surface 144 of frame 106 (for example, due to spray-coat material passing through cell openings toward inner frame surface 144 on the opposite side of the frame). Additionally, the spray nozzle can be positioned internally within the frame 106, in parallel to or along the centerline 20, during the coating process.

In some examples, step (b) comprises rotating/spinning the frame 106 during the coating process, to create a uniform coating thereon. In further examples, step (b) comprises rotating/spinning the spray nozzle during the coating process, to create uniform coating around or along frame 106.

By positioning the spray nozzle internally within the frame 106, portions of frame 106 (e.g., the outer frame surface 146) which are not required to be coated by coating 182, can remain uncoated, thus allowing the spray-coating process described herein to be utilized as a targeted coating process for covering solely desired portions of the frame 106, such as portions that may be contacted by the leaflets 124 in their fully-open state.

In some examples, prior to spray-coating the inner frame surface 144 (and optionally the outer frame surface 146) during step (b), the process comprises masking portions of the frame 106 (e.g., junctions 114 or portions thereof) by masking layers (e.g., a removable adhesive layer), thereby forming a plurality of masked junctions thereon. In further such examples, following step (c) of solidifying the coating layer formed on frame 106, the masking layers are removed, thereby exposing the junction portions. By utilizing masking layers, portions of frame 106 which are not required to become coated by coating 182 (e.g., junctions 114, 114^{d}) can remain uncoated, thus allowing the spray-coating process as described herein to be utilized as a targeted coating process to cover solely desired portions of the frame 106, such as strut segments 112 that may be contacted by the leaflets 124 in their fully-open state. Targeted coating of strut segments 112, without coating the junctions 114, may facilitate improved/undisturbed movement of the immediate portions extending from the junctions between the compressed and expanded configurations of the frame 106.

In some examples, spray-coating the surface of the frame 106 during step (b), utilizing a coating nozzle, comprises spray-coating only a portion of the inner frame surface 144 (and optionally a corresponding portion of the outer frame surface 146). Spray-coating only a portion of the surface of the frame can be achieved by targeting the coating nozzle to coat solely the desired portions of the frame 106, from within the frame and/or externally thereto. The desired portions of the frame to be coated can be the portion of the frame 106 configured to be contacted at least partially by leaflets 124, such as a portion of the frame 106 proximal to the attachment of the leaflet assembly 122 thereto along the scallop line 134. Additionally or alternatively, spray-coating only a portion of the surface of the frame can be achieved by masking a portion of the frame 106, using a removable adhesive layer which can be removed afterwards, thus obtaining a coating solely along the desired portions of the frame to be coated as disclosed herein. Therefore, the spray-coating process described herein can be utilized as a targeted coating process to cover solely desired portions of the frame 106, such as the portion of the inner frame surface 144 that may be contacted at least partially by the leaflets 124 in their fully-open state.

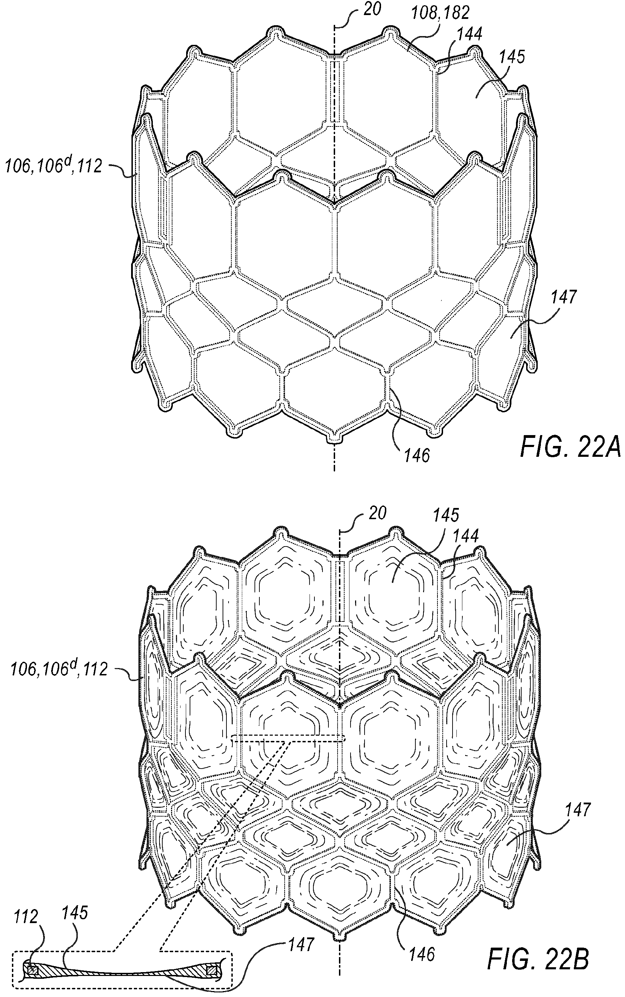

Reference is now made to Figs. 22A-B, which are views in perspective of a frame 106 encapsulated between inner and outer coating layers 145 and 147, according to some examples.

In some examples, coating 182 is applied along the frame 106 or a portion thereof by a lamination process comprising: (a) providing a frame 106 of a prosthetic valve 100 as described above at an expanded configuration, prior to attachment of leaflet assembly 122 thereto.

As used herein, the term "lamination" refers to a process of manufacturing a composite material comprising multiple layers, created using heat, pressure, welding, adhesives, and the like. The final composite material typically achieves improved properties such as strength, stability, etc.

In some examples, the lamination process further comprises: (b) providing an inner coating layer 145 and/or an outer coating layer 147. In some examples, each one of the inner and outer coating layers 145 and 147 are characterized by having the same characteristics and/or comprising the same materials as coating 182 or pad 170, as disclosed hereinabove.

In some examples, each one of the inner and outer coating layers 145 and 147 can be made of various suitable biocompatible synthetic materials, such as, but not limited to, a thermoplastic material. Suitable thermoplastics biocompatible materials are selected from, but not limited, polyamides, polyesters, polyethers, polyurethanes, polyolefins (such as polyethylene and/or polypropylenes), polytetrafluoroethylenes, and combinations and copolymers thereof. Each possibility represents a different example. In further examples, the thermoplastic material comprises a thermoplastic elastomer (TPE). In still further examples, the thermoplastic elastomer is selected from the group consisting of: thermoplastic polyurethane (TPU), styrene block copolymers (TPS), Thermoplastic polyolefinelastomers (TPO), thermoplastic vulcanizates (TPV), thermoplastic copolyester (TPC), thermoplastic polyamides (TPA), and combinations and variations thereof. Each possibility represents a different example.

According to some examples, each one of the inner and outer coating layers 145 and 147 comprises a thermoplastic elastomer comprising TPU. In some examples, each one of the inner and outer coating layers 145 and 147 comprises TPU and at least one biocompatibility-enhancing polymer. In some examples, each one of the inner and outer coating layers 145 and 147 comprises TPU which is laminated with a UHMWPE layer for surface modification.

In some examples, each one of the inner and outer coating layers 145 and 147 comprises a polyester layer which can be coated with at least one biocompatibility-enhancing polymer.

In some examples, each one of the inner and outer coating layers 145 and 147 comprises polycarbonate urethane (PCU). In some examples, each one of the inner and outer coating layers 145 and 147 do not comprise poly(ether urethane) and/or poly(ether urethane ureas).

In some examples, each one of the inner and outer coating layers 145 and 147 comprises at least one material selected from a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof. Each possibility represents a different example.

In some examples, each one of the inner and outer coating layers 145 and 147 are characterized by having the same desired surface or material features as coating 182 or pad 170, as disclosed hereinabove. In further such examples, each one of the inner and outer coating layers 145 and 147 are characterized by having at least one property selected from: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a flexural strength in the range of about 5,000-10,000 psi; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

**In** some examples, each one of the inner and outer coating layers 145 and 147 are characterized by a Ra value that is 80% or less of the Ra value of the surface of the frame 106. In further examples, said Ra value is 50% or less of the Ra value of the frame 106.

**In** some examples, each one of the inner and outer coating layers 145 and 147 is biodurable, adapted to preserve at least 80%, at least 90%, or more, of one or more properties during exposure to biological environment over a minimal period of time. The preserved properties can include at least one of: a durometer hardness ranging from about 40 Shore A to about 98 Shore A; a durometer hardness ranging from about 0 Shore D to about 55 Shore D; a Ra value of about 0.2 µm or less; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a flexural strength in the range of about 5,000-10,000 psi; a tensile strength in the range of about 6,000-9,000 psi; a tensile strain at the failure point of about 200-500 %; a secant tensile modulus in the range of about 700-2,400 psi, for a tensile strain of about 100 % or 300 %; a number average molecular weight (Mn) selected from the range of 70,000-90,000; a weight average molecular weight (Mw) selected from the range of 200,000-300,000, and combinations thereof.

**In** some examples, each one of the inner and outer coating layers 145 and 147 are in the form of thin polymeric sheets.

**In** some examples, the lamination process further comprises: (c) adhering or laminating the inner and outer coating layers 145 and 147 to the frame 106 or a portion thereof, so that frame 106 becomes encapsulated between the inner and outer coating layer 145 and 147. In further such examples, the inner coating layer 145 is configured to become be adhered or laminated to the inner frame surface 144 of the frame 106, thereby forming the coating 182 therealong. In still further such examples, the outer coating layer 147 is configured to become be adhered or laminated to the outer frame surface 146 of the frame 106, thereby forming the coating 182 therealong. For example, by adhering or laminating the inner and outer coating layers 145 and 147 to the frame 106 or a portion thereof, the coating 182 can cover the inner and outer frame surfaces 144 and 146 or corresponding portions thereof, as illustrated in Fig. 22A.

The adhering or laminating of the inner and outer coating layers 145 and 147 to the frame 106 can be performed by the application of at least one of heat, pressure, ultrasonic welding, adhesives, a combination thereof, or other suitable known methods.

In some other examples, the inner and outer coating layers 145 and 147 can become bonded to each other during the lamination process of step (c), such that the frame 106 is encapsulated between the layers 145 and 147. More specifically, in certain examples, the inner and outer coating layers 145 and 147 can be adhered to each other, through openings defined between the strut segments 112 or cells 108 of the frame 106, as illustrated in Fig. 22B. In further such examples, the inner and outer coating layers 145 and 147 are not adhered to the frame 106, but solely to each other, thereby forming the coating 182 over the entire length of the frame 106 or a portion thereof. This can allow the frame 106 to be movable between the radially compressed and the radially expanded configurations, without becoming restricted by the laminating coating layers 145 and 147 attached to the frame 106.

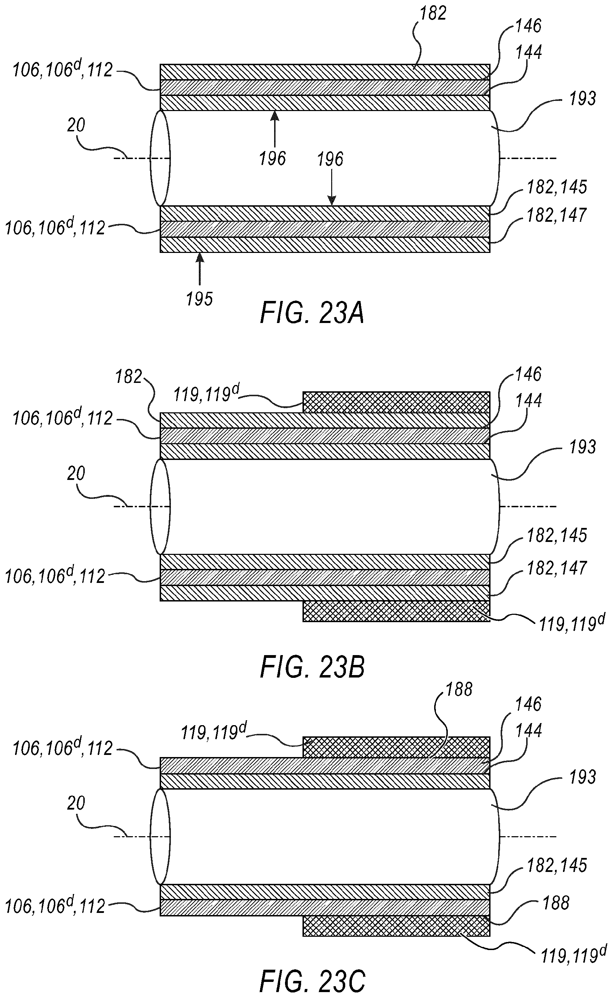

Reference is now made to Figs. 23A-C, which are cross-sectional views of a coated frame 106 disposed around a mandrel 193, according to some examples.

In some examples, step (c) of the lamination process as presented above can comprises: (i) wrapping or situating the inner coating layer 145 around a mandrel 193, as illustrated in Fig. 23A. The inner coating layer 145 may be in the form of a pre-fabricated thin polymeric sheet that is applied by being wrapped around the mandrel 193, or may be applied to the mandrel by dip coating, spray-coating, electrospinning, etc. In further such examples, the inner coating layer 145 is not irreversibly attached to the mandrel 193, so that the layer 145 can become detached therefrom and attached to a frame 106, during future additional steps.

The term "mandrel", as used herein, refers to an elongated member, such as a rod or a pipe, that may serve as a core over which the coating layers and the frame 106 are disposed and are bonded over. The mandrel may be shaped as an elongated cylinder.

In some examples, the inner coating layer 145 is applied around the mandrel 193 by electrospinning. As used herein, the term "electrospinning" refers to a process of manufacturing polymeric nano-fibers by drawing charged threads of polymeric solutions or melts using electric force, and wrapping them around an elongated collector (e.g., the mandrel and/or the frame), thereby forming a thin polymeric sheet thereon (e.g., layers 145 and/or 147).

In some examples, step (c) of the lamination process further comprises: (ii) situating the frame 106 around the inner coating layer 145, thereby contacting the inner frame surface 144 of the frame 106 therewith. The frame 106 can be situated around the inner coating layer 145 by radially expanding the frame 106, adjusting it in the desired location, and optionally radially compressing the frame 106 to contact the inner coating layer 145.

In some examples, step (c) of the lamination process further comprises: (iii) wrapping or situating an outer coating layer 147 around the frame 106, thereby contacting the outer frame surface 146 of the frame 106 therewith, as illustrated in Fig. 23A. The outer coating layer 147 may be in the form of a pre-fabricated thin polymeric sheet that is applied by being wrapped around the frame 106, or may be applied to the frame by dip coating, spray-coating, electrospinning, etc.

In some examples, step (c) of the lamination process further comprises: (iv) forcing the inner and outer coating layers 145 and 147 to become bonded to the frame 106 and/or to each other, thereby forming the coating 182 along or over the inner and outer frame surfaces 144 and 146 of the frame 106, by the application of at least one of heat, pressure, ultrasonic welding, adhesives, or a combination thereof. In further such examples, the inner and outer coating layers 145 and 147 become bonded to the frame 106 or to each other using a combination of heat and pressure.

The pressure may be applied externally, by pressing radially inward the outer coating layer 147 toward centerline 20, along pressure direction line 195, as illustrated in Fig. 23A, utilizing for example, clamp(s), crimper(s) or pincer(s). Additionally or alternatively, the pressure may be applied internally, by pressing radially outward the inner coating layer 145 in the opposite direction to centerline 20, along pressure direction line 196, as illustrated in Fig. 23A, utilizing for example, an inflatable or otherwise stretchable/expandable mandrel.

The heat may be applied by exposing the coated mandrel to a heat source, such as placed in an oven, at a temperature above the softening temperature of the coating layers 145 and 147. The coating layers 145 and 147 can then be allowed to cool.

In some examples, the inner and outer coating layers 145 and 147 can become bonded to each other during the bonding process of step (iv), such that the frame 106 is encapsulated between the layers 145 and 147, and the inner and outer coating layers 145 and 147 are adhered to each other through openings defined between the strut segments 112 or cells 108 of the frame 106.

In some examples, step (c) of the lamination process further comprises: (v) removing the coated frame 106 from the mandrel 193, wherein the coated frame 106 comprises both the inner and outer coating layers 145 and 147.

In some alternative examples, phase (iii) of step (c) further comprises wrapping or situating/mounting the outer skirt 119 (e.g., outer skirt 119^{d}) on the outer surface of the outer coating layer 147, as illustrated in Fig. 23B. In such examples, phase (iv) of step (c) comprises forcing the outer skirt 119 to become bonded to the outer coating layer 147, and the inner and outer coating layers 145 and 147 to become bonded to the frame 106 or to each other, by the application of at least one of heat, pressure, ultrasonic welding, adhesives, or a combination thereof. In further such examples, the outer skirt 119 can become bonded to the outer coating layer 147 using a combination of heat and pressure, as disclosed herein above, and optionally adhesives. The heat may cause the outer coating layer 147 to become soft or at least partially melted, so that the outer skirt 119 can become attached thereto. Additionally, in some examples, phase (iii) of step (c) can further comprise temporarily holding in place the outer skirt 119 against outer coating layer 147, utilizing at least one of clamps, removable adhesives, removable sutures, and the like. Advantageously, by bonding the outer skirt 119 directly to the outer coating layer 147 as disclosed herein, the outer skirt 119 can become connected to the outer surface of the frame 106 without the utilization of sutures.

In some other examples, phase (iii) of step (c) alternatively comprises wrapping or situating/mounting the outer skirt 119 (e.g., outer skirt 119^{d}) on the outer frame surface 146 of the frame 106, thereby contacting the outer frame surface 146 of the frame 106 with an inner surface 188 of the outer skirt 119, as illustrated in Fig. 23C. In such examples, phase (iii) of step (c) is devoid of utilizing the outer coating layer 147. In further examples, phase (iv) of step (c) comprises forcing the outer skirt 119 to become bonded directly to the inner coating layer 145, through openings defined between the strut segments 112 or cells 108 of the frame 106, and optionally to become bonded to the frame 106 itself, by the application of at least one of heat, pressure, ultrasonic welding, adhesives, or a combination thereof.

In some examples, the outer skirt 119 can become bonded directly to the inner coating layer 145, through frame 106, using a combination of heat and pressure, and optionally adhesives. The heat may cause the inner coating layer 145 to become soft or at least partially melted, so that the outer skirt 119 can become attached thereto, through frame 106. Additionally, in some examples, phase (iii) of step (c) can further comprise temporarily holding in place the outer skirt 119 against frame 106, utilizing at least one of clamps, removable adhesives, removable sutures, and the like. Advantageously, by bonding the outer skirt 119 directly to the inner coating layer 145 as disclosed herein, the outer skirt 119 can become connected to the outer surface of the frame 106 without the utilization of sutures.

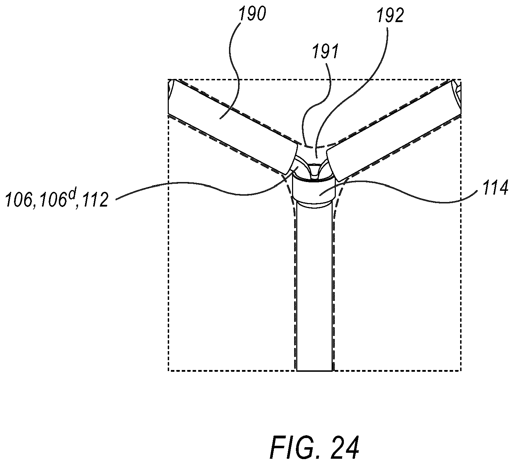

Reference is now made to Fig. 24, which is an enlarged view of a portion of the frame 106 covered by polymeric coating layers 190 and 191, according to some examples.

In some examples, the coating 182 is applied to the frame 106 prior to the attachment of the leaflet assembly 122 thereto, utilizing the various processes as disclosed hereinabove, thus enabling to form the polymeric covering layer attached to the frame 106 or a portion thereof having the desired surface features as disclosed herein. In some examples, the coating 182, having desired surface features, forms a polymeric covering layer attached to the frame 106 or a portion thereof, wherein the polymeric covering layer is formed directly on the frame 106 or a portion thereof in a single step.

In alternative examples, the polymeric covering layer is formed by a double-step process comprising: (a) initially coating strut segments 112 of the frame 106, or portions of strut segments 112, using a first polymeric layer 190, configured to become disposed therearound. In some examples, the double-step process further comprises: (b) coating the first polymeric layer 190 using a second polymeric layer 191, configured to become attached to the first polymeric layer 190 without bonding with the junctions 114, as illustrated at Fig. 24, wherein the second polymeric layer 191 is characterized by having the desired surface features of coating 182, as disclosed herein above. In some examples, the second polymeric layer 191 is configured to become attached to the first polymeric layer 190 and disposed over the frame 106 or a portion thereof, so as to form inner void portions 192 at junctions 114 of the frame 106. In further examples, the second polymeric layer 191 does not contact directly the frame 106 or a portion thereof (e.g., junctions 114), since it is disposed over the first polymeric layer 190.

The formation of inner void portions 192 by the second polymeric layer 191 over junctions 114 can allow movement of the immediate portions extending from the junction between the compressed and expanded configurations of the frame 106. The coating of the frame utilizing the first and second polymeric layers 190 and 191 can be performed utilizing the various coating processes as disclose herein above.

The first and/or second polymeric layers 190 and 191 can include the same materials as any one of the inner and outer coating layers 145 and 147, coating 182 or pad 170, as disclosed herein above.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1. A prosthetic valve, comprising:
a frame movable between a radially compressed and a radially expanded configuration;
a plurality of commissural support members attached to the frame, wherein each commissural support member is covered by a heat-shrink wrap, tightly shrunk thereover; and
a leaflet assembly mounted within the frame and comprising a plurality of leaflets configured to regulate flow through the prosthetic valve, wherein each leaflet comprises a pair of oppositely-directed tabs, and wherein the tabs of adjacent leaflets are paired to form commissure assemblies;
wherein each commissure assembly is attached to a corresponding commissural support member over the heat-shrink wrap.

Example 2. The prosthetic valve of any example herein, particularly example 1, further comprising:
a plurality of expansion and locking assemblies, each comprising:
an outer member, coupled to the frame at a first location; and
an inner member, coupled to the frame at a second location spaced apart from the first location;

wherein movement of the inner member in a first direction, relative to the outer member, causes the frame to foreshorten axially and expand radially; and
wherein a portion of each outer member, extending from the first location in the first direction, is the commissural support member.

Example 3. The prosthetic valve of any example herein, particularly example 2, wherein the first direction is a proximally oriented direction.

Example 4. The prosthetic valve of any example herein, particularly any one of examples 2 or 3, wherein the frame comprises a plurality of inflow apices, a plurality of outflow apices, and a plurality of non-apical junctions, and wherein the first location is a proximal-most non-apical junction.

Example 5. The prosthetic valve of any example herein, particularly any one of examples 1 to 4, wherein the commissural support member has an exposed proximal end.

Example 6. The prosthetic valve of any example herein, particularly any one of examples 1 to 5, wherein the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

Example 7. The prosthetic valve of any example herein, particularly any one of examples 1 to 6, wherein the heat-shrink wrap is a heat-shrink sleeve.

Example 8. The prosthetic valve of any example herein, particularly example 7, wherein the length of the heat-shrink sleeve is in the range of 90% to 110% of the length of the commissural support member.

Example 9. The prosthetic valve of any example herein, particularly any one of examples 1 to 8, wherein the commissural support member has a rectangular cross section, defining a support member radial depth and a support member lateral width.

Example 10. The prosthetic valve of any example herein, particularly example 9, wherein the heat-shrink wrap has an elliptical cross section in a pre-shrunk state thereof, defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

Example 11. The prosthetic valve of any example herein, particularly example 10, wherein the heat-shrink wrap has a circular cross section in a pre-shrunk state thereof that can be squeezed to an elliptical shape defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

Example 12. The prosthetic valve of any example herein, particularly any one of examples 10 or 11, wherein the sleeve first diameter is at least as great as 120% of the support member radial depth.

Example 13. The prosthetic valve of any example herein, particularly any one of examples 10 or 11, wherein the sleeve first diameter is at least as great as 150% of the support member radial depth.

Example 14. The prosthetic valve of any example herein, particularly any one of examples 10 or 11, wherein the sleeve first diameter is at least as great as 200% of the support member radial depth.

Example 15. The prosthetic valve of any example herein, particularly any one of examples 10 or 11, wherein the sleeve second diameter is at least as great as 120% of the support member lateral width.

Example 16. The prosthetic valve of any example herein, particularly any one of examples 10 or 11, wherein the sleeve second diameter is at least as great as 150% of the support member lateral width.

Example 17. The prosthetic valve of any example herein, particularly any one of examples 10 or 11, wherein the sleeve second diameter is at least as great as 200% of the support member lateral width.

Example 18. The prosthetic valve of any example herein, particularly any one of examples 1 to 8, wherein the commissural support member defines a maximal support member diameter, and wherein the heat-shrink sleeve, in a pre-shrunk state thereof, defines a minimal sleeve diameter which is greater than the maximal support member diameter.

Example 19. The prosthetic valve of any example herein, particularly example 18, wherein the minimal sleeve diameter is at least as great as 120% of the maximal support member diameter.

Example 20. The prosthetic valve of any example herein, particularly example 18, wherein the minimal sleeve diameter is at least as great as 150% of the maximal support member diameter.

Example 21. The prosthetic valve of any example herein, particularly example 18, wherein the minimal sleeve diameter is at least as great as 200% of the maximal support member diameter.

Example 22. A prosthetic valve, comprising:
a frame movable between a radially compressed and a radially expanded configuration, the frame comprising a plurality of interconnected strut segments, each strut segment having a strut width, a strut thickness, and a strut segment length;
an inner skirt disposed along an inner surface of the frame, the inner skirt comprising a skirt proximal end attached to strut segments extending along the circumference of the frame;
a leaflet assembly mounted within the frame and comprising a plurality of leaflets configured to regulate flow through the prosthetic valve, wherein the leaflet assembly is sutured to the inner skirt along a scallop line that tracks the lower edge of the leaflet assembly; and
a plurality of heat-shrink wraps tightly shrunk over the strut segments to which the skirt proximal end is attached.

Example 23. The prosthetic valve of any example herein, particularly example 22, wherein the skirt proximal end is sutured to the strut segment with at least one skirt attachment suture.

Example 24. The prosthetic valve of any example herein, particularly any one of examples 22 or 23, wherein the frame comprises a plurality of struts arranged in a lattice pattern and pivotably coupled to each other, wherein each strut comprises a plurality of the strut segments.

Example 25. The prosthetic valve of any example herein, particularly any one of examples 22 or 23, wherein the strut segments are angled strut segments.

Example 26. The prosthetic valve of any example herein, particularly any one of examples 22 to 25, wherein the skirt proximal end is attached to the strut segments following a zig-zag pattern.

Example 27. The prosthetic valve of any example herein, particularly any one of examples 22 to 26, wherein each strut segment has a rectangular cross-section, wherein the strut width is a linear strut width, and wherein the strut thickness is a linear strut thickness.

Example 28. The prosthetic valve of any example herein, particularly any one of examples 22 to 26, wherein each strut segment has an elliptic cross-section, wherein the strut width is a first diameter of the cross-section, and wherein the strut thickness is a second diameter of the cross-section.

Example 29. The prosthetic valve of any example herein, particularly any one of examples 22 to 26, wherein each strut segment has circular cross-section, and wherein the strut width and the strut thickness are equal to each other and to a circular diameter of the cross-section.

Example 30. The prosthetic valve of any example herein, particularly any one of examples 22 to 29, wherein the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

Example 31. The prosthetic valve of any example herein, particularly any one of examples 22 to 30, wherein the heat-shrink wrap is a heat-shrink film, having a pre-shrunk rectangular shape with a film length and a film width.

Example 32. The prosthetic valve of any example herein, particularly example 31, wherein the film length is not greater than the strut segment length.

Example 33. The prosthetic valve of any example herein, particularly any one of examples 31 or 32, wherein the film width in a pre-shrunk state thereof, is greater than the strut width plus twice the strut thickness.

Example 34. The prosthetic valve of any example herein, particularly any one of examples 31 or 32, wherein the film width in a pre-shrunk state thereof, is greater than twice the strut width plus twice the strut thickness.

Example 35. The prosthetic valve of any example herein, particularly example 23, wherein the skirt attachment suture is looped through the skirt proximal end and the heat-shrink wrap, in a whip stitch pattern around the strut segment.

Example 36. The prosthetic valve of any example herein, particularly example 35, wherein the stitch pattern comprises two sections of the skirt attachment suture extending substantially parallel to each other within each loop formed by the stitching pattern, between the strut segment and opposite sections of the heat-shrink wrap.

Example 37. The prosthetic valve of any example herein, particularly example 35, wherein the stitch pattern comprises two sections of the skirt attachment suture extending substantially parallel to each other within each loop formed by the stitching pattern, wherein one section extends between the strut segment and an inner side of a section of the heat-shrink wrap, and another section extends parallel thereto over an external side of a section of the heat-shrink wrap.

Example 38. The prosthetic valve of any example herein, particularly example 23, wherein the skirt attachment suture is sutured over the external surface of the heat-shrink wrap, without extending through the heat-shrink wrap.

Example 39. The prosthetic valve of any example herein, particularly any one of examples 22 to 30, wherein the heat-shrink wrap is a C-shaped heat-shrink tube, having a tube length, a tube first diameter and a tube second diameter, wherein the tube second diameter is oriented in the radial direction, wherein the tube first diameter is substantially orthogonal to the radial direction, wherein the tube first diameter is greater than the strut width, and wherein the tube second diameter is greater than the strut thickness.

Example 40. The prosthetic valve of any example herein, particularly example 39, wherein the C-shaped heat-shrink tube has a circular cross-section such that the tube first diameter is equal to the tube second diameter.

Example 41. The prosthetic valve of any example herein, particularly any one of examples 39 or 40, wherein the tube length is not greater than the strut segment length.

Example 42. The prosthetic valve of any example herein, particularly any one of examples 39 to 41, wherein the C-shaped heat-shrink tube comprises, in a pre-shrunk state thereof, a gap having a gap width that is smaller than the strut width and/or the strut thickness, in a free state of the C-shaped heat-shrink tube.

Example 43. The prosthetic valve of any example herein, particularly any one of examples 39 to 42, wherein the tube first diameter is at least as great as 120% of the strut width.

Example 44. The prosthetic valve of any example herein, particularly any one of examples 39 to 42, wherein the tube first diameter is at least as great as 150% of the strut width.

Example 45. The prosthetic valve of any example herein, particularly any one of examples 39 to 42, wherein the tube first diameter is at least as great as 200% of the strut width.

Example 46. The prosthetic valve of any example herein, particularly any one of examples 39 to 42, wherein the tube second diameter is at least as great as 120% of the strut thickness.

Example 47. The prosthetic valve of any example herein, particularly any one of examples 39 to 42, wherein the tube second diameter is at least as great as 150% of the strut thickness.

Example 48. The prosthetic valve of any example herein, particularly any one of examples 39 to 42, wherein the tube second diameter is at least as great as 200% of the strut thickness.

Example 49. The prosthetic valve of any example herein, particularly any one of examples 39 to 48, wherein at least one skirt attachment suture is looped through the skirt proximal end and the C-shaped heat-shrink tube, in a whip stitch pattern around the strut segment.

Example 50. The prosthetic valve of any example herein, particularly example 49, wherein the stitch pattern comprises two sections of the skirt attachment suture extending substantially parallel to each other within each loop formed by the stitching pattern, between the strut segment and opposite sections of the heat-shrink tube.

Example 51. The prosthetic valve of any example herein, particularly example 49, wherein the stitch pattern comprises two sections of the skirt attachment suture extending substantially parallel to each other within each loop formed by the stitching pattern, wherein one section extends between the strut segment and an inner side of a section of the heat-shrink tube, and another section extends parallel thereto over an external side of a section of the heat-shrink tube.

Example 52. The prosthetic valve of any example herein, particularly any one of examples 39 to 48, wherein at least one skirt attachment suture is sutured through the inner skirt and over the external surface of the heat-shrink tube, without extending through the heat-shrink tube.

Example 53. A prosthetic valve, comprising:
a frame movable between a radially compressed and a radially expanded configuration, the frame comprising a plurality of commissural support members, each commissural support member having a support member radial depth and a support member lateral width, wherein at least a portion of each commissural support member is covered by a heat-shrink wrap, tightly shrunk thereover; and
a leaflet assembly mounted within the frame and comprising a plurality of leaflets configured to regulate flow through the prosthetic valve, wherein each leaflet comprises a pair of oppositely-directed tabs, wherein the tabs of adjacent leaflets are paired to form commissure assemblies;
wherein each commissure assembly is attached to a corresponding commissural support member over the heat-shrink wrap.

Example 54. The prosthetic valve of any example herein, particularly example 53, wherein the frame comprises a plurality of axially extending strut segments, wherein at least some of the axially extending strut segments are the commissural support members.

Example 55. The prosthetic valve of any example herein, particularly example 53, wherein each commissural support member is a commissure window comprising two axially extending window strut segments circumferentially spaced from each other, having a heat-shrink wrap tightly shrunk around each of the axially extending window strut segments, wherein each axially extending window strut segment has a vertical segment radial depth an a vertical segment lateral width.

Example 56. The prosthetic valve of any example herein, particularly any one of examples 53 to 55, wherein the commissural support members are integrally formed with the frame.

Example 57. The prosthetic valve of any example herein, particularly any one of examples 53 to 56, wherein the plurality of commissural support members includes three commissural support members.

Example 58. The prosthetic valve of any example herein, particularly any one of examples 53 to 57, wherein the heat-shrink wrap comprises at least one thermoplastic polymeric material selected from: polyolefins, fluoropolymers, polyvinyl chloride (PVC), polychloroprenes (neoprenes), silicone elastomers, and combinations thereof.

Example 59. The prosthetic valve of any example herein, particularly any one of examples 53 to 54 or 56 to 58, wherein each commissural support member has a rectangular cross section, wherein the support member radial depth is a linear radial depth, and wherein the support member lateral width is a linear lateral width.

Example 60. The prosthetic valve of any example herein, particularly any one of examples 53 to 54 or 56 to 58, wherein each commissural support member has an elliptic cross section, wherein the support member radial depth is a support member first diameter, and wherein the support member lateral width is a support member second diameter.

Example 61. The prosthetic valve of any example herein, particularly any one of examples 53 to 54 or 56 to 58, wherein each commissural support member has a circular cross section, and wherein the support member radial depth and the support member lateral width are equal to each other and to a circular diameter of the commissural support member.

Example 62. The prosthetic valve of any example herein, particularly example 55, wherein each axially extending window strut segment has a rectangular cross section, wherein the vertical segment radial depth is a linear radial depth, and wherein the vertical segment lateral width is a linear lateral width.

Example 63. The prosthetic valve of any example herein, particularly any one of examples 53 to 61, wherein each commissural support member has an exposed proximal end.

Example 64. The prosthetic valve of any example herein, particularly example 63, wherein the heat-shrink wrap is a heat-shrink sleeve.

Example 65. The prosthetic valve of any example herein, particularly example 64, wherein the length of the heat-shrink sleeve is in the range of 90% to 110% of the length of the commissural support member.

Example 66. The prosthetic valve of any example herein, particularly any one of examples 64 or 65, wherein the heat-shrink sleeve has a circular cross section in a pre-shrunk state thereof, defining a sleeve circular diameter that is greater than the support member radial depth and/or than the support member lateral width.

Example 67. The prosthetic valve of any example herein, particularly example 66, wherein the sleeve circular diameter is at least as great as 120% of the support member radial depth and/or of the support member lateral width.

Example 68. The prosthetic valve of any example herein, particularly example 66, wherein the sleeve circular diameter is at least as great as 150% of the support member radial depth and/or of the support member lateral width.

Example 69. The prosthetic valve of any example herein, particularly example 66, wherein the sleeve circular diameter is at least as great as 200% of the support member radial depth and/or of the support member lateral width.

Example 70. The prosthetic valve of any example herein, particularly any one of examples 53 to 61, wherein each commissural support member is bound between angled strut segments of the frame.

Example 71. The prosthetic valve of any example herein, particularly example 63, wherein the heat-shrink wrap is a heat-shrink film, having a pre-shrunk rectangular shape with a film length and a film width.

Example 72. The prosthetic valve of any example herein, particularly example 71, wherein the film length is not greater than the support member length.

Example 73. The prosthetic valve of any example herein, particularly any one of examples 71 or 72, wherein the film width in a pre-shrunk state thereof, is greater than the support member lateral width plus twice the support member radial depth.

Example 74. The prosthetic valve of any example herein, particularly any one of examples 71 or 72, wherein the film width in a pre-shrunk state thereof, is greater than twice the support member lateral width plus twice the support member radial depth.

Example 75. The prosthetic valve of any example herein, particularly example 63, wherein the heat-shrink wrap is a C-shaped heat-shrink tube, having a tube length, a tube first diameter and a tube second diameter, wherein the tube second diameter is oriented in the radial direction, wherein the tube first diameter is substantially orthogonal to the radial direction, wherein the tube first diameter is greater than the strut width, and wherein the tube second diameter is greater than the strut thickness.

Example 76. The prosthetic valve of any example herein, particularly example 75, wherein the tube length is not greater than the support member length.

Example 77. The prosthetic valve of any example herein, particularly any one of examples 75 or 76, wherein the C-shaped heat-shrink tube comprises, in a pre-shrunk state thereof, a gap having a gap width that is smaller than the support member radial depth and/or the support member lateral width, in a free state of the C-shaped heat-shrink tube.

Example 78. The prosthetic valve of any example herein, particularly any one of examples 75 to 77, wherein the tube first diameter is at least as great as 120% of the support member lateral width.

Example 79. The prosthetic valve of any example herein, particularly any one of examples 75 to 77, wherein the tube first diameter is at least as great as 150% of the support member lateral width.

Example 80. The prosthetic valve of any example herein, particularly any one of examples 75 to 77, wherein the tube first diameter is at least as great as 200% of the support member lateral width.

Example 81. The prosthetic valve of any example herein, particularly any one of examples 75 to 77, wherein the tube second diameter is at least as great as 120% of the support member radial depth.

Example 82. The prosthetic valve of any example herein, particularly any one of examples 75 to 77, wherein the tube second diameter is at least as great as 150% of the support member radial depth.

Example 83. The prosthetic valve of any example herein, particularly any one of examples 75 to 77, wherein the tube second diameter is at least as great as 200% of the support member radial depth.

Example 84. A method of assembling a prosthetic valve, the method comprising:
providing a prosthetic valve comprising frame movable between a radially compressed and a radially expanded configuration, and a plurality of commissural support members attached to the frame;
disposing a heat-shrink sleeve over each commissural support member;
applying heat to the heat-shrink sleeve, thereby causing it to shrink radially inward and to become tightly attached around the corresponding commissural support member; and
attaching a commissure assembly of a leaflet assembly to each commissural support member covered by the heat-shrink sleeve.

Example 85. The method of any example herein, particularly example 84, wherein the commissural support member has an exposed proximal end, and wherein disposing the heat-shrink sleeve over each commissural support member comprises sliding the heat-shrink sleeve from the exposed upper end over the corresponding commissural support member.

Example 86. The method of any example herein, particularly any one of examples 84 or 85, wherein the leaflet assembly comprises a plurality of leaflets configured to regulate flow through the prosthetic valve, wherein each leaflet comprises a pair of oppositely-directed tabs, and wherein the tabs of adjacent leaflets are paired to form the commissure assemblies.

Example 87. The method of any example herein, particularly any one of examples 84 to 86, wherein the prosthetic valve comprises a plurality of expansion and locking assemblies, each comprising an outer member coupled to the frame at a first location and an inner member coupled to the frame at a second location spaced apart from the first location, wherein movement of the inner member in a first direction, relative to the outer member, causes the frame to foreshorten axially and expand radially, and wherein a portion of each outer member, extending from the first location in the first direction, is the commissural support member.

Example 88. The method of any example herein, particularly any one of examples 84 to 87, wherein the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

Example 89. The method of any example herein, particularly any one of examples 84 to 88, wherein applying heat to the heat-shrink sleeve comprises applying heat at a temperature of above 90°C.

Example 90. The method of any example herein, particularly example 89, wherein applying heat to the heat-shrink sleeve comprises applying heat at a temperature of above 135°C.

Example 91. The method of any example herein, particularly any one of examples 84 to 90, wherein the length of the heat-shrink sleeve is in the range of 90% to 110% of the length of the commissural support member.

Example 92. The method of any example herein, particularly any one of examples 84 to 90, wherein the length of the heat-shrink sleeve is greater than 110% of the length of the commissural support member, and wherein the method further comprises a step of cutting the heat-shrink sleeve to a length that is not higher than 110% of the length of the commissural support member prior to the step of applying heat.

Example 93. The method of any example herein, particularly any one of examples 84 to 92, wherein the commissural support member has a rectangular cross section, defining a support member radial depth and a support member lateral width, wherein the heat-shrink sleeve has an elliptical cross section in a pre-shrunk state thereof, defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

Example 94. The method of any example herein, particularly any one of examples 84 to 90, wherein the commissural support member has a rectangular cross section, defining a support member radial depth and a support member lateral width, wherein the heat-shrink sleeve has a circular cross section in a pre-shrunk state thereof that can be squeezed to an elliptical shape defining a sleeve first diameter oriented in the radial direction, and a sleeve second diameter oriented in the lateral direction, wherein the sleeve first diameter is greater than the support member radial depth, and wherein the sleeve second diameter is greater than the support member lateral width.

Example 95. The method of any example herein, particularly any one of examples 93 or 94, wherein the sleeve first diameter is at least as great as 120% of the support member radial depth.

Example 96. The method of any example herein, particularly any one of examples 93 or 94, wherein the sleeve first diameter is at least as great as 150% of the support member radial depth.

Example 97. The method of any example herein, particularly any one of examples 93 or 94, wherein the sleeve first diameter is at least as great as 200% of the support member radial depth.

Example 98. The method of any example herein, particularly any one of examples 93 or 94, wherein the sleeve second diameter is at least as great as 120% of the support member lateral width.

Example 99. The method of any example herein, particularly any one of examples 93 or 94, wherein the sleeve second diameter is at least as great as 150% of the support member lateral width.

Example 100. The method of any example herein, particularly any one of examples 93 or 94, wherein the sleeve second diameter is at least as great as 200% of the support member lateral width.

Example 101. The method of any example herein, particularly any one of examples 84 to 92, wherein the commissural support member defines a maximal support member diameter, and wherein the heat-shrink sleeve, in a pre-shrunk state thereof, defines a minimal sleeve which is greater than the maximal support member diameter.

Example 102. The method of any example herein, particularly example 101, wherein the minimal sleeve diameter is at least as great as 120% of the maximal support member diameter.

Example 103. The method of any example herein, particularly example 101, wherein the minimal sleeve diameter is at least as great as 150% of the maximal support member diameter.

Example 104. The method of any example herein, particularly example 101, wherein the minimal sleeve diameter is at least as great as 200% of the maximal support member diameter.

Example 105. A method of assembling a prosthetic valve, the method comprising:
providing a prosthetic valve comprising a frame movable between a radially compressed and a radially expanded configuration, an inner skirt having a skirt proximal end, and a leaflet assembly sutured to the inner skirt along a scallop line that tracks the lower edge of the leaflet assembly;
folding a plurality of heat-shrink films over a matching plurality of strut segments of the frame, wherein each heat-shrink film has a film length and a film width prior to folding thereof, and wherein each strut segment has a strut width, a strut thickness, and a strut segment length;
suturing the inner skirt along the skirt proximal end to the folded heat-shrink films with at least one skirt attachment suture; and
applying heat to the heat-shrink films, thereby causing them to shrink radially inward and to become tightly attached around the corresponding strut segments.

Example 106. The method of any example herein, particularly example 105, wherein the heat-shrink film comprises at least one thermoplastic polymeric material selected from: polyolefins, fluoropolymers, polyvinyl chloride (PVC), polychloroprenes (neoprenes), silicone elastomers, and combinations thereof.

Example 107. The method of any example herein, particularly any one of examples 105 or 106, wherein the film length is not greater than the strut segment length.

Example 108. The method of any example herein, particularly any one of examples 105 to 107, wherein the film width in a pre-shrunk state thereof, is greater than the strut width plus twice the strut thickness.

Example 109. The method of any example herein, particularly any one of examples 105 to 107, wherein the film width in a pre-shrunk state thereof, is greater than twice the strut width plus twice the strut thickness.

Example 110. The method of any example herein, particularly any one of examples 105 to 109, wherein the step of suturing comprises looping the skirt attachment suture in a whip stitch pattern.

Example 111. The method of any example herein, particularly any one of examples 105 to 110, wherein the heat-shrink film is folded over a corresponding strut segment in a manner that defines a heat-shrink outer section disposed radially outward to the strut segment, heat shrink proximal and distal sections folded over proximal and distal edges of the strut segment, and first and second opposing heat-shrink inner sections folded therefrom and disposed radially inward to the strut segment.

Example 112. The method of any example herein, particularly example 111, wherein the step of suturing comprises:
passing the skirt attachment suture through the inner skirt and the first heat-shrink inner section;
extending the skirt attachment suture between the strut segment and the heat-shrink distal section toward and through the heat-shrink outer section;
folding the skirt attachment suture over the outer side of the heat-shrink outer section and passing it back through the heat-shrink outer section such that it further extends between the strut segment and the heat-shrink proximal section, forming a substantially U-shaped configuration around the strut segment; and
passing the skirt attachment suture back through the second heat-shrink inner section and the inner skirt, thereby forming a looped portion of the skirt attachment suture around the strut segment.

Example 113. The method of any example herein, particularly example 111, wherein the step of suturing comprises:
passing the skirt attachment suture through the inner skirt and the first heat-shrink inner section;
extending the skirt attachment suture between the strut segment and the heat-shrink distal section toward and through the heat-shrink outer section;
folding the skirt attachment suture over the outer side of the heat-shrink outer section and folding it back to extend over the external side of the heat-shrink proximal section, forming a substantially U-shaped configuration around the strut segment; and
passing the skirt attachment suture back through the inner skirt, thereby forming a looped portion of the skirt attachment suture around the strut segment.

Example 114. The method of any example herein, particularly any one of examples 105 to 113, wherein suturing the skirt proximal end is performed following a zig-zag pattern of the strut segments.

Example 115. The method of any example herein, particularly any one of examples 105 to 110, wherein applying heat to the heat-shrink films comprises applying heat at a temperature of above 90°C.

Example 116. The method of any example herein, particularly example 111, wherein applying heat to the heat-shrink film comprises applying heat at a temperature of above 135°C.

Example 117. A method of assembling a prosthetic valve, the method comprising:
providing a prosthetic valve comprising a frame movable between a radially compressed and a radially expanded configuration, an inner skirt having a skirt proximal end, and a leaflet assembly sutured to the inner skirt along a scallop line that tracks the lower edge of the leaflet assembly;
folding a plurality of heat-shrink films over a matching plurality of strut segments of the frame, wherein each heat-shrink film has a film length and a film width prior to folding thereof, and wherein each strut segment has a strut width, a strut thickness, and a strut segment length;
forming at least one suture loop around each folded heat-shrink film;
applying heat to the heat-shrink films, thereby causing them to shrink radially inward and to become tightly attached around the corresponding strut segments; and
suturing the inner skirt to the strut segments by passing at least one skirt attachment suture through the skirt proximal end and around the shrunken heat-shrink films.

Example 118. The method of any example herein, particularly example 117, wherein the heat-shrink film comprises at least one thermoplastic polymeric material selected from: polyolefins, fluoropolymers, polyvinyl chloride (PVC), polychloroprenes (neoprenes), silicone elastomers, and combinations thereof.

Example 119. The method of any example herein, particularly any one of examples 117 or 118, wherein the film length is not greater than the strut segment length.

Example 120. The method of any example herein, particularly any one of examples 117 to 119, wherein the film width in a pre-shrunk state thereof, is greater than the strut width plus twice the strut thickness.

Example 121. The method of any example herein, particularly any one of examples 117 to 119, wherein the film width in a pre-shrunk state thereof, is greater than twice the strut width plus twice the strut thickness.

Example 122. The method of any example herein, particularly any one of examples 117 to 121, wherein the heat-shrink film is folded over a corresponding strut segment in a manner that defines a heat-shrink outer section disposed radially outward to the strut segment, heat shrink proximal and distal sections folded over proximal and distal edges of the strut segment, and first and second opposing heat-shrink inner sections folded therefrom and disposed radially inward to the strut segment.

Example 123. The method of any example herein, particularly any one of examples 117 to 122, wherein the step of forming at least one suture loop comprises forming at least two suture loops around each folded heat-shrink film.

Example 124. The method of any example herein, particularly example 123, wherein the two suture loops are positioned at opposite end portions of the folded heat-shrink film.

Example 125. The method of any example herein, particularly any one of examples 117 to 124, wherein the perimeter of each suture loop is at least as great as the film width.

Example 126. The method of any example herein, particularly any one of examples 117 to 125, wherein forming the suture loop comprises surrounding a suture around the folded heat-shrink film, and tying a knot between free ends thereof.

Example 127. The method of any example herein, particularly any one of examples 117 to 126, wherein applying heat to the heat-shrink films comprises applying heat at a temperature of above 90°C.

Example 128. The method of any example herein, particularly example 127, wherein applying heat to the heat-shrink films comprises applying heat at a temperature of above 135°C.

Example 129. The method of any example herein, particularly any one of examples 117 to 128, further comprising cutting and removing the suture loops after applying heat to shrink the heat-shrink films.

Example 130. The method of any example herein, particularly any one of examples 117 to 128, further comprising cutting and removing the suture loops after suturing the inner skirt.

Example 131. A method of assembling a prosthetic valve, the method comprising:
providing a prosthetic valve comprising a frame movable between a radially compressed and a radially expanded configuration, and an inner skirt having a skirt proximal end;
disposing a plurality of C-shaped heat-shrink tubes over a matching plurality of strut segments of the frame, wherein each C-shaped heat-shrink tube has a tube length, a tube first diameter and a tube second diameter, and wherein each strut segment has a strut width, a strut thickness, and a strut segment length; and
applying heat to the C-shaped heat-shrink tubes, thereby causing them to shrink radially inward and to become tightly attached around the corresponding strut segments.

Example 132. The method of any example herein, particularly example 131, wherein the heat-shrink tube comprises at least one thermoplastic polymeric material selected from: polyolefins, fluoropolymers, polyvinyl chloride (PVC), polychloroprenes (neoprenes), silicone elastomers, and combinations thereof.

Example 133. The method of any example herein, particularly any one of examples 131 or 132, wherein disposing the C-shaped heat-shrink tubes over the strut segments comprises pushing each C-shaped heat-shrink tube at the side including a gap thereof, against the corresponding strut segment, until the strut segment is accommodated within the C-shaped heat-shrink tube.

Example 134. The method of any example herein, particularly example 133, wherein the gap has a gap width that is smaller than the strut width and/or the strut thickness, in a free state of the C-shaped heat-shrink tube.

Example 135. The method of any example herein, particularly any one of examples 131 to 134, wherein the tube length is not greater than the strut segment length.

Example 136. The method of any example herein, particularly any one of examples 131 to 135, wherein the tube first diameter is at least as great as 120% of the strut width.

Example 137. The method of any example herein, particularly any one of examples 131 to 135, wherein the tube first diameter is at least as great as 150% of the strut width.

Example 138. The method of any example herein, particularly any one of examples 131 to 135, wherein the tube first diameter is at least as great as 200% of the strut width.

Example 139. The method of any example herein, particularly any one of examples 131 to 135, wherein the tube second diameter is at least as great as 120% of the strut thickness.

Example 140. The method of any example herein, particularly any one of examples 131 to 135, wherein the tube second diameter is at least as great as 150% of the strut thickness.

Example 141. The method of any example herein, particularly any one of examples 131 to 135, wherein the tube second diameter is at least as great as 200% of the strut thickness.

Example 142. The method of any example herein, particularly any one of examples 131 to 135, wherein applying heat to the heat-shrink tubes comprises applying heat at a temperature of above 90°C.

Example 143. The method of any example herein, particularly example 133, wherein applying heat to the heat-shrink tubes comprises applying heat at a temperature of above 135°C.

Example 144. The method of any example herein, particularly any one of examples 131 to 143, further comprising a step of suturing an inner skirt along a skirt proximal end thereof, to the C-shaped heat-shrink tubes, with at least one skirt attachment suture, performed prior to the step of applying heat.

Example 145. The method of any example herein, particularly example 144, wherein each C-shaped heat-shrink tube is disposed over a corresponding strut segment such that it defines a heat-shrink outer section disposed radially outward to the strut segment, heat shrink proximal and distal sections folded over proximal and distal edges of the strut segment, and first and second opposing side arms disposed radially inward to the strut segment.

Example 146. The method of any example herein, particularly example 145, wherein the step of suturing comprises:
passing the skirt attachment suture through the inner skirt and the first side arm;
extending the skirt attachment suture between the strut segment and the heat-shrink distal section toward and through the heat-shrink outer section;
folding the skirt attachment suture over the outer side of the heat-shrink outer section and passing it back through the heat-shrink outer section such that it further extends between the strut segment and the heat-shrink proximal section, forming a substantially U-shaped configuration around the strut segment; and
passing the skirt attachment suture back through the second side arm and the inner skirt, thereby forming a looped portion of the skirt attachment suture around the strut segment.

Example 147. The method of any example herein, particularly example 145, wherein the step of suturing comprises:
passing the skirt attachment suture through the inner skirt and the first side arm;
extending the skirt attachment suture between the strut segment and the heat-shrink distal section toward and through the heat-shrink outer section;
folding the skirt attachment suture over the outer side of the heat-shrink outer section and folding it back to extend over the external side of the heat-shrink proximal section, forming a substantially U-shaped configuration around the strut segment; and
passing the skirt attachment suture back through the inner skirt, thereby forming a looped portion of the skirt attachment suture around the strut segment.

Example 148. The method of any example herein, particularly any one of examples 131 to 143, further comprising a step of suturing an inner skirt to the strut segments, by passing at least one skirt attachment suture through a skirt proximal end of the inner skirt, and around the shrunken heat-shrink films.

Example 149. A method of assembling a prosthetic valve, the method comprising:
providing a prosthetic valve comprising frame movable between a radially compressed and a radially expanded configuration, the frame comprising a plurality of commissural support members, each commissural support member having a support member radial depth and a support member lateral width;
disposing a heat-shrink sleeve over each commissural support member, wherein each heat-shrink sleeve has a sleeve length and a sleeve circular diameter, and wherein each commissural support member has support member length, a support member radial depth, and a support member lateral width;
applying heat to the heat-shrink sleeve, thereby causing it to shrink radially inward and to become tightly attached around the corresponding commissural support member; and
attaching a commissure assembly of a leaflet assembly to each commissural support member covered by the heat-shrink sleeve.

Example 150. The method of any example herein, particularly example 149, wherein each commissural support member is an axial strut segment integrally formed with the frame.

Example 151. The method of any example herein, particularly any one of examples 149 or 150, wherein the commissural support member has an exposed proximal end, and wherein disposing the heat-shrink sleeve over each commissural support member comprises sliding the heat-shrink sleeve from the exposed upper end over the corresponding commissural support member.

Example 152. The method of any example herein, particularly any one of examples 149 to 151, wherein the leaflet assembly comprises a plurality of leaflets configured to regulate flow through the prosthetic valve, wherein each leaflet comprises a pair of oppositely-directed tabs, and wherein the tabs of adjacent leaflets are paired to form the commissure assemblies.

Example 153. The method of any example herein, particularly any one of examples 149 to 152, wherein the heat-shrink sleeve comprises at least one thermoplastic polymeric material selected from: polyolefins, fluoropolymers, polyvinyl chloride (PVC), polychloroprenes (neoprenes), silicone elastomers, and combinations thereof.

Example 154. The method of any example herein, particularly any one of examples 149 to 153, wherein applying heat to the heat-shrink sleeve comprises applying heat at a temperature of above 90°C.

Example 155. The method of any example herein, particularly example 154, wherein applying heat to the heat-shrink sleeve comprises applying heat at a temperature of above 135°C.

Example 156. The method of any example herein, particularly any one of examples 149 to 155, wherein the sleeve length is in the range of 90% to 110% of the support member length.

Example 157. The method of any example herein, particularly any one of examples 149 to 155, wherein the sleeve length is greater than 110% of the support member length, and wherein the method further comprises a step of cutting the heat-shrink sleeve to a length that is not higher than 110% of the support member length prior to the step of applying heat.

Example 158. The method of any example herein, particularly any one of examples 149 to 157, wherein the sleeve circular diameter is at least as great as 120% of the support member radial depth and/or of the support member lateral width.

Example 159. The method of any example herein, particularly any one of examples 149 to 157, wherein the sleeve circular diameter is at least as great as 150% of the support member radial depth and/or of the support member lateral width.

Example 160. The method of any example herein, particularly any one of examples 149 to 157, wherein the sleeve circular diameter is at least as great as 200% of the support member radial depth and/or of the support member lateral width.

Example 161. A method of assembling a prosthetic valve, the method comprising:
providing a prosthetic valve comprising frame movable between a radially compressed and a radially expanded configuration, the frame comprising a plurality of commissural support members, each commissural support member having a support member radial depth and a support member lateral width;
folding a plurality of heat-shrink films over the matching plurality of commissural support members, wherein each heat-shrink film has a film length and a film width prior to folding thereof, and wherein each commissural support member has support member length, a support member radial depth, and a support member lateral width;
forming at least one suture loop around each folded heat-shrink film;
applying heat to the heat-shrink films, thereby causing them to shrink radially inward and to become tightly attached around the corresponding commissural support member; and
attaching a commissure assembly of a leaflet assembly to each commissural support member covered by the heat-shrink film.

Example 162. The method of any example herein, particularly example 161, wherein each commissural support member is an axial strut segment integrally formed with the frame.

Example 163. The method of any example herein, particularly any one of examples 161 or 162, wherein each commissural support member is bound between angled strut segments of the frame.

Example 164. The method of any example herein, particularly any one of examples 161 to 163, wherein the heat-shrink film comprises at least one thermoplastic polymeric material selected from: polyolefins, fluoropolymers, polyvinyl chloride (PVC), polychloroprenes (neoprenes), silicone elastomers, and combinations thereof.

Example 165. The method of any example herein, particularly any one of examples 161 to 164, wherein the film length is not greater than the support member length.

Example 166. The method of any example herein, particularly any one of examples 161 to 165, wherein the film width in a pre-shrunk state thereof, is greater than the support member lateral width plus twice the support member radial depth.

Example 167. The method of any example herein, particularly any one of examples 161 to 165, wherein the film width in a pre-shrunk state thereof, is greater than twice the support member lateral width plus twice the support member radial depth.

Example 168. The method of any example herein, particularly any one of examples 161 to 167, wherein the step of forming at least one suture loop comprises forming at least two suture loops around each folded heat-shrink film.

Example 169. The method of any example herein, particularly example 168, wherein the two suture loops are positioned at opposite end portions of the folded heat-shrink film.

Example 170. The method of any example herein, particularly any one of examples 161 to 169, wherein the perimeter of each suture loop is at least as great as the film width.

Example 171. The method of any example herein, particularly any one of examples 161 to 170, wherein forming the suture loop comprises surrounding a suture around the folded heat-shrink film, and tying a knot between free ends thereof.

Example 172. The method of any example herein, particularly any one of examples 161 to 171, wherein applying heat to the heat-shrink films comprises applying heat at a temperature of above 90°C.

Example 173. The method of any example herein, particularly example 172, wherein applying heat to the heat-shrink films comprises applying heat at a temperature of above 135°C.

Example 174. The method of any example herein, particularly any one of examples 161 to 173, further comprising cutting and removing the suture loops after applying heat to shrink the heat-shrink films.

Example 175. The method of any example herein, particularly any one of examples 161 to 173, further comprising cutting and removing the suture loops after attaching the commissure assembly.

Example 176. A method of assembling a prosthetic valve, the method comprising:
providing a prosthetic valve comprising a frame movable between a radially compressed and a radially expanded configuration, the frame comprising a plurality of commissural support members, each commissural support member having a support member radial depth and a support member lateral width;
disposing a plurality of C-shaped heat-shrink tubes over the matching plurality of commissural support members, wherein each C-shaped heat-shrink tube has a tube length, a tube first diameter and a tube second diameter, and wherein each commissural support member has support member length, a support member radial depth, and a support member lateral width;
applying heat to the C-shaped heat-shrink tubes, thereby causing them to shrink radially inward and to become tightly attached around the corresponding commissural support member; and
attaching a commissure assembly of a leaflet assembly to each commissural support member covered by the heat-shrink tube.

Example 177. The method of any example herein, particularly example 176, wherein each commissural support member is an axial strut segment integrally formed with the frame.

Example 178. The method of any example herein, particularly any one of examples 176 or 177, wherein each commissural support member is bound between angled strut segments of the frame.

Example 179. The method of any example herein, particularly any one of examples 176 to 178, wherein the heat-shrink tube comprises at least one thermoplastic polymeric material selected from: polyolefins, fluoropolymers, polyvinyl chloride (PVC), polychloroprenes (neoprenes), silicone elastomers, and combinations thereof.

Example 180. The method of any example herein, particularly any one of examples 176 to 179, wherein disposing the C-shaped heat-shrink tubes over the commissural support members comprises pushing each C-shaped heat-shrink tube at the side including a gap thereof, against the corresponding commissural support member, until the commissural support member is accommodated within the C-shaped heat-shrink tube.

Example 181. The method of any example herein, particularly example 180, wherein the gap has a gap width that is smaller than the strut width and/or the strut thickness, in a free state of the C-shaped heat-shrink tube.

Example 182. The method of any example herein, particularly any one of examples 176 to 181, wherein the tube length is not greater than the support member length.

Example 183. The method of any example herein, particularly any one of examples 176 to 182, wherein the tube first diameter is at least as great as 120% of the of the support member lateral width.

Example 184. The method of any example herein, particularly any one of examples 176 to 182, wherein the tube first diameter is at least as great as 150% of the of the support member lateral width.

Example 185. The method of any example herein, particularly any one of examples 176 to 182, wherein the tube first diameter is at least as great as 200% of the of the support member lateral width.

Example 186. The method of any example herein, particularly any one of examples 176 to 182, wherein the tube second diameter is at least as great as 120% of the of the support member radial depth.

Example 187. The method of any example herein, particularly any one of examples 176 to 182, wherein the tube second diameter is at least as great as 150% of the of the support member radial depth.

Example 188. The method of any example herein, particularly any one of examples 176 to 182, wherein the tube second diameter is at least as great as 200% of the of the support member radial depth.

Example 189. The method of any example herein, particularly any one of examples 176 to 188, wherein applying heat to the heat-shrink tubes comprises applying heat at a temperature of above 90°C.

Example 190. The method of any example herein, particularly example 189, wherein applying heat to the heat-shrink tubes comprises applying heat at a temperature of above 135°C.

Example 191. A prosthetic valve, comprising:
a frame movable between a radially compressed and a radially expanded configuration, the frame comprising a plurality of strut segments interconnected at junctions;
a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and
a plurality of pads covering at least some of the strut segments,
wherein each pad has an outer surface having a surface roughness Ra value of 0.2 µm or less.

Example 192. The prosthetic valve of any example herein, particularly example 191, wherein the strut segments comprise a plurality of angled strut segments covered by the pads.

Example 193. The prosthetic valve of any example herein, particularly any one of examples 191 or 192, wherein the strut segments comprise a plurality of axially extending strut segments covered by the pads.

Example 194. The prosthetic valve of any example herein, particularly any one of examples 191 to 193, wherein each pad comprises an attachment line facing radially outward from the frame.

Example 195. The prosthetic valve of any example herein, particularly any one of examples 191 to 194, wherein each pad comprises a base layer and a coating layer, wherein the coating layer defines the outer surface.

Example 196. The prosthetic valve of any example herein, particularly any one of examples 191 to 195, wherein each strut segment has an elliptic cross-section, wherein the strut width is a first diameter of the cross-section, wherein the base layer defines an inner surface facing the corresponding strut segment, and wherein the strut thickness is a second diameter of the cross-section.

Example 197. The prosthetic valve of any example herein, particularly any one of examples 191 to 196, wherein all of the strut segments covered by the pads are proximal to the scallop line.

Example 198. The prosthetic valve of any example herein, particularly any one of examples 191 to 197, wherein the plurality of pads further comprise a plurality of junction-pads covering at least portions of the junctions.

Example 199. The prosthetic valve of any example herein, particularly any one of examples 191 to 198, wherein the outer surface of each pad has a durometer hardness ranging from about 40 Shore A to about 98 Shore A.

Example 200. The prosthetic valve of any example herein, particularly any one of examples 191 to 199, wherein the outer surface of each pad has a durometer hardness ranging from about 0 Shore D to about 55 Shore D.

Example 201. The prosthetic valve of any example herein, particularly any one of examples 191 to 200, wherein the outer surface of each pad has an abrasion resistance value of less than about 0.05%.

Example 202. The prosthetic valve of any example herein, particularly any one of examples 191 to 201, wherein the outer surface of each pad has a kinetic coefficient of friction in the range of about 0.3-1.7.

Example 203. The prosthetic valve of any example herein, particularly any one of examples 191 to 202, wherein the outer surface of each pad has a water absorption capacity of less than 1.5%.

Example 204. The prosthetic valve of any example herein, particularly any one of examples 191 to 203, wherein each pad has a Vicat softening temperature selected from the range of about 150-180 °C.

Example 205. The prosthetic valve of any example herein, particularly any one of examples 191 to 204, wherein each pad has an MFI in the range of 5-6 g/10 min.

Example 206. The prosthetic valve of any example herein, particularly any one of examples 191 to 205, wherein each pad has a flexural strength in the range of about 5,000-10,000 psi.

Example 207. The prosthetic valve of any example herein, particularly any one of examples 191 to 206, wherein each pad has a tensile strength in the range of about 6,000-9,000 psi.

Example 208. The prosthetic valve of any example herein, particularly any one of examples 191 to 207, wherein each pad has a tensile strain at the failure point of about 200-500%.

Example 209. The prosthetic valve of any example herein, particularly any one of examples 191 to 208, wherein each pad has a secant tensile modulus in the range of about 700-2,400 psi, for a tensile strain of about 100 % or 300 %.

Example 210. The prosthetic valve of any example herein, particularly any one of examples 191 to 209, wherein each pad is biodurable.

Example 211. The prosthetic valve of any example herein, particularly any one of examples 191 to 210, wherein each pad comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 212. The prosthetic valve of any example herein, particularly example 211, wherein each pad comprises TPU.

Example 213. A prosthetic valve, comprising:
a frame movable between a radially compressed and a radially expanded configuration, the frame comprising a plurality of strut segments interconnected at junctions;
a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and
a plurality of pads covering at least some of the strut segments,
wherein each pad has an outer surface having a surface roughness Ra value that is 80% or less of a Ra value of a surface of the frame.

Example 214. The prosthetic valve of any example herein, particularly example 213, wherein the strut segments comprise a plurality of angled strut segments covered by the pads.

Example 215. The prosthetic valve of any example herein, particularly any one of examples 213 or 214, wherein the strut segments comprise a plurality of axially extending strut segments covered by the pads.

Example 216. The prosthetic valve of any example herein, particularly any one of examples 213 to 215, wherein each pad comprises an attachment line facing radially outward from the frame.

Example 217. The prosthetic valve of any example herein, particularly any one of examples 213 to 216, wherein all of the strut segments covered by the pads are proximal to the scallop line.

Example 218. The prosthetic valve of any example herein, particularly any one of examples 213 to 217, wherein each pad has a Ra value that is 50% or less of the Ra value of the surface of the frame.

Example 219. A prosthetic valve, comprising:
a frame movable between a radially compressed and a radially expanded configuration, the frame comprising a plurality of strut segments interconnected at junctions;
a leaflet assembly mounted within the frame and comprising a plurality of leaflets configured to regulate flow through the prosthetic valve by transitioning between a fully-open state and a closed state, wherein the leaflet assembly is coupled to the frame along a scallop line that tracks the lower edge of the leaflet assembly; and
a plurality of pads covering at least some of the strut segments, wherein each pad comprises an outer surface,
wherein the leaflets are configured to at least partially contact the pads in the fully-open state.

Example 220. The prosthetic valve of any example herein, particularly example 219, wherein the strut segments comprise a plurality of angled strut segments covered by the pads.

Example 221. The prosthetic valve of any example herein, particularly any one of examples 219 or 220, wherein the strut segments comprise a plurality of axially extending strut segments covered by the pads.

Example 222. The prosthetic valve of any example herein, particularly any one of examples 219 to 221, wherein each pad comprises an attachment line facing radially outward from the frame.

Example 223. The prosthetic valve of any example herein, particularly any one of examples 219 to 222, wherein each pad comprises a base layer and a coating layer, wherein the coating layer defines the outer surface.

Example 224. The prosthetic valve of any example herein, particularly any one of examples 219 to 223, wherein each strut segment has an elliptic cross-section, wherein the strut width is a first diameter of the cross-section, wherein the base layer defines an inner surface facing the corresponding strut segment, and wherein the strut thickness is a second diameter of the cross-section.

Example 224. The prosthetic valve of any example herein, particularly any one of examples 219 to 221, wherein all of the strut segments covered by the pads are proximal to the scallop line.

Example 226. The prosthetic valve of any example herein, particularly any one of examples 219 to 225, wherein the plurality of pads further comprise a plurality of junction-pads covering at least portions of the junctions.

Example 227. The prosthetic valve of any example herein, particularly any one of examples 219 to 226, wherein the outer surface of each pad has a surface roughness Ra value of 0.2 µm or less.

Example 228. The prosthetic valve of any example herein, particularly any one of examples 219 to 226, wherein the outer surface of each pad has a surface roughness Ra value that is 80% or less of a Ra value of a surface of the frame.

Example 229. The prosthetic valve of any example herein, particularly example 228, wherein each pad has a Ra value that is 50% or less of the Ra value of the surface of the frame.

Example 230. The prosthetic valve of any example herein, particularly any one of examples 219 to 229, wherein the outer surface of each pad has a durometer hardness ranging from about 40 Shore A to about 98 Shore A.

Example 231. The prosthetic valve of any example herein, particularly any one of examples 219 to 230, wherein the outer surface of each pad has a durometer hardness ranging from about 0 Shore D to about 55 Shore D.

Example 232. The prosthetic valve of any example herein, particularly any one of examples 219 to 231, wherein the outer surface of each pad has an abrasion resistance value of less than about 0.05%.

Example 233. The prosthetic valve of any example herein, particularly any one of examples 219 to 232, wherein the outer surface of each pad has a kinetic coefficient of friction in the range of about 0.3-1.7.

Example 234. The prosthetic valve of any example herein, particularly any one of examples 219 to 233, wherein the outer surface of each pad has a water absorption capacity of less than 1.5%.

Example 235. The prosthetic valve of any example herein, particularly any one of examples 219 to 234, wherein each pad has a Vicat softening temperature selected from the range of about 150-180 °C.

Example 236. The prosthetic valve of any example herein, particularly any one of examples 219 to 235, wherein each pad has an MFI in the range of 5-6 g/10 min.

Example 237. The prosthetic valve of any example herein, particularly any one of examples 219 to 236, wherein each pad has a flexural strength in the range of about 5,000-10,000 psi.

Example 238. The prosthetic valve of any example herein, particularly any one of examples 219 to 237, wherein each pad has a tensile strength in the range of about 6,000-9,000 psi.

Example 239. The prosthetic valve of any example herein, particularly any one of examples 219 to 238, wherein each pad has a tensile strain at the failure point of about 200-500 %.

Example 240. The prosthetic valve of any example herein, particularly any one of examples 219 to 239, wherein each pad has a secant tensile modulus in the range of about 700-2,400 psi, for a tensile strain of about 100 % or 300 %.

Example 241. The prosthetic valve of any example herein, particularly any one of examples 219 to 240, wherein each pad is biodurable.

Example 242. The prosthetic valve of any example herein, particularly any one of examples 219 to 239, wherein each pad comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 243. The prosthetic valve of any example herein, particularly example 242, wherein each pad comprises TPU.

Example 244. A prosthetic valve, comprising:
a frame movable between a radially compressed and a radially expanded configuration, wherein the frame comprises a plurality of strut segments interconnected at junctions, and wherein the frame defines an inner frame surface and an outer frame surface;
a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and
a coating covering the inner frame surface or at least a portion thereof,
wherein the coating forms a polymeric covering layer characterized by having a surface roughness Ra value of 0.2 µm or less.

Example 245. The prosthetic valve of any example herein, particularly example 244, wherein the coating covers the portion of the inner frame surface which is proximal to the scallop line.

Example 246. The prosthetic valve of any example herein, particularly any one of examples 244 or 245, wherein the coating further covers the outer frame surface or a portion thereof.

Example 247. The prosthetic valve of any example herein, particularly any one of examples 244 to 246, wherein the polymeric covering layer forming the coating is characterized by having a durometer hardness ranging from about 40 Shore A to about 98 Shore A.

Example 248. The prosthetic valve of any example herein, particularly any one of examples 244 to 247, wherein the polymeric covering layer forming the coating is characterized by having a durometer hardness ranging from about 0 Shore D to about 55 Shore D.

Example 249. The prosthetic valve of any example herein, particularly any one of examples 244 to 248, wherein the polymeric covering layer forming the coating is characterized by having an abrasion resistance value of less than about 0.05%.

Example 250. The prosthetic valve of any example herein, particularly any one of examples 244 to 249, wherein the polymeric covering layer forming the coating is characterized by having a kinetic coefficient of friction in the range of about 0.3-1.7.

Example 251. The prosthetic valve of any example herein, particularly any one of examples 244 to 250, wherein the polymeric covering layer forming the coating is characterized by having a water absorption capacity of less than 1.5%.

Example 252. The prosthetic valve of any example herein, particularly any one of examples 244 to 251, wherein the polymeric covering layer forming the coating is characterized by having a Vicat softening temperature selected from the range of about 150-180 °C.

Example 253. The prosthetic valve of any example herein, particularly any one of examples 244 to 252, wherein the polymeric covering layer forming the coating is characterized by having an MFI in the range of 5-6 g/10 min.

Example 254. The prosthetic valve of any example herein, particularly any one of examples 244 to 253, wherein the polymeric covering layer forming the coating is characterized by having a tensile strength in the range of about 6,000-9,000 psi.

Example 255. The prosthetic valve of any example herein, particularly any one of examples 244 to 254, wherein each pad has a tensile strain at the failure point of about 200-500 %.

Example 256. The prosthetic valve of any example herein, particularly any one of examples 244 to 255, wherein each pad has a secant tensile modulus in the range of about 700-2,400 psi, for a tensile strain of about 100 % or 300 %.

Example 257. The prosthetic valve of any example herein, particularly any one of examples 244 to 256, wherein the coating is non-biodegradable.

Example 258. The prosthetic valve of any example herein, particularly any one of examples 244 to 257, wherein the coating is non-porous.

Example 259. The prosthetic valve of any example herein, particularly any one of examples 244 to 258, wherein the coating is biodurable.

Example 260. The prosthetic valve of any example herein, particularly any one of examples 244 to 259, wherein the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 261. The prosthetic valve of any example herein, particularly example 260, wherein the coating comprises TPU.

Example 262. The prosthetic valve of any example herein, particularly any one of examples 244 to 261, wherein the coating covering the frame is applied by a method comprising: (a) coating strut segments of the frame using a first polymeric layer, and (b) coating the first polymeric layer using a second polymeric layer configured to bond with the first polymeric layer without bonding with the junctions.

Example 263. A prosthetic valve, comprising:
a frame movable between a radially compressed and a radially expanded configuration, wherein the frame comprises a plurality of strut segments interconnected at junctions, and wherein the frame defines an inner frame surface and an outer frame surface;
a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and
a coating covering the inner frame surface or at least a portion thereof,
wherein the coating forms a polymeric covering layer characterized by having a surface roughness Ra value that is 80% or less of a Ra value of a surface of the frame.

Example 264. The prosthetic valve of any example herein, particularly example 263, wherein the coating covers the portion of the inner frame surface which is proximal to the scallop line.

Example 265. The prosthetic valve of any example herein, particularly any one of examples 263 or 264, wherein the coating further covers the outer frame surface or a portion thereof.

Example 266. The prosthetic valve of any example herein, particularly any one of examples 263 to 265, wherein the polymeric covering layer forming the coating is characterized by having a durometer hardness ranging from about 40 Shore A to about 98 Shore A.

Example 267. The prosthetic valve of any example herein, particularly any one of examples 263 to 266, wherein the polymeric covering layer forming the coating is characterized by having a durometer hardness ranging from about 0 Shore D to about 55 Shore D.

Example 268. The prosthetic valve of any example herein, particularly any one of examples 263 to 267, wherein the polymeric covering layer forming the coating is characterized by having an abrasion resistance value of less than about 0.05%.

Example 269. The prosthetic valve of any example herein, particularly any one of examples 263 to 268, wherein the polymeric covering layer forming the coating has a Ra value that is 50% or less of the Ra value of the surface of the frame.

Example 270. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface,
(b) coating the frame via dip coating by immersing the frame in a solution comprising a coating material; extracting the frame from within the coating material solution, wherein a thin coating layer forms on the surface of the frame during the extraction thereof; and solidifying the coating layer formed on the surface of the frame, thereby forming the coating thereon, and
(c) mounting within the frame a leaflet assembly comprising a plurality of leaflets, wherein the leaflet assembly is coupled to the frame along a scallop line that tracks the lower edge of the leaflet assembly,
wherein the coating is characterized by having a surface roughness Ra value of 0.2 µm or less.

Example 271. The method of any example herein, particularly example 270, wherein the coating covers both of the inner and outer frame surfaces.

Example 272. The method of any example herein, particularly example 270, wherein step (a) further comprises masking a portion of the frame with a masking layer, so that the frame is provided having a first unmasked portion and a second masked portion, wherein the first unmasked portion comprises a portion of the frame which is proximal to the designated scallop line along which leaflet assembly is to be coupled to the frame in step (c).

Example 273. The method of any example herein, particularly example 272, wherein step (b) comprises forming a coating layer on the first unmasked portion and the second masked portion comprising the masking layer, during the extraction of the frame from within the coating material solution.

Example 274. The method of any example herein, particularly example 273, wherein step (b) further comprises removing the masking layer from the second masked portion following the formation of the solidified coating on the first unmasked portion.

Example 275. The method of any example herein, particularly any one of examples 272 to 274, wherein the masking of the second masked portion comprises utilizing a removable adhesive layer.

Example 276. The method of any example herein, particularly any one of examples 270 to 275, wherein the coating is characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

Example 277. The method of any example herein, particularly any one of examples 270 to 276, wherein the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 278. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface,
(b) coating the frame via dip coating by immersing the frame in a solution comprising a coating material; extracting the frame from within the coating material solution, wherein a thin coating layer forms on the surface of the frame during the extraction thereof; and solidifying the coating layer formed on the surface of the frame, thereby forming the coating thereon, and
(c) mounting within the frame a leaflet assembly comprising a plurality of leaflets, wherein the leaflet assembly is coupled to the frame along a scallop line that tracks the lower edge of the leaflet assembly,
wherein the coating is characterized by having a surface roughness Ra value that is 80% or less of a Ra value of a surface of the frame.

Example 279. The method of any example herein, particularly example 278, wherein the coating covers both of the inner and outer frame surfaces.

Example 280. The method of any example herein, particularly example 279, wherein step (a) further comprises masking a portion of the frame with a masking layer, so that the frame is provided having a first unmasked portion and a second masked portion, wherein the first unmasked portion comprises a portion of the frame which is proximal to the designated scallop line along which leaflet assembly is to be coupled to the frame in step (c).

Example 281. The method of any example herein, particularly example 280, wherein step (b) comprises forming a coating layer on the first unmasked portion and the second masked portion comprising the masking layer, during the extraction of the frame from within the coating material solution.

Example 282. The method of any example herein, particularly example 281, wherein step (b) further comprises removing the masking layer from the second masked portion following the formation of the solidified coating on the first unmasked portion.

Example 283. The method of any example herein, particularly any one of examples 278 to 282, wherein the Ra value of the coating is 50% or less of the Ra value of the surface of the frame.

Example 284. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface;
(b) spray-coating the surface of the frame or a portion thereof, by positioning a spray nozzle internally within the inner frame surface, thereby forming a coating layer thereon, and
(c) mounting within the frame a leaflet assembly comprising a plurality of leaflets, wherein the leaflet assembly is coupled to the frame along a scallop line that tracks the lower edge of the leaflet assembly,
wherein the coating is characterized by having a surface roughness Ra value of 0.2 µm or less.

Example 285. The method of any example herein, particularly example 284, wherein step (b) further comprises spray-coating the surface of the outer frame surface, or a portion thereof, by positioning the spray nozzle externally to the outer frame surface, thereby forming a coating layer thereon.

Example 286. The method of any example herein, particularly any one of examples 284 or 285, wherein step (b) further comprises rotating at least one of the frame, the spray nozzle, or both during the coating process, in order to create a uniform coating around or along frame.

Example 287. The method of any example herein, particularly any one of examples 284 to 286, wherein step (a) further comprises masking a plurality of junctions of the frame utilizing removable adhesive masking layers wrapped therearound, wherein following the spray-coating of step (b) the masking layers are removed from the junctions, resulting in coating solely the strut segments of the frame.

Example 288. The method of any example herein, particularly any one of examples 284 to 286, wherein step (a) further comprises masking a portion of the frame utilizing a removable adhesive layer thereby forming a masked portion and an unmasked portion thereof, wherein following the spray-coating of step (b) the masking layer is removed from the masked portion, resulting in coating solely of the unmasked portion, and wherein the unmasked portion of the frame is configured to be contacted at least partially by the leaflets of step (c).

Example 289. The method of any example herein, particularly any one of examples 284 to 286, wherein step (b) comprises targeting the spray nozzle to coat solely a desired portion of the frame, wherein said desired portion is configured to be contacted at least partially by the leaflets of step (c).

Example 290. The method of any example herein, particularly any one of examples 284 to 289, wherein the coating is characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

Example 291. The method of any example herein, particularly any one of examples 284 to 290, wherein the coating comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 292. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface;
(b) spray-coating the surface of the frame or a portion thereof, by positioning a spray nozzle internally within the inner frame surface, thereby forming a coating layer thereon, and
(c) mounting within the frame a leaflet assembly comprising a plurality of leaflets, wherein the leaflet assembly is coupled to the frame along a scallop line that tracks the lower edge of the leaflet assembly,
wherein the coating is characterized by having a surface roughness Ra value that is 80% or less of a Ra value of a surface of the frame.

Example 293. The method of any example herein, particularly example 292, wherein the coating has a Ra value that is 50% or less of the Ra value of the surface of the frame.

Example 294. The method of any example herein, particularly any one of examples 292 or 293, wherein step (a) further comprises masking a plurality of junctions of the frame utilizing removable adhesive masking layers wrapped therearound, wherein following the spray-coating of step (b) the masking layers are removed from the junctions, resulting in coating solely the strut segments of the frame.

Example 295. The method of any example herein, particularly any one of examples 292 or 293, wherein step (a) further comprises masking a portion of the frame utilizing a removable adhesive layer thereby forming a masked portion and an unmasked portion thereof, wherein following the spray-coating of step (b) the masking layer is removed from the masked portion, resulting in coating solely of the unmasked portion, and wherein the unmasked portion of the frame is configured to be contacted at least partially by the leaflets of step (c).

Example 296. The method of any example herein, particularly any one of examples 292 or 293, wherein step (b) comprises targeting the spray nozzle to coat solely a desired portion of the frame, wherein said desired portion is configured to be contacted at least partially by the leaflets of step (c).

Example 297. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface;
(b) situating an inner coating layer around a mandrel;
(c) situating the frame of step (a) around the inner coating layer of step (b), thereby contacting the inner frame surface therewith;
(d) laminating the inner coating layer to the inner surface of the frame;
(e) removing the laminated frame of step (d) from the mandrel, and
(f) mounting within the frame a leaflet assembly comprising a plurality of leaflets, wherein the leaflet assembly is coupled to the frame along a scallop line that tracks the lower edge of the leaflet assembly,
wherein the inner coating layer is characterized by having a surface roughness Ra value of 0.2 µm or less.

Example 298. The method of any example herein, particularly example 297, wherein the inner coating layer is in the form of a pre-fabricated thin polymeric sheet that is applied by being wrapped around the mandrel.

Example 299. The method of any example herein, particularly example 297, wherein the inner coating layer is applied to the mandrel by dip coating, spray-coating, electrospinning, or a combination thereof.

Example 300. The method of any example herein, particularly any one of examples 297 to 299, wherein step (c) further comprises situating an outer coating layer around the frame, thereby contacting the outer frame surface therewith.

Example 301. The method of any example herein, particularly example 300, wherein the outer coating layer is in the form of a pre-fabricated thin polymeric sheet that is applied by being wrapped around the frame.

Example 302. The method of any example herein, particularly example 300, wherein the outer coating layer is applied to the frame by dip coating, spray-coating, electrospinning, or a combination thereof.

Example 303. The method of any example herein, particularly any one of examples 300 to 302, wherein step (d) further comprises laminating the inner and outer coating layers to the frame or to each other, so that the frame becomes encapsulated between.

Example 304. The method of any example herein, particularly any one of examples 300 to 302, wherein step (c) further comprises situating an outer skirt on the outer coating layer.

Example 305. The method of any example herein, particularly example 304, wherein step (d) further comprises laminating the inner and outer coating layers to the frame or to each other, and laminating the outer skirt to the outer coating layer, so that the outer skirt becomes bonded to the outer coating layer and the frame becomes encapsulated between the inner and outer coating layers.

Example 306. The method of any example herein, particularly any one of examples 297 or 298, wherein step (c) further comprises situating an outer skirt around the frame, thereby contacting an outer frame surface therewith.

Example 307. The method of any example herein, particularly example 306, wherein step (d) further comprises laminating the outer skirt to the inner coating layer, so that the outer skirt becomes bonded directly to the inner coating layer, through openings defined between the strut segments.

Example 308. The method of any example herein, particularly any one of examples 297 to 307, wherein the lamination of step (d) is performed by the application of at least one of heat, pressure, ultrasonic welding, adhesives, or a combination thereof.

Example 309. The method of any example herein, particularly example 308, wherein the lamination of step (d) comprises pressure applied externally to the frame by utilizing at least one of a clamp, a crimper, a pincer, or a combination thereof.

Example 310. The method of any example herein, particularly any one of examples 308 or 309, wherein the lamination of step (d) comprises pressure applied internally to the frame by utilizing an inflatable or an expandable mandrel.

Example 311. The method of any example herein, particularly any one of examples 300 to 305, wherein at least one of the inner and outer coating layers comprises at least one material selected from the group consisting of: a polycarbonate, a polyamide, a polyester, polytetrafluoroethylene (PTFE), ePTFE, UHMWPE, a polyolefin, a polyether, a polyurethane, a thermoplastic polyurethane (TPU), and combinations and copolymers thereof.

Example 312. The method of any example herein, particularly any one of examples 300 to 305, wherein at least one of the inner and outer coating layers are characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

Example 313. The method of any example herein, particularly any one of examples 300 to 305, wherein at least one of the inner and outer coating layers is biodurable.

Example 314. A method of assembling a prosthetic valve, the method comprising the steps of:
(a) providing a prosthetic valve comprising a frame movable between a radially compressed and a radially expanded configuration, the frame comprising a plurality of strut segments interconnected at junctions, and wherein the frame defines an inner frame surface and an outer frame surface;
(b) situating an inner coating layer around a mandrel;
(c) situating the frame of step (a) around the inner coating layer of step (b), thereby contacting the inner frame surface therewith;
(d) laminating the inner coating layer to the inner surface of the frame;
(e) removing the laminated frame of step (d) from the mandrel, and
(f) mounting within the frame a leaflet assembly comprising a plurality of leaflets, wherein the leaflet assembly is coupled to the frame along a scallop line that tracks the lower edge of the leaflet assembly,
wherein the inner coating layer is characterized by having a surface roughness Ra value that is 80% or less of a Ra value of a surface of the frame.

Example 315. The method of any example herein, particularly example 314, wherein step (c) further comprises situating an outer coating layer around the frame, thereby contacting the outer frame surface therewith.

Example 316. The method of any example herein, particularly example 315, wherein step (d) further comprises laminating the inner and outer coating layers to the frame or to each other, so that the frame becomes encapsulated between.

Example 317. The method of any example herein, particularly example 316, wherein step (c) further comprises situating an outer skirt on the outer coating layer, and wherein step (d) further comprises laminating the inner and outer coating layers to the frame or to each other, and laminating the outer skirt to the outer coating layer, so that the outer skirt becomes bonded to the outer coating layer and the frame becomes encapsulated between the inner and outer coating layers.

Example 318. The method of any example herein, particularly example 314, wherein step (c) further comprises situating an outer skirt around the frame, thereby contacting an outer frame surface therewith, and wherein step (d) further comprises laminating the outer skirt to the inner coating layer, so that the outer skirt becomes bonded directly to the inner coating layer, through openings defined between the strut segments.

Example 319. The method of any example herein, particularly any one of examples 314 to 318, wherein the inner coating layer has a Ra value that is 50% or less of the Ra value of the surface of the frame.

It is appreciated that certain features of the invention, which are, for clarity, described in the context of separate examples, may also be provided in combination in a single example. Conversely, various features of the invention, which are, for brevity, described in the context of a single example, may also be provided separately or in any suitable sub-combination or as suitable in any other described example of the invention. No feature described in the context of an example is to be considered an essential feature of that example, unless explicitly specified as such.

In view of the many possible examples to which the principles of the disclosure may be applied, it should be recognized that the illustrated examples are only preferred examples and should not be taken as limiting the scope. Rather, the scope is defined by the following claims. We therefore claim all that comes within the scope and spirit of these claims.

The invention further comprises the following embodiments:
1. A prosthetic valve, comprising: a frame movable between a radially compressed and a radially expanded configuration, wherein the frame comprises a plurality of strut segments interconnected at junctions, and wherein the frame defines an inner frame surface and an outer frame surface; a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and a coating covering the inner frame surface or at least a portion thereof, wherein the coating forms a polymeric covering layer characterized by having a surface roughness Ra value of 0.2 µm or less.
2. The prosthetic valve of embodiment 1, wherein the coating covers the portion of the inner frame surface which is proximal to the scallop line.
3. The prosthetic valve of any one of embodiments 1 or 2, wherein the polymeric covering layer forming the coating is characterized by having a durometer hardness ranging from about 40 Shore A to about 98 Shore A.
4. The prosthetic valve of any one of embodiments 1 to 3, wherein the polymeric covering layer forming the coating is characterized by having an abrasion resistance value of less than about 0.05%.
5. The prosthetic valve of any one of embodiments 1 to 4, wherein the polymeric covering layer forming the coating is characterized by having a kinetic coefficient of friction in the range of about 0.3-1.7.
6. The prosthetic valve of any one of embodiments 1 to 5, wherein the polymeric covering layer forming the coating is characterized by having a water absorption capacity of less than 1.5%.
7. The prosthetic valve of any one of embodiments 1 to 6, wherein the polymeric covering layer forming the coating is characterized by having a Vicat softening temperature selected from the range of about 150-180 °C.
8. The prosthetic valve of any one of embodiments 1 to 7, wherein the polymeric covering layer forming the coating is characterized by having an MFI in the range of 5-6 g/10 min.
9. The prosthetic valve of any one of embodiments 1 to 8, wherein the polymeric covering layer forming the coating is characterized by having a tensile strength in the range of about 6,000-9,000 psi.
10. The prosthetic valve of any one of embodiments 1 to 99, wherein each pad has a tensile strain at the failure point of about 200-500 %.
11. The prosthetic valve of any one of embodiments 1 to 10, wherein each pad has a secant tensile modulus in the range of about 700-2,400 psi, for a tensile strain of about 100 % or 300 %.
12. The prosthetic valve of any one of embodiments 1 to 11, wherein the coating covering the frame is applied by a method comprising: (a) coating strut segments of the frame using a first polymeric layer, and (b) coating the first polymeric layer using a second polymeric layer configured to bond with the first polymeric layer without bonding with the junctions.
13. A prosthetic valve, comprising: a frame movable between a radially compressed and a radially expanded configuration, wherein the frame comprises a plurality of strut segments interconnected at junctions, and wherein the frame defines an inner frame surface and an outer frame surface; a leaflet assembly mounted within the frame and comprising a plurality of leaflets configured to regulate flow through the prosthetic valve, wherein the leaflet assembly is coupled to the frame along a scallop line that tracks the lower edge of the leaflet assembly; and a coating covering the inner frame surface or at least a portion thereof, wherein the coating forms a polymeric covering layer characterized by having a surface roughness Ra value that is 80% or less of a Ra value of a surface of the frame.
14. The prosthetic valve of embodiment 13, wherein the coating covers the portion of the inner frame surface which is proximal to the scallop line.
15. The prosthetic valve of any one of embodiments 13 or 14, wherein the polymeric covering layer forming the coating is characterized by having a durometer hardness ranging from about 40 Shore A to about 98 Shore A.
16. The prosthetic valve of any one of embodiments 13 to 15, wherein the polymeric covering layer forming the coating is characterized by having an abrasion resistance value of less than about 0.05%.
17. A method of assembling a prosthetic valve, the method comprising: (a) providing a prosthetic valve comprising a frame movable between a radially compressed and a radially expanded configuration, the frame comprising a plurality of strut segments interconnected at junctions, and wherein the frame defines an inner frame surface and an outer frame surface, (b) coating the frame via dip coating by immersing the frame in a solution comprising a coating material; extracting the frame from within the coating material solution, wherein a thin coating layer forms on the surface of the frame during the extraction thereof; and solidifying the coating layer formed on the surface of the frame, thereby forming the coating thereon, and (c) mounting within the frame a leaflet assembly comprising a plurality of leaflets, wherein the leaflet assembly is coupled to the frame along a scallop line that tracks the lower edge of the leaflet assembly, wherein the coating is characterized by having a surface roughness Ra value of 0.2 µm or less.
18. The method of embodiment 17, wherein step (a) further comprises masking a portion of the frame with a masking layer, so that the frame is provided having a first unmasked portion and a second masked portion, wherein the first unmasked portion comprises a portion of the frame which is proximal to the designated scallop line along which leaflet assembly is to be coupled to the frame in step (c).
19. The method of embodiment 18, wherein step (b) comprises forming a coating layer on the first unmasked portion and the second masked portion comprising the masking layer, during the extraction of the frame from within the coating material solution.
20. The method of any one of embodiments 17 to 19, wherein the coating is characterized by having at least one property selected from the group consisting of: a durometer hardness ranging from about 40 Shore A to about 98 Shore A; a durometer hardness ranging from about 0 Shore D to about 55 Shore D; an abrasion resistance value of less than about 0.05%; a kinetic coefficient of friction in the range of about 0.3-1.7; a water absorption capacity of less than 1.5%; a Vicat softening temperature selected from the range of about 150-180 °C; a MFI in the range of 5-6 g/10 min; a tensile strength in the range of about 6,000-9,000 psi; a tensile strain at the failure point of about 200-500 %; a secant tensile modulus in the range of about 700-2,400 psi, for a tensile strain of about 100 % or 300 %, and combinations thereof.

## Claims

1. A prosthetic heart valve comprising:
an expandable frame (106);
a leaflet assembly (122) mounted within the frame (106) and coupled thereto via a plurality of commissure assemblies (130), formed from tabs (126) of adjacent leaflets (124) secured to each other; and
a protective cover configured to be disposed around various segments of the prosthetic valve (100);
wherein the protective cover comprises at least one heat-shrink wrap (150).

2. The prosthetic heart valve of claim 1, wherein the commissure assemblies (130) are secured, directly or indirectly, to commissural support members (128); wherein the commissural support members (128) are either integrally formed with the frame (106), or provided as separate components attached to the frame (106).

3. The prosthetic heart valve of claim 2, wherein the least one heat-shrink wrap (150) is configured to be disposed around around at least a portion of a commissural support member (128).

4. The prosthetic heart valve of any one of the preceding claims, wherein each commissural support member (128) is covered by a heat-shrink wrap (150) tightly shrunk thereover; and
wherein each commissure assembly (130) is attached to a corresponding commissural support member (128) over the heat-shrink wrap (150).

5. The prosthetic heart valve of any one of the preceding claims, wherein the frame (106) includes a plurality of interconnected struts (110), wherein the struts define strut segments (112) which are struts or segments of struts defined between junctions (114) of the frame (106).

6. The prosthetic heart valve of claim 5, wherein the least one heat-shrink wrap (150) is configured to be disposed around around at least a portion of the strut segments (112).

7. The prosthetic heart valve of any one of claims 1-4, further comprising an inner skirt (120) disposed along an inner surface (144) of the frame (106);
wherein the inner skirt (120) comprises a skirt proximal end (121) attached to strut segments (112) extending along the circumference of the frame (106);
wherein the leaflet assembly (122) is sutured to the inner skirt (120) along a scallop line (134) that tracks the lower edge of the leaflet assembly (122); and
wherein the heat-shrink wraps (150) are wraps are tightly shrunk over the strut segments (112) to which the skirt proximal end (121) is attached.

8. The prosthetic heart valve of claim 7, wherein the skirt proximal end (121) is sutured to the strut segment with at least one skirt attachment suture (136).

9. The prosthetic heart valve of claim 8, wherein the skirt attachment suture (136) is looped through the skirt proximal end (121) and the heat-shrink film, in a whip stitch pattern around the strut segment.

10. The prosthetic heart valve of claim 8, wherein the skirt attachment suture (136) is sutured over the external surface of the heat-shrink film, without extending through the heat-shrink film.

11. The prosthetic heart valve of any one of claims 2-4, wherein the frame (106) comprises a plurality of axially extending strut segments (113), wherein at least some of the axially extending strut segments (113) are the commissural support members (128).

12. The prosthetic heart valve of claim 11, wherein each commissural support member (128) has an exposed proximal end.

13. The prosthetic heart valve of any one of claims 11 or 12, wherein each commissural support member (128) is bound between angled strut segments (112) of the frame (106).

14. The prosthetic heart valve of any one of the preceding claims, wherein the heat-shrink wrap (150) comprises at least one thermoplastic polymeric material selected from: polyolefins, fluoropolymers, polyvinyl chloride, polychloroprenes, silicone elastomers, and combinations thereof.

15. The prosthetic heart valve of any one of the preceding claims, wherein the prosthetic valve (100) is balloon-expandable.
